# EUROPEAN PATENT SPECIFICATION

(11) **EP 1 178 973 B1**
(45) Date of publication and mention of the grant of the patent: **21.12.2005**
(21) Application number: 00931877.5
(22) Date of filing: 19.05.2000
(51) Int. Cl.: C07D 241/18, C07D 241/20, C07D 405/12, A61K 31/497, A61P 25/00

(54) **PYRAZINYL-PIPERAZINE- COMPOUNDS, THEIR USE AND PREPARATION**
PYRAZINYL-PIPERAZIN-VERBINDUNGEN, IHRE ANWENDUNG UND HERSTELLUNG
COMPOSES PYRAZINYL-PIPERAZINE, UTILISATION ET PREPARATION DE CES DERNIERS

(30) Priority: 21.05.1999 SE 9901884; 03.06.1999 US 137527 P
(43) Date of publication of application: 13.02.2002
(73) Proprietor: BIOVITRUM AB, 112 76 Stockholm (SE)
(72) Inventor: NILSSON, Björn, S-754 23 Uppsala (SE); TEJBRANT, Jan, S-122 42 Enskede (SE); PELCMAN, Benjamin, S-113 52 Stockholm (SE); RINGBERG, Erik, S-753 26 Uppsala (SE); THOR, Markus, S-741 40 Knivsta (SE); NILSSON, Jonas, S-752 43 Uppsala (SE); JÖNSSON, Mattias, S-756 49 Uppsala (SE)
(74) Representative: Höglund, Lars
(86) International application number: PCT/SE2000/001017
(87) International publication number: WO 2000/076984

(56) References cited:
- WO-A1-95/01976
- WO-A1-96/11930
- WO-A2-99/58490
- GB-A- 1 440 722
- WILLIAM C. LUMMA, JR. ET AL.: 'Piperazinylpyrazines with central serotoninmimetic activity' JOURNAL OF MEDICINAL CHEMISTRY vol. 21, no. 6, 1978, pages 536 - 542, XP002933691

## Description

### Field of the invention

The present invention relates to novel compounds, to pharmaceutical compositions comprising the compounds, to processes for their preparation, as well as to the use of the compounds for the preparation of a medicament which particularly acts on the central nervous system.

### Background of the invention

Many diseases of the central nervous system are influenced by the adrenergic. the dopaminergic, and the serotonergic neurotransmitter systems. For example, serotonin has been implicated in a number of diseases and conditions which originate in the central nervous system. A number of pharmacological and genetic experiments involving receptors for serotonin strongly implicate the 5-HT_{2c} receptor subtype in the regulation of food intake (Obes. Res. 1995, 3, Suppl. 4, 449S-462S). The 5-HT_{2c} receptor subtype is transcribed and expressed in hypothalamic structures associated with appetite regulation. It has been demonstrated that the non-specific 5-HT_{2c} receptor agonist m-chlorophenylpiperazine (mCPP), which has some preference for the 5-HT_{2c} receptor, causes weight loss in mice that express the normal 5-HT_{2c} receptor while the compound lacks activity in mice expressing the mutated inactive form of the 5-HT_{2c} receptor (Nature 1995, 374, 542-546). In a recent clinical study, a slight but sustained reduction in body weight was obtained after 2 weeks of treatment with mCPP in obese subjects (Psychopharmacology 1997, 133, 309-312). Weight reduction has also been reported from clinical studies with other "serotonergic" agents (see e.g. IDrugs 1998, 1, 456-470). For example, the 5-HT reuptake inhibitor fluoxetine and the 5-HT releasing agent/reuptake inhibitor dexfenfluramine have exhibited weight reduction in controlled studies. However, currently available drugs that increase serotonergic transmission appear to have only a moderate and, in some cases, transient effects on the body weight.

The 5-HT_{2c} receptor subtype has also been suggested to be involved in CNS disorders such as depression and anxiety (Exp. Opin. Invest. Drugs 1998, 7, 1587-1599; IDrugs, 1999, 2, 109-120).

The 5-HT_{2c} receptor subtype has further been suggested to be involved in urinary disorders such as urinary incontinence (IDrugs, 1999, 2, 109-120).

Compounds which have a selective effect on the 5-HT_{2c} receptor may therefore have a therapeutic potential in the treatment of disorders like those mentioned above. Of course, selectivity also reduces the potential for adverse effects mediated by other serotonin receptors.

### Information disclosure

US-A-3,253,989 discloses the use of mCPP as an anorectic agent.

EP-A1-863 136 discloses azetidine and pyrrolidine derivatives which are selective 5-HT_{2c} receptor agonists having antidepressant activity and which can be used for treating or preventing serotonin-related diseases, including eating disorders and anxiety.

EP-A-657 426 discloses tricyclic pyrrole derivatives having activity on the 5-HT_{2c} receptor and which inter alia may be used for treating eating disorders.

EP-A-655 440 discloses 1-aminoethylindoles having activity on the 5-HT_{2c} receptor and which may be used for treating eating disorders.

EP-A-572 863 discloses pyrazinoindoles having activity on the 5-HT_{2c} receptor and which may be used for treating eating disorders.

J. Med. Chem. 1978, 21, 536-542 and US-A-4,081,542 disclose a series of piperazinylpyrazines having central serotonin-mimetic activity. <insert page 2a>

J. Med. Chem. 1981, 24, 93-101 discloses a series of piperazinylquinoxalines with central serotoninmimetic activity.

WO 00/12475 discloses indoline derivatives as 5-HT_{2b} and/or 5-HT_{2c} receptor ligands, especially for the treatment of obesity.

WO 00/12510 discloses pyrroloindoles, pyridoindoles and azepinoindoles as 5-HT_{2c} receptor agonists, particluarly for the treatment of obesity.

WO 00/12482 discloses indazole derivatives as selective, directly active 5-HT_{2c} receptor ligands, preferably 5-HT_{2c} receptor agonists, particularly for use as anti-obesity agents.

WO 00/12502 discloses pyrroloquinolines as 5-HT_{2c} receptor agonists, particularly for use as anti-obesity agents.

However, the affinity towards the 5-HT_{2c} receptor of the present compounds is considerably higher compared to J. Med. Chem. 1978, 21, 536-542.

WO 00/12475 discloses indoline derivatives as 5-HT_{2b} and/or 5-HT_{2c} receptor ligands, especially for the treatment of obesity.

GB-B-1,457,005 discloses 1-piperazinyl-2-[2-(phenyl)ethenyl]-quinoxaline derivatives which exhibit anti-inflammatory activity.

Chem. Pharm. Bull. 1993, 41(10) 1832-1841 discloses 5-HT₃ antagonists including 2-(4-methyl-1-piperazinyl)-4-phenoxyquinoxaline.

GB-B-1,440,722 discloses 2-(1'-piperazinyl)-quinoxaline compounds having pharmaceutical activity against depression.

WO 96/11920 discloses CNS-active pyridinylurea derivatives.

WO 95/01976 discloses indoline derivatives active as 5-HT_{2c} antagonists and of potential use in the treatment of CNS disorders.

WO 97/14689 discloses aryl-piperazine cyclic amine derivatives which are selective 5-HT_{1d} receptor antagonists.

WO 98/42692 discloses piperazines derived from cyclic amines which are selective antagonists of human 5-HT₁ₐ, 5-HT_{1d} and 5-HT_{1b} receptors.

GB-B-1,465,946 discloses substituted pyridazinyl, pyrimidinyl and pyridyl compounds which are active as β-receptor blocking agents.

EP-A-711757 discloses [3-(4-phenyl-piperazin-1-yl)propylamino]-pyridine, pyrimidine and benzene derivatives as α-adrenoceptor antagonists.

WO 99/03833 discloses aryl-piperazine derivatives which are 5-HT₂ antagonists and 5-HT₁ₐ receptor agonists and therefore are useful as remedies or preventives for psychoneurosis.

WO 96/02525 discloses aryl-piperazine-derived piperazide derivatives having 5-HT receptor antagonistic activity.

WO 99/58490 disloses aryl-hydronaphthalen-alkanamines which may effectuate partial or complete blockage of serotonergic 5-HT_{2c} receptors in an organism.

### Summary of the invention

According to the present invention, a class of novel compounds have been developed which bind to the 5-HT_{2c} receptor (agonists and antagonists) and which therefore may be used for the treatment of serotonin-related disorders.

In one aspect, the invention provides a compound of the general formula (Ib): 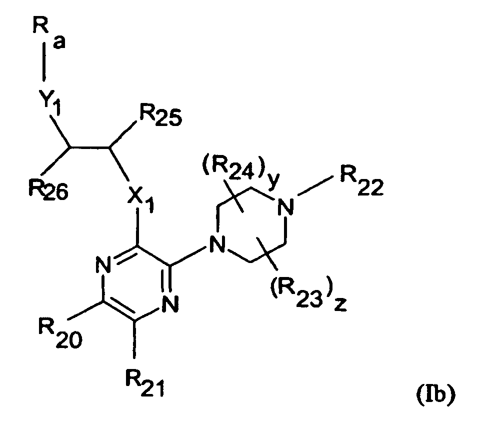 wherein:
X₁ is independently -O-, -S- or -N(R₂₇)-;
Y₁ is independently -O-, -S- or -N(R₂₇)-;
Rₐ is aryl or heteroaryl, each of which may be unsubstituted or substituted in one or more positions independently of each other by C₁₋₆-alkyl, C₁₋₆-alkoxy, fluoro-C₁₋₆-alkoxy, 2,2,2-trifluoroethoxy, C₃₋₅-alkynyloxy, C₃₋₅-alkenyloxy, dimethylamino-C₁₋₆-alkoxy, methylamino-C₁₋₆-alkoxy, C₁₋₆-alkylthio, fluoromethyl, difluoromethyl, trifluoromethyl, fluoromethylthio, difluoromethoxy, difluoromethylthio, trifluoromethoxy, trifluoromethylthio, halogen, hydroxy, nitro, cyano, trifluoromethylsulphonyloxy, C₁₋₆-alkylsulphonamido, C₂₋₆-alkenyl, C₂₋₆-alkynyl, C₁₋₆-acyl, C₁₋₆-alkylcarbonyl-C₁₋₆-alkyl, C₁₋₆-alkylsulphonyl, C₁₋₆-alkylsulphonyloxy, C₁₋₆-alkoxy-C₁₋₆-alkyl, C₁₋₆-alkoxycarbonyl, C₁₋₆-alkoxycarbonyl-C₁₋₆-alkyl, C₁₋₆-acyloxy-C₁₋₆-alkyl, hydroxy-C₁₋₆-alkyl, hydroxy-C₁₋₆-alkoxy, C₁₋₆-alkoxy-C₁₋₆-alkoxy, C₁₋₆-alkoxy-C₁₋₆-alkylthio, hydroxy-C₁₋₆-alkylthio, heteroaryl-C₁₋₆-alkylthio, aryl-C₁₋₆-alkylthio, C₁₋₆-alkoxy-C₁₋₆-alkylamino, N-(C₁₋₆-allcoxy-C₁₋₆-alkyl)-N-methylamino, C₃₋₆-cycloalkyl-C₁₋₆-alkoxy, aryl-C₁₋₆-alkoxy, heterocyclyl-C₁₋₆-alkoxy, C₃₋₈-cycloalkyl, C₃₋₈-cycloalkyloxy, aryl, aryloxy, arylthio, arylsulphonyl, aryl-C₁₋₆-acyl, aryl-C₁₋₆-alkyl, aryl-C₂₋₆-alkenyl, aryl-C₂₋₆-alkynyl, heterocyclyl-C₁₋₆-alkyl, heteroclcyl-C₂₋₆-alkenyl, heterocyclyl-C₂₋₆-alkynyl. heterocyclyl, heterocyclyloxy, heterocyclylthio, heterocyclylsulphonyl, heterocyclylamino, heterocyclyl-C₁₋₆-acyl, -N(R₂)(R₃), -CON(R₇)(R₈),
   wherein any cycloalkyl, aryl and heterocyclyl residues as substituents on aryl or heteroaryl or as part of substituents on aryl or heteroaryl in turn may be substituted in one or more positions independently of each other by C₁₋₄-alkyl, C₁₋₄-alkoxy, methanesulphonamido, C₁₋₄-alkylthio, C₁₋₄-alkylsulphonyl, C₁₋₄-acyl, heterocyclyl, heterocyclyloxy, heterocyclylthio, aryloxy, arylthio, fluoromethyl, trifluoromethyl, trifluoromethoxy, trifluoromethylthio, halogen, hydroxy, nitro, cyano, N(R₂)(R₃) or, for C₃₋₈-cycloalkyl and partially or fully saturated heterocyclyl, oxo or hydroxy;
R₂₀ and R₂₁ independently of each other are hydrogen, C₁₋₄-alkyl (preferably methyl), C₁₋₄-alkoxy (preferably methoxy), C₁₋₄-alkylthio (preferably methylthio), halogen, amino, methylamino, dimethylamino, acetamido, phenyl, phenoxy or phenylthio, wherein phenyl, phenoxy and phenylthio may be unsubstituted or substituted in one or more positions (preferably mono- or disubstituted) independently of each other by halogen, methyl, methoxy, methylthio, cyano, hydroxy or trifluoromethyl, or R₂₀ and R₂₁ together with the carbon atoms to which they are bound form a 5- or 6-membered aromatic or heteroaromatic ring, which optionally is independently substituted in one or more positions by halogen, methyl, methoxy, methylthio, methylsulphonyl, nitro, cyano, hydroxy, trifluoromethyl, trifluoromethylthio, amino, methylamino, dimethylamino or acetamido;
R₂₂ is hydrogen, hydroxy, C₁₋₄-alkyl, C₃₋₄-alkenyl, C₁₋₄-acyl, C₁₋₄-alkoxycarbonyl, 2-hydroxyethyl, 2-cyanoethyl or tetrahydropyran-2-yl;
R₂₃ and R₂₄ independently of each other are hydrogen, C₁₋₄-alkyl, hydroxymethyl, C₁₋₄-alkoxymethyl or fluoromethyl;
R₂₅ is hydrogen or C₁₋₄-alkyl;
R₂₆ is hydrogen, C₁₋₄-alkyl or is linked to a carbon atom in Rₐ adjacent to the atom binding to Y₁ to form a 5- or 6-membered ring which may contain an additional heteroatom,
R₂₇ is hydrogen or C₁₋₄-alkyl, preferably methyl or ethyl; and
y and z independently of each other are 1 or 2,
and pharmaceutically acceptable salts, hydrates, geometrical isomers, tautomers, optical isomers, and N-oxides thereof.

R₂ and R₃ independently of each other are hydrogen, C₁₋₆-alkyl, C₁₋₆-acyl, -CON(R₇)(R₈), aryl, heterocyclyl, aryl-C₁₋₆-alkyl, heterocyclyl-C₁₋₆-alkyl, aryl-C₁₋₆-acyl or heterocyclyl-C₁₋₆-acyl, wherein any aryl and heterocyclyl residues in turn may be substituted in one or more positions independently of each other by halogen, C₁₋₄-alkyl, C₁₋₄-alkoxy, C₁₋₄-alkylthio, C₁₋₄-alkylsulphonyl, methanesulphonamido, nitro, cyano, hydroxy, trifluoromethyl, trifluoromethoxy, trifluoromethylthio or -N(R₂)(R₃); or R₂ and R₃ together with the nitrogen atom to which they are bound form a saturated heterocyclic ring having 4-7 ring members and optionally containing a further heteroatom, which ring may be substituted by C₁₋₆-alkyl, C₁₋₆-alkoxy, oxo or hydroxy.

R₇ and R₈ independently of each other are hydrogen, C₁₋₆-alkyl, aryl, heteroaryl, aryl-C₁₋₄-alkyl or heteroaryl-C₁₋₄-alkyl, wherein aryl and heteroaryl residues in turn may be substituted in one or more positions independently of each other by halogen, C₁₋₆-alkyl, C₁₋₆-alkoxy, C₁₋₆-alkylthio, C₁₋₆-alkylsulphonyl, methanesulphonamido, nitro, cyano, hydroxy, trifluoromethyl, trifluoromethoxy, trifluoromethylthio or -N(R₂)(R₃); or R₇ and R₈ together with the nitrogen atom to which they are bound form a saturated heterocyclic ring having 4-7 ring members and optionally containing a further heteroatom.

In case the compounds of formula (Ib) can be in the form of optical isomers, the invention comprises the racemic mixture as well as the individual enantiomers as such.

In case the compounds of formula (Ib) contain groups which may exist in tautomeric forms, the invention comprises the tautomeric forms of the compounds as well as mixtures thereof, e.g. a 2-hydroxypyridine and its tautomer 1H-2-pyridone.

In case the compounds of formula (Ib) can be in the form of geometrical isomers, the invention comprises the geometrical isomers as well as mixtures thereof.

In another aspect, the invention provides the compounds according to formula (Ib) above for use in therapy of a human being or an animal.

It is preferred that the therapy is directed towards prophylaxis or treatment of a serotonin-related disease related to the 5-HT_{2c} receptor.

It is preferred that the serotonin-related disease is selected from eating disorders, especially obesity, memory disorders, mood disorders, comprising major depression and bipolar depression including both mild and bipolar disorder, seasonal affective disorder (SAD), anxiety disorders, including situational anxiety, generalized anxiety disorder, primary anxiety disorders, secondary anxiety disorders, sexual dysfunctions, urinary disorders, pain, and schizophrenia.

Another aspect of the invention provides the use of the compounds according to formula (I) above for the manufacture of a medicament for treating or preventing a serotonin-related disease, particularly 5-HT_{2c} receptor-related.

It is preferred that the serotonin-related disease is selected from eating disorders, especially obesity, memory disorders, schizophrenia, mood disorders, anxiety disorders, pain, sexual dysfunctions, and urinary disorders.

Another aspect of the invention provides a pharmaceutical composition comprising a compound according to formula (Ib) as an active ingredient, together with a pharmacologically and pharmaceutically acceptable carrier.

Another aspect of the invention provides a method for modulating 5-HT_{2c} receptor function, comprising contacting the receptor with an effective inhibitory amount of a compound according to formula (Ib).

Another aspect of the invention provides a process for the preparation of a compound of formula (Ib), which process comprises:
reacting a compound of formula (IIIa): 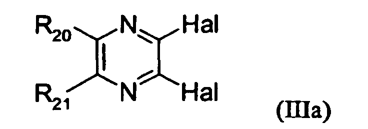
wherein R₂₀ and R₂₁ are as defined for formula (Ib), and Hal is halogen, with a compound of formula (IIIb): 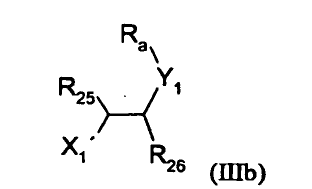 wherein X₁' is selected from -OH, -SH, or -NH(R₂₇), and R₂₅, R₂₆, R₂₇, Y₁, and Rₐ are as defined for formula (Ib), in the presence of a base such as potassium- or sodium *tert*-butoxide or an alkali metal hydride such as sodium hydride or potassium hydride in a suitable solvent such as THF, dioxane or DMF to produce a compound of formula (IIIc): 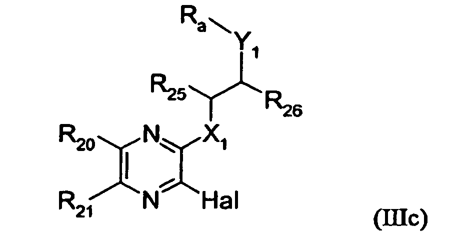 wherein R₂₀, R₂₁, R₂₅, R₂₆, Hal, X₁, Y₁, and Rₐ are as defined above, and subsequently reacting the compound of formula (IIIc) with an appropriate piperazine derivative of formula (IIId): 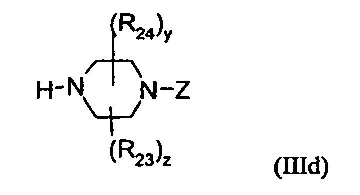 wherein
R_{23,} R_{24,} y and z are as defined for formula (Ib) to produce the compound of formula (Ib); and
Z is as defined for R₂₂ in formula (Ib) or is a suitable protecting group such as *tert*-butoxycarbonyl, trityl or benzyl.

Another aspect of the invention provides a process for the preparation of a compound of formula (Ib), wherein Y₁ is selected from oxygen or sulphur, which process comprises:
reacting a compound of formula (IIIe): 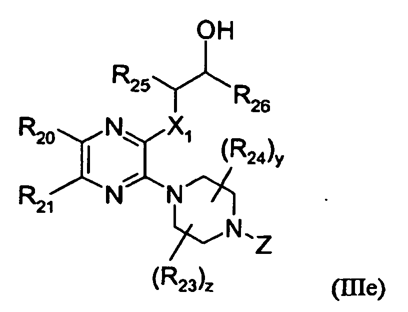
wherein
X₁ is selected from oxygen or sulfur,
R₂₀, R₂₁, R₂₃, R₂₄, y and z are as defined for formula (Ib),
R₂₅ and R₂₆ are each independently hydrogen or methyl, preferably hydrogen; and
Z is as defined for R₂₂ in formula (Ib) or is a suitable protecting group such as *tert*-butoxycarbonyl, trityl or benzyl; with an appropriate phenol or thiophenol corresponding to Rₐ in formula (Ib) under Mitsunobu conditions, e.g., in the presence of diethyl azodicarboxylate (DEAD) or 1,1'-azobis(*N*,*N*-dimethylformanide) (TMAD); and triphenylphosphine or tri-n-butylphosphine; in a suitable solvent such as tetrahydrofuran (THF) to produce the compound of formula (Ib).

Another aspect of the invention provides a process for the preparation of a compound of formula (Ib) by reacting a compound of formula (IIIa): 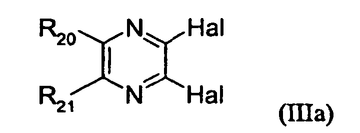 wherein R₂₀ and R₂₁ are as defined for formula (Ib) and Hal is halogen, with a piperazine derivative of formula (IIId): 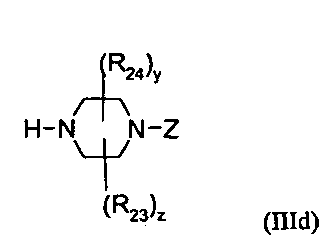 wherein R₂₃, R₂₄, y and z are as defined for formula (Ib), and
Z is as defined for R₂₂ in formula (Ib) or is a suitable protecting group such as *tert*-butoxycarbonyl, trityl or benzyl; to produce a compound of formula (IIIf): 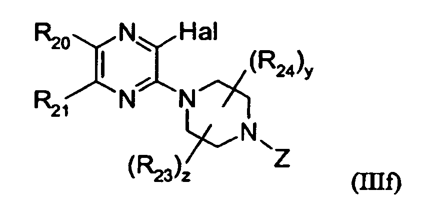 wherein Z, R₂₀, R₂₁, R₂₃, R₂₄, Hal, y and z are as defined above, and subsequently reacting the compound of formula (IIIf) with a compound of formula (IIIb): 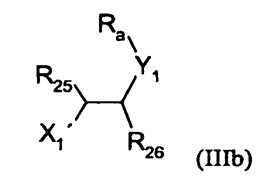 wherein X₁' is selected from -OH, -SH, or -NH(R₂₇) and R₂₅, R₂₆, R₂₇, Y₁, and Rₐ are as defined for formula (Ib), in the presence of a base such as potassium- or sodium *tert*-butoxide or an alkali metal hydride such as sodium hydride or potassium hydride in a suitable solvent such as THF, dioxane or DMF to produce a compound of formula (Ib).

Another aspect of the invention provides a process for the preparation of a compound of formula (Ib) by any one of
(a) converting a resulting compound of formula (Ib) to another compound of formula (Ib);
(b) isolating an isomer of a compound of formula (Ib) from a mixture (e.g., a racemic mixture) thereof; or
(c) converting the free base of a compound of formula (Ib) to a salt thereof.

Another aspect of the invention provides a process for the preparation of compounds of formula (Ib) where R₂₂ is H, wherein Z in the corresponding intermediate of formula (IIId), (IIIe) or (IIIf) is a protecting group selected from *tert*- butoxycarbonyl (*t*-BOC), benzyl or trityl.

Another aspect of the invention provides a process where the said protecting group is cleaved off to produce a compound of formula (Ib).

Another aspect of the invention provides a process, wherein the intermediate of formula (IIIe) is 2-[3-(4-*tert*-butoxycarbonyl-1-piperazinyl)-2-pyrazinyloxy]ethanol.

### Detailed description of the invention

First, the various terms used, separately and in combinations, in the above definition of the compounds having the general formula (I) will be explained.

By "heteroatom" is meant nitrogen, oxygen, sulphur, and in heterocyclic rings (including heteroaromatic as well as saturated and partially saturated heterocyclic rings), also selenium.

The term "aryl" is intended to include aromatic rings (monocyclic or bicyclic) having from 6 to 10 ring carbon atoms, such as phenyl, naphthyl and 1,2,3,4-tetrahydronaphthyl (substitutions may be in any ring).

The term "heteroaryl" means a monocyclic, bi- or tricyclic aromatic ring system (only one ring need to be aromatic, and substitutions may be in any ring) having from 5 to 14, preferably 5 to 10 ring atoms (mono- or bicyclic), in which one or more of the ring atoms are other than carbon, such as nitrogen, sulphur, oxygen and selenium. Examples of such heteroaryl rings are pyrrole, imidazole, thiophene, furan, thiazole, isothiazole, thiadiazole, oxazole, isoxazole, oxadiazole, pyridine, pyrazine, pyrimidine, pyridazine, pyrazole, triazole, tetrazole, chroman, isochroman, coumarin, quinoline, quinoxaline, isoquinoline, phthalazine, cinnoline, quinazoline, indole, isoindole, indoline, isoindoline, benzothiophene, benzofuran, 2,3-dihydrobenzofuran, isobenzofuran, benzoxazole, 2,1,3-benzoxadiazole, benzothiazole, 2,1,3-benzothiadiazole, 2,1,3-benzoselenadiazole, benzimidazole, indazole, 2,3-dihydro-1,4-benzodioxine, indane, 1,3-benzodioxole, 1,2,3,4-tetrahydroquinoline, 3,4-dihydro-2*H*-1,4-benzoxazine, 1,5-naphthyridine, 1,8-naphthyridine, acridine, fenazine, xanthene, 3,4-dihydro-2H-pyrido[3,2-b]-1,4-oxazine, and 2,3-dihydro-1,4-benzoxathiine. The heteroaryl ring may be linked to the divalent group A in formula (I) via a carbon or nitrogen atom thereof. If a bi- or tricyclic ring is substituted, it may be substituted in any ring.

The term "heteroalkylene" means an alkylene group which contains a terminal heteroatom at one or both ends and/or one or more carbon chain-interrupting heteroatom(s) selected from N, O and S. The number of heteroatoms is at least one, and usually from one to three, especially one or two. When heteroalkylene is substituted, it is usually substituted at a carbon atom by C₁₋₄-alkyl or oxo, but it may alternatively or additionally be substituted at a nitrogen by C₁₋₄-alkyl or at a sulphur atom by oxo (S=O or O=S=O), if present.

The term "heterocyclyl" is intended to include fully unsaturated (i.e. aromatic) as well as partially and fully saturated mono-, bi- and tricyclic rings having from 4 to 14, preferably 4 to 10 ring atoms, and containing one or more heteroatoms selected from oxygen, sulphur and nitrogen, such as, for example, the heteroaryl groups mentioned above as well as their corresponding partially saturated or fully saturated heterocyclic rings. Exemplary saturated heterocyclic rings are azetidine, pyrrolidine, piperidine, piperazine, morpholine and thiomorpholine.

C₁₋₆-alkyl, which may be straight or branched, is preferably C₁₋₄-alkyl. Exemplary alkyl groups include methyl, ethyl, propyl, isopropyl, butyl, sec-butyl, tert-butyl, pentyl, isopentyl, hexyl, and isohexyl.

C₁₋₆-alkoxy, which may be straight or branched, is preferably C₁₋₄-alkoxy. Exemplary alkoxy groups include methoxy, ethoxy, propoxy, isopropoxy, butoxy, sec-butoxy, tert-butoxy, pentyloxy, isopentyloxy, hexyloxy, and isohexyloxy.

C₂₋₆-alkenyl, which may be straight or branched, is preferably C₂₋₄-alkenyl, e.g. 1-propenyl, 2-propenyl, vinyl.

C₂₋₆-alkynyl, which may be straight or branched, is preferably C₂₋₄-alkynyl, e.g. propargyl, ethynyl.

C₃₋₈-cycloalkyl is preferably C₄₋₇-cycloalkyl. Exemplary cycloalkyl groups include cyclopropyl, cyclobutyl, cyclopentyl, cyclohexyl, cycloheptyl and cyclooctyl.

C₁₋₆-acyl may be saturated or unsaturated and is preferably C₁₋₄-acyl. Exemplary acyl groups include formyl, acetyl, propionyl, butyryl, isobutyryl, valeryl, isovaleryl, butenoyl (e.g. 3-butenoyl), hexenoyl (e.g. 5-hexenoyl).

C₁₋₈-alkylene (wherein 1-8 is the number of chain carbon atoms) and heteroalkylene having 2 to 8 chain atoms, which may contain one or more unsaturations (double and/or triple bonds), are preferably C₁₋₄-alkylene and heteroalkylene of 2 to 4 chain atoms, respectively. Exemplary alkylene groups include methylene, ethylene, propylene, butylene and their isomers (e.g. 1,3-butylene, 2-methyl-1,3-propylene). Exemplary heteroalkylene groups include oxymethylene (and methylenoxy),
oxyethylene (and ethylenoxy), oxypropylene (and propylenoxy), oxybutylene (and butylenoxy), ethylenedioxy, propylenedioxy, butylenedioxy, oxyallyl (and allyloxy), etc. An exemplary alkyl-substituted heteroalkylene group is methylethylenedioxy.

Hydroxy-C₁₋₆-alkyl may be straight or branched. Exemplary hydroxyalkyl groups include hydroxymethyl, 1-hydroxyethyl, 2-hydroxyethyl, 2-hydroxypropyl, 3-hydroxypropyl, 2-hydroxybutyl, 3-hydroxybutyl, 4-hydroxybutyl.

Exemplary aryl-C₁₋₆-acyl groups include benzoyl, 1-naphthoyl, 2-naphthoyl, cinnamoyl and phenylacetyl.

Exemplary C₁₋₆-alkylcarbonyl-C₁₋₆-alkyl groups include 2-oxobutyl.

Exemplary C₁₋₆-alkoxy-C₁₋₆-alkyl groups include 2-ethoxyethyl.

Exemplary C₁₋₆-alkoxycarbonyl-C₁₋₆-alkyl groups include ethoxycarbonylbutyl.

Exemplary C₁₋₆-acyloxy-C₁₋₆-alkyl groups include propanoyloxypropyl.

Exemplary saturated and unsaturated (partially and fully) azacyclic and saturated azabicyclic rings include azetidine, pyrrolidine, piperidine, morpholine, hexahydroazepine, tetrahydropyridine, pyridine and 1-azabicyclo[2.2.2]octane. The azacyclic and azabicyclic rings are coupled via a ring carbon atom when n = 0.

Exemplary saturated and unsaturated aminoazacyclic rings include aminopiperidine (e.g. 4-aminopiperidine), aminoazetidine (e.g. 3-aminoazetidine), aminopyrrolidine (e.g. 3-aminopyrrolidine), and aminopyridine (e.g. 4-aminopyridine). The aminoazacyclic rings are preferably coupled to B or Ar in formula (I) via either the aza nitrogen atom or the amino nitrogen atom. An example of a saturated aminoazabicyclic ring is 3-aminoazabicyclo[2.2.2]octane, and the coupling is preferably via the 3-amino nitrogen atom.

An example of a saturated aminodiazacyclic ring is 1-aminopiperazine, and the coupling is preferably via either the 4-aza nitrogen atom or the 1-amino nitrogen atom.

Exemplary saturated diazacyclic rings include piperazine and homopiperazine, and an example of a diazabicyclic ring is diazabicyclo[2.2.1]heptane. The diazacyclic and diazabicyclic rings are preferably coupled via one of the ring nitrogens.

Halogen includes fluorine, chlorine, bromine and iodine.

Where it is stated above that aryl, heteroaryl and heterocyclyl residues may be substituted, this applies to aryl, heteroaryl and heterocyclyl *per se* as well as to any combined groups containing aryl, heteroaryl or heterocyclyl residues, such as aryl-C₁₋₆-acyl, heteroaryl-C₂₋₄-alkenyl, heterocyclylthio, etc.

The term "N-oxides" means that one or more nitrogen atoms, when present in a compound, are in N-oxide form (N→O).

"Pharmaceutically acceptable" means being useful in preparing a pharmaceutical composition that is generally safe, non-toxic and neither biologically nor otherwise undesirable and includes being useful for veterinary use as well as human pharmaceutical use.

"Pharmaceutically acceptable salts" mean salts which are pharmaceutically acceptable, as defined above, and which possess the desired pharmacological activity. Such salts include acid addition salts formed with organic and inorganic acids, such as hydrogen chloride, hydrogen bromide, hydrogen iodide, sulfuric acid, phosphoric acid, acetic acid, glycolic acid, maleic acid, malonic acid, oxalic acid, toluenesulphonic acid, methanesulphonic acid, trifluoroacetic acid, fumaric acid, succinic acid, tartaric acid, citric acid, benzoic acid, ascorbic acid and the like.

In the compound of the general formula (Ib), it is preferred that R₂₀ and R₂₁ independently of each other are hydrogen, halogen or C₁₋₄-alkyl.

In the compound of the general formula (Ib), it is preferred that R₂₀ and R₂₁ form a ring together with the ring carbons to which they are bound.

In the compound of the general formula (Ib), it is preferred that R₂₀ and R₂₁ together with the carbon atoms to which they are bound form benzene, to form a quinoxaline ring, optionally mono- or di-substituted by halogen, or thiophene, to form a thieno[3,4-b]pyrazine ring.

In the compound of the general formula (Ib), it is preferred that X₁ and Y₁ both are -O-.

In the compound of the general formula (Ib), it is preferred that X₁ is -S- and Y₁ is -O-.

In the compound of the general formula (Ib), it is preferred that X₁ is -O- and Y₁ is -S-.

In the compound of the general formula (Ib), it is preferred that X₁ and Y₁ both are -S-.

In the compound of the general formula (Ib), it is preferred that R₂₂ is hydrogen.

In the compound of the general formula (Ib), it is preferred that R₂₃ is methyl, z is 1 and R₂₄ is hydrogen, preferably in (R)-configuration.

In the compound of the general formula (Ib), it is preferred that R₂₅ is hydrogen and R₂₆ is methyl.

In the compound of the general formula (Ib), it is preferred that R₂₅ is hydrogen.

Preferred compounds are:
2-(2-Phenoxy)ethyl 3-(1-piperazinyl)-2-pyrazinyl ether
2-(2-Fluorophenoxy)ethyl 3-(1-piperazinyl)-2-pyrazinyl ether
2-(3-Cyanophenoxy)ethyl 3-(1-piperazinyl)-2-pyrazinyl ether
2-(3-Methoxyphenoxy)ethyl 3-(1-piperazinyl)-2-pyrazinyl ether
2-[3-(2-hydroxyethoxy)phenoxy]ethyl 3-(1-piperazinyl)-2-pyrazinyl ether
2,3-Dihydro-1,4-benzodioxin-2-ylmethyl 3-(1-piperazinyl)-2-pyrazinyl ether
2-(2-Methoxyphenoxy)ethyl 3-(1-piperazinyl)-2-pyrazinyl ether
2-(2,5-Difluorophenoxy)ethyl 3-(1-piperazinyl)-2-pyrazinyl ether
2-(3,5-Dimethoxyphenoxy)ethyl 3-(1-piperazinyl)-2-pyrazinyl ether
2-(3,4-Dihydro-2*H*-pyrido[3,2-*b*]-1,4-oxazin-2-ylmethoxy)-3-(1-piperazinyl)pyrazine
2-Methyl-1-[3-(2-phenoxyethoxy)-2-pyrazinyl]piperazine, particularly the (*R*)-enantiomer thereof
2-Methyl-1-{3-(2-(3-pyridinyloxy)ethoxy]-2-pyrazinyl}piperazine, particularly the (*R*)-enantiomer thereof
2-(Quinazolinyl-8-yloxy)ethyl 3-(1-piperazinyl)-2-pyrazinyl ether
2-(Isoquinolinyl-5-yloxy)ethyl 3-(1-piperazinyl)-2-pyrazinyl ether
2-[2-(3-Pyridinyloxy)ethoxy]-3-(1-piperazinyl)-6,7-difluoroquinoxaline
2-[2-(3-Pyridinyloxy)ethoxy]-3-(1-piperazinyl)thieno[3,4-*b*]pyrazine
2-(3,4-Dihydro-2*H*-1,4-benzoxazin-2-ylmethoxy)-3-(1-piperazinyl)pyrazine
2-Methyl-1-{3-[2-(2-amino-8-quinolinyloxy)ethoxy]-2-pyrazinyl}piperazine, particularly the (*R*)-enantiomer thereof
1-{3-[2-(2-Methoxy-3-pyridinyloxy)ethoxy]-2-pyrazinyl}piperazine
2-Methyl-1-{3-[2-(2-methoxy-3-pyridinyloxy)ethoxy]-2-pyrazinyl}piperazine, particularly the (*R*)-enantiomer thereof,
2-{2-[(2-Chloro-3-pyridinyl)oxy]ethoxy}-3-(1-piperazinyl)pyrazine,
2-{2-[(2-Ethoxy-3-pyridinyl)oxy]ethoxy}-3-(1-piperazinyl)pyrazine,
1-(3-{2-[(2-Ethoxy-3-pyridinyl)oxy]ethoxy}-2-pyrazinyl)-2-methylpiperazine, particularly the (*R*)-enantiomer thereof,
2-(2-{[2-(Methylsulfanyl)-3-pyridinyl]oxy} ethoxy)-3-(1-piperazinyl)pyrazine,
2-Methyl-1-[3-(2-{[2-(methylsulfanyl)-3-pyridinyl]oxy}ethoxy)-2-pyrazinyl]piperazine, particularly the (*R*)-enantiomer thereof,
2-{2-[(2-Bromo-3-pyridinyl)oxy]ethoxy}-3-(1-piperazinyl)pyrazine,
1-(3-{2-[(2-Bromo-3-pyridinyl)oxy]ethoxy}-2-pyrazinyl)-2-methylpiperazine, particularly the (*R*)-enantiomer thereof,
2-(1-Piperazinyl)-3-(2-{3-[2-(2-pyridinyl)ethoxy]phenoxy}ethoxy)pyrazine,
2-(2-{3-[2-(4-Methyl-1,3-thiazol-5-yl)ethoxy]phenoxy}ethoxy)-3-(1-piperazinyl)pyrazine,
2-(1-Piperazinyl)-3-{2-[3-(tetrahydro-3-furanylmethoxy)phenoxy]ethoxy}pyrazine,
2-(1-Piperazinyl)-3-{2-[3-(tetrahydro-3-furanyloxy)phenoxy]ethoxy}pyrazine,
1-{2-[3-(2-{[3-(1-Piperazinyl)-2-pyrazinyl]oxy}ethoxy)phenoxy]ethyl}-2-pyrrolidinone,
2-{2-[3-(2-Methoxyethoxy)phenoxy]ethoxy}-3-(1-piperazinyl)pyrazine,
2-{[3-(2- {[3-(1-Piperazinyl)-2-pyrazinyl]oxy}ethoxy)phenoxy]methyl}-benzonitrile,
2-(1-Piperazinyl)-3-{2-[3-(tetrahydro-2H-pyran-4-yloxy)phenoxy]ethoxy}-pyrazine,
N,N-Dimethyl-N-{2-[3-(2-{[3-(1-piperazinyl)-2-pyrazinyl]oxy}ethoxy)-phenoxy]-ethyl}amine,
7-(2-{[3-(1-Piperazinyl)-2-pyrazinyl]oxy}ethoxy)-2H-chromen-2-one,
1-(3-{2-[(2-Chloro-3-pyridinyl)oxy]ethoxy}-2-pyrazinyl)-2-methylpiperazine, particularly the (R)-enantiomer thereof,
7-Isoquinolinyl 2-{[3-(1-piperazinyl)]-2-pyrazinyl}oxy}ethyl ether,
2-(2-Chloro-4-methoxyphenoxy)ethyl 3-(1-piperazinyl)-2-pyrazinyl ether,
4-(2-{[3-(1-Piperazinyl)-2-pyrazinyl]oxy}ethoxy)-2-quinolinamine,
or a pharmacologically acceptable salt or solvate thereof.

An obtained compound of formula (Ib) may be converted to another compound of formula (Ib) by methods well-known in the art (illustrated in e.g. Example 216).

The processes described above may be carried out to give a compound of the invention in the form of a free base or as an acid addition salt. A pharmaceutically acceptable acid addition salt may be obtained by dissolving the free base in a suitable organic solvent and treating the solution with an acid, in accordance with conventional procedures for preparing acid addition salts from base compounds. Examples of addition salt forming acids are maleic acid, fumaric acid, succinic acid, methanesulfonic acid, trifluoroacetic acid, acetic acid, oxalic acid, benzoic acid, hydrochloric acid, sulphuric acid, phosphoric acid, and the like.

The compounds of formula (Ib) may possess one or more chiral carbon atoms, and they may therefore be obtained in the form of optical isomers, e.g. as a pure enantiomer, or as a mixture of enantiomers (racemate) or as a mixture containing diastereomers. The separation of mixtures of optical isomers to obtain pure enantiomers is well known in the art and may, for example, be achieved by fractional crystallization of salts with optically active (chiral) acids or by chromatographic separation on chiral columns.

The necessary starting materials for preparing the compounds of formula (Ib) are either known or may be prepared in analogy with the preparation of known compounds. For example, the aryloxy- and heteroaryloxyethanols used in the preparation of the novel compounds of formula (Ib) may be prepared using the methods depicted in Scheme 1 below.

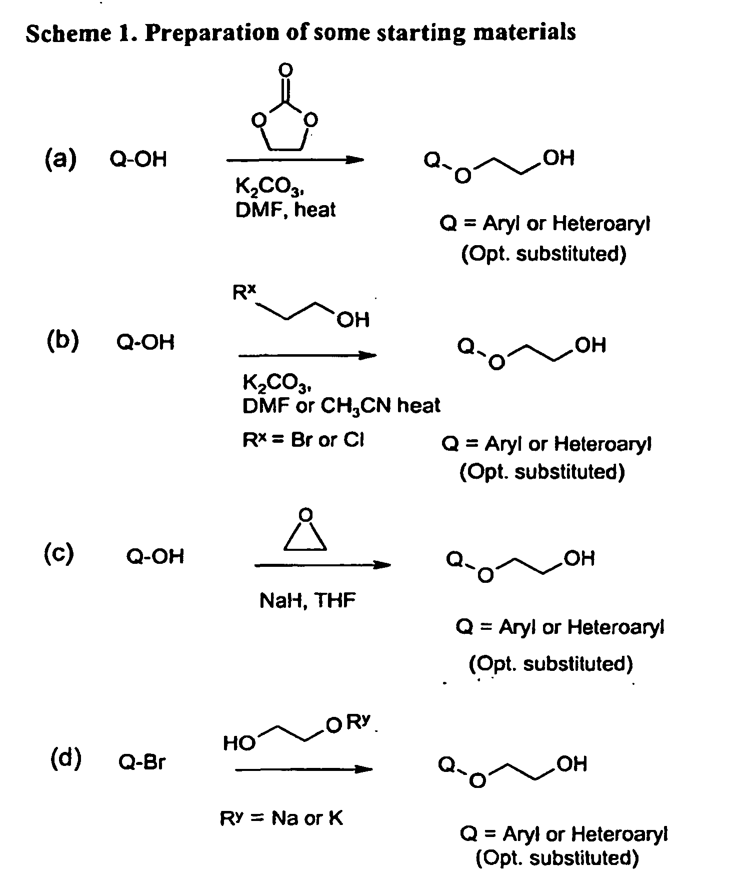

In accordance with the present invention, the compounds of formula (Ib), in the form of free bases or salts with physiologically acceptable acids, can be brought into suitable galenic forms, such as compositions for oral use, for injection, for nasal spray administration or the like, in accordance with accepted pharmaceutical procedures. Such pharmaceutical compositions according to the invention comprise an effective amount of the compounds of formula (Ib) in association with compatible pharmaceutically acceptable carrier materials, or diluents, as are well known in the art. The carriers may be any inert material, organic or inorganic, suitable for enteral, percutaneous, subcutaneous or parenteral administration, such as: water, gelatin, gum arabicum, lactose, microcrystalline cellulose, starch, sodium starch glycolate, calcium hydrogen phosphate, magnesium stearate, talcum, colloidal silicon dioxide, and the like. Such compositions may also contain other pharmacologically active agents, and conventional additives, such as stabilizers, wetting agents, emulsifiers, flavouring agents, buffers, and the like.

The compositions according to the invention can e.g. be made up in solid or liquid form for oral administration, such as tablets, pills, capsules, powders, syrups, elixirs, dispersable granules, cachets, suppositories and the like, in the form of sterile solutions, suspensions or emulsions for parenteral administration, sprays, e.g. a nasal spray, transdermal preparations, e.g. patches, and the like.

As mentioned above, the compounds of the invention may be used for the treatment of serotonin-related disorders in a human being or an animal, such as eating disorders, particularly obesity, memory disorders, schizophrenia, mood disorders, anxiety disorders, pain, sexual dysfunctions, and urinary disorders. The compounds may also be useful for treating gastrointestinal disorders, such as gastrointestinal mobility disorders, e.g. irritable bowel syndrome (IBS), or glaucoma. The dose level and frequency of dosage of the specific compound will vary depending on a variety of factors including the potency of the specific compound employed, the metabolic stability and length of action of that compound, the patient's age, body weight, general health, sex, diet, mode and time of administration, rate of excretion, drug combination, the severity of the condition to be treated, and the patient undergoing therapy. The daily dosage may, for example, range from about 0.001 mg to about 100 mg per kilo of body weight, administered singly or multiply in doses, e.g. from about 0.01 mg to about 25 mg each. Normally, such a dosage is given orally but parenteral administration may also be chosen.

The invention will now be further illustrated by the following non-limiting Examples.

### EXAMPLES

### General:

The structures of the prepared compounds were confirmed by standard spectroscopical methods, and elemental analysis and/or high resolution MS. The NMR data were obtained on a JEOL JNM-EX 270, a Bruker 400 DPX or a Bruker DRX 500 spectrometer. IR spectra were obtained on a Perkin Elmer SPECTRUM 1000 FT-IR spectrometer. High resolution MS were obtained on a Micromass LCT spectrometer. Elemental analysis was performed by Mikro Kemi AB, Uppsala, Sweden. Melting points, when given, were obtained on a Büchi or a Gallenkamp melting point apparatus and are uncorrected.

### EXAMPLE 1

### 2-(2-Phenoxy)ethyl 3-(1-piperazinyl)-2-pyrazinyl ether, Maleate.

### Step 1: 2-Chloro-3-(2-phenoxyethoxy)pyrazine.

NaO-*t*-Bu (2.91 g, 30.29 mmol) was added to a mixture of 2,3-dichloropyrazine (4.75 g, 31.9 mmol) and 2-phenoxyethanol (4.18 g, 30.3 mmol) in dioxane (25 mL). The reaction mixture was stirred at ambient temperature for 1.5 h and then filtered. The filtrate was concentrated under reduced pressure and the crystalline residue obtained was dried in a vacuum oven affording 4.4 g (62%) of the title compound as white waxy crystals: mp 55-54 °C. Anal. (C₁₂H₁₁ClN₂O₂) C, H, N.

### Step 2: 2-(2-Phenoxy)ethyl 3-(1-piperazinyl)-2-pyrazinyl ether, Maleate.

A mixture of the product from Step 1 (1.705 g, 6.80 mmol), piperazine (1.75 g, 20.3 mmol), and K₂CO₃ (1.16 g, 8.39 mmol) in acetonitrile (10 mL) was stirred at 50 °C for 1.5 h and for a further 3 h at 80 °C. The reaction mixture was diluted with CH₂Cl₂, filtered, and concentrated. Purification of the semi-solid residue by chromatography on silica gel using CHCl₃/MeOH (9:1) as eluent gave a beige oil. This material was redissolved in ether/CHCl₃ (9: 1), dried over K₂CO₃, and filtered through a short (3 cm) plug of alumina. The filtrate was concentrated *in vacuo* to afford 1.33 g (65%) of the title compound as the free base. The free base was converted to the maleate and recrystallized from MeOH/ether: mp 155-157 °C; HRMS *m*/*z* calcd for C₁₆H₂₀N₄O₂ (M)⁺ 300.1586, found 300.1573. Anal. (C₁₆H₂₀N₄O₂ · C₄H₄O₄) C, H, N.

### EXAMPLE 2

### 2-(2-Furylmethoxy)-3-(1-piperazinyl)pyrazine, Maleate.

### Step 1: 2-Chloro-3-(2-furylmethoxy)pyrazine.

The title compound was prepared according to the procedure described in Example 1, Step 1, starting from 2-furanmethanol (4.18 g, 42.7 mmol), 2,3-dichloropyrazine (2.05 g, 13.8 mmol) and KO-*t*-BuO (1.82 g, 16.2 mmol). The product, which was obtained as a yellowish oil in 68% yield, was used directly in the next step.

### Step 2: 2-(2-Furylmethoxy)-3-(1-piperazinyl)pyrazine, Maleate.

The title compound was prepared according to the procedure described in Example 1, Step 2, starting from 2-chloro-3-(2-furylmethoxy)pyrazine (1.47 g, 7.0 mmol): yield 0.62 g (34%) as the free base. A portion of the free base was converted to the maleate salt: mp 118-120 °C; HRMS *m*/*z* calcd for C₁₃H₁₆N₄O₂ (M)⁺ 260.1273, found 260.1270. Anal. (C₁₃H₁₆N₄O₂ · C₄H₄O₄) C, H, N.

### EXAMPLE 3

### 2-(2-Phenoxyethoxy)-3-(1-piperazinyl)quinoxaline, Hydrochloride.

### Step 1: 2-Chloro-3-(2-phenoxyethoxy)quinoxaline.

The title compound was prepared according to the procedure described in Example 1, Step 1, starting from 2-phenoxyethanol (3.7 g, 26.8 mmol), 2,3-dichloroquinoxaline (1.33 g, 6.7 mmol) and KO-*t*-Bu (0.75 g, 6.7 mmol): yield 0.74 g (37%); mp 99.5-101.5 °C; HRMS *m*/*z* calcd for C₁₆H₁₃ClN₂O₂ (M)⁺ 300.0666, found 300.0672. Anal. (C₁₆H₁₃ClN₂O₂) C, H, N.

### Step 2: 2-(2-Phenoxyethoxy)-3-(1-piperazinyl)quinoxaline, Hydrochloride.

The title compound was prepared according to the procedure described in Example 1, Step 2, starting from 2-chloro-3-(2-phenoxyethoxy)quinoxaline (0.65 g, 2.15 mmol): yield 0.44 g (58%) as the free base. A portion of the free base was converted to its hydrochloride salt: mp 123-126 °C; HRMS *m*/*z* calcd for C₂₀H₂₂N₄O₂ (M)⁺ 350.1743, found 350.1748. Anal. (C₂₀H₂₂N₄O₂ · 1.75 HCl · 0.5 H₂O) C, H, N.

### EXAMPLE 4

### 2-[2-(2-Naphthyloxy)ethoxy]-3-(1-piperazinyl)pyrazine, Trifluoroacetate.

### Step 1: 2-Chloro-3-12-(2-naphthyloxy)ethoxylpyrazine.

Sodium hydride (55% dispersion in mineral oil; 44 mg, 1.0 mmol) was added to a mixture of 2-(2-naphthoxy)ethanol (188 mg, 1.00 mmol) and 2,3-dichloropyrazine (149 mg, 1.00 mmol) in dioxane (0.5 mL), and the reaction mixture was stirred at 40 °C for 15 h. The crude material was used directly in Step 2.

### Step 2: 2-[2-(2-Naphthyloxy)ethoxy]-3-(1-piperazinyl)pyrazine, Trifluoroacetate.

A solution of piperazine (430 mg, 5.00 mmol) in a mixture of dioxane (0.5 mL) and DMF (1 mL) was added to the crude material from Step 1. The reaction mixture was stirred at 60 °C for 15 h, then diluted with EtOAc, washed with water, dried (MgSO₄), and concentrated under reduced pressure. The residue was purified by chromatography on silica gel using EtOAc/MeOH/HOAc/H₂O (20:3:3:2) as eluent. The product-containing fractions were combined and concentrated. The residue was further purified by C-18 HPLC using CH₃CN/H₂O/TFA (gradient: CH₃CN 0% to 100%, TFA 0.1%) to give 34.5 mg (10%) of the title product. HRMS *m*/*z* calcd for C₂₀H₂₂N₄O₂ (M)⁺ 350.1743, found 350.1742.

Examples 5-20 were prepared in an analogous manner starting from 2,3-dichloropyrazine (1 mmol) and the appropriate alcohol (1 mmol).

### EXAMPLE 5

### 2-(4-Bromophenoxy)ethyl 3-(1-piperazinyl)-2-pyrazinyl ether, Trifluoroacetate.

The title compound was prepared starting from 2-(4-bromophenoxy)ethanol to give 47 mg (12%). HRMS *m*/*z* calcd for C₁₆H₁₉BrN₄O₂ (M)⁺ 378.0691, found 378.0698.

### EXAMPLE 6

### 2-(2-Chlorophenoxy)ethyl 3-(1-piperazinyl)-2-pyrazinyl ether, Trifluoroacetate.

The title compound was prepared starting from 2-(2-chlorophenoxy)ethanol to give 32 mg (10%). HRMS *m*/*z* calcd for C₁₆H₁₉ClN₄O₂ (M)⁺ 334.1197, found 334.1195.

### EXAMPLE 7

### 2-(4-Chlorophenoxy)ethyl 3-(1-piperazinyl)-2-pyrazinyl ether, Trifluoroacetate.

The title compound was prepared starting from 2-(4-chlorophenoxy)ethanol to give 56 mg (17%). HRMS *m*/*z* calcd for C₁₆H₁₉ClN₄O₂ (M)⁺ 334.1197, found 334.1182.

### EXAMPLE 8

### 2-Phenoxypropyl 3-(1-piperazinyl)-2-pyrazinyl ether, Trifluoroacetate.

The title compound was prepared starting from 2-phenoxypropanol to give 24 mg (6%).

HRMS *m*/*z* calcd for C₁₇H₂₂N₄O₂ (M)⁺ 314.1743, found 314.1742.

### EXAMPLE 9

### 2-Phenoxy-1-methylethyl 3-(1-piperazinyl)-2-pyrazinyl ether, Trifluoroacetate.

The title compound was prepared starting from 1-phenoxy-2-propanol to give 29 mg (9%). HRMS *m*/*z* calcd for C₁₇H₂₂N₄O₂ (M)⁺ 314.1743, found 314.1732.

### EXAMPLE 10

### 2-(2-Methoxyphenoxy)-1-methylethyl 3-(1-piperazinyl)-2-pyrazinyl ether, Trifluoroacetate.

The title compound was prepared starting from 1-(2-methoxyphenoxy)-2-propanol to give 25 mg (5%). HRMS *m*/*z* calcd for C₁₈H₂₄N₄O₃ (M)⁺ 344.1848, found 344.1852.

### EXAMPLE 11

### 2-(3-Methoxyphenoxy)-1-methylethyl 3-(1-piperazinyl)-2-pyrazinyl ether, Trifluoroacetate.

The title compound was prepared starting from 1-(3-methoxyphenoxy)-2-propanol to give 38 mg (11%). HRMS *m*/*z* calcd for C₁₈H₂₄N₄O₃ (M)⁺ 344.1848, found 344.1842.

### EXAMPLE 12

### 2-(2-Methylphenoxy)-1-methylethyl 3-(1-piperazinyl)-2-pyrazinyl ether, Trifluoroacetate.

The title compound was prepared starting from 1-(2-methylphenoxy)-2-propanol to give 60 mg (18%). HRMS *m*/*z* calcd for C₁₈H₂₄N₄O₂ (M)⁺ 328.1899, found 328.1898.

### EXAMPLE 13

### 2-(4-Methylphenoxy)-1-methylethyl 3-(1-piperazinyl)-2-pyrazinyl ether, Trifluoroacetate.

The title compound was prepared starting from 1-(4-methylphenoxy)-2-propanol to give 12 mg (3%). HRMS *m*/*z* calcd for C₁₈H₂₄N₄O₂ (M)⁺ 328.1899, found 328.1896.

### EXAMPLE 14

### 2-(Phenylthio)ethyl 3-(1-piperazinyl)-2-pyrazinyl ether, Trifluoroacetate.

The title compound was prepared starting from 2-(phenylthio)ethanol to give 30 mg (10%). HRMS *m*/*z* calcd for C₁₆H₂₀N₄O_{S} (M)⁺ 316.1358, found 316.1359.

### EXAMPLE 15

### 2-(Anilino)ethyl 3-(1-piperazinyl)-2-pyraziny ether, Trifluoroacetate.

The title compound was prepared starting from 2-anilinoethanol to give 48 mg (16%). HRMS *m*/*z* calcd for C₁₆H₂₁N₅O (M)⁺ 299.1746, found 299.1754.

### EXAMPLE 16

### 2-(N-Ethylanilino)ethyl 3-(1-piperazinyl)-2-pyrazinyl ether, Trifluoroacetate.

The title compound was prepared starting from 2-(*N*-ethylanilino)ethanol to give 7 mg (2%). HRMS *m*/*z* calcd for C₁₈H₂₅N₅O (M)⁺ 327.2059, found 327.2057.

### EXAMPLE 17

### 2,3-Dihydro-1,4-benzodioxin-2-ylmethyl 3-(1-piperazinyl)-2-pyrazinyl ether, Trifluoroacetate.

The title compound was prepared starting from 2-hydroxymethyl-1,4-benzodioxane to give 22 mg (5%). HRMS *m*/*z* calcd for C₁₇H₂₀N₄O₃ (M)⁺ 328.1535, found 328.1519.

### EXAMPLE 18

### 3-Phenylpropyl 3-(1-piperazinyl)-2-pyrazinyl ether, Trifluoroacetate.

The title compound was prepared starting from 3-phenyl-1-propanol to give 9 mg (2%). HRMS *m*/*z* calcd for C₁₇H₂₂N₄O (M)⁺ 298.1794, found 298.1795.

### EXAMPLE 19

### 4-Phenylbutyl 3-(1-piperazinyl)-2-pyrazinyl ether, Trifluoroacetate.

The title compound was prepared starting from 4-phenyl-1-butanol to give 10 mg (2%). HRMS *m*/*z* calcd for C₁₈H₂₄N₄O (M)⁺ 312.1950, found 312.1963.

### EXAMPLE 20

### 2-(Benzyloxy)ethyl 3-(1-piperazinyl)-2-pyrazinyl ether, Trifluoroacetate.

The title compound was prepared starting from 2-benzyloxyethanol to give 31 mg (7%). HRMS *m*/*z* calcd for C₁₇H₂₂N₄O₂ (M)⁺ 314.1743, found 314.1739.

### EXAMPLE 21

### 4-(2-{[3-(1-Piperazinyl)-2-pyrazinyl]oxy}ethoxy)-1,3-benzoxazol-2-amine, Fumarate.

### Step 1: 4-(2-Hydroxyethoxy)-2-amino-1, 3-benzoxazol.

The title compound was prepared according to the procedure of Example 91, Step 1, starting from 4-hydroxy-2-amino-1,3-benzoxazol* (0,97 g, 6.5 mmol) and ethylene carbonate (0.63 g, 7.1 mmol). Solid; yield 46%; mp 124-126 °C. Anal. (C₉H₁₀N₂O₃) C, H, N.
*Previously described in *J. Chem. Soc.* **1960**, 2369-2370.

### Step 2: 4-{2-[(3-Chloro-2-pyrazinyl)oxy]ethoxy}-1,3-benzoxazol-2-amine.

The title compound was prepared according to the procedure of Example 4, Step 1, except that 4 equiv of both NaH and 2,3-dichloropyrazine were used, starting from the product of Step 1. The reaction temperature was 90 °C. The crude product was used directly in the next step.

### Step 3: 4-(2-{[3-(1-Piperazinyl)-2-pyrazinyl]oxy}ethoxy)-1,3-benzoxazol-2-amine, Fumarate.

The title compound was prepared according to the procedure of Example 1, Step 2, starting from the product of Step 2 above. Yield of free base 24%. The free base was converted to the fumarate salt: mp 138-140 °C. Anal. (C₁₇H₂₀N₆O₃ · C₄H₄O₄ · 0.25H₂O) C, H, N.

### EXAMPLE 22

### 2-(Phenoxy)ethyl 3-(3-amino-1-pyrrolidinyl)-2-pyrazinyl ether.

The title compound was prepared according to the procedure described in Example 4, Step 2, starting from 2-chloro-3-(2-phenoxyethoxy)pyrazine (150 mg, 0.60 mmol; from Example 1, Step 1) and 3-aminopyrrolidine (270 mg, 3.13 mmol) with the exception that a final extraction step between EtOAc and 5% aqueous NaOH was carried out. This gave 121 mg (67%) of the title product. Anal. (C₁₆H₂₀N₄O₂) C, H; N: calcd, 18.65; found, 18.0.

### EXAMPLE 23

### 2-(2-Chlorophenoxy)ethyl 3-(3-amino-1-pyrrolidinyl)-2-pyrazinyl ether.

The title compound was prepared according to the procedure described in Example 4, Step 2, starting from 2-chloro-3-[2-(2-chlorophenoxy)ethoxy]pyrazine* (150 mg, 0.53 mmol) and 3-aminopyrrolidine (252 mg, 2.92 mmol) with the exception that a final extraction step between EtOAc and 5% aqueous NaOH was carried out. This gave 100 mg (56%) of the title product. Anal. (C₁₆H₁₉ClN₄O₂) C, H; N: calcd, 16.73; found, 16.0.
*Prepared according to the procedure described in Example 4, Step 1.

### EXAMPLE 24

### 2-(4-Chlorophenoxy)ethyl 3-(3-amino-1-pyrrolidinyl)-2-pyrazinyl ether.

The title compound was prepared according to the procedure described in Example 4, Step 2, starting from 2-chloro-3-[2-(4-chlorophenoxy)ethoxy]pyrazine* (150 mg, 0.53 mmol) and 3-aminopyrrolidine (247 mg, 2.87 mmol) with the exception that a final extraction step between EtOAc and 5% aqueous NaOH was carried out. This gave 123 mg (69%) of the title product. Anal. (C₁₆H₁₉ClN₄O₂) H, N; C: calcd, 57.40; found, 56.9.

### EXAMPLE 25

### 2-(Phenoxy)ethyl 3-(1,4-diazepan-1-yl)-2-pyrazinyl ether.

The title compound was prepared according to the procedure described in Example 4, Step 2, starting from 2-chloro-3-(2-phenoxyethoxy)pyrazine (150 mg, 0.60 mmol; from Example 1, Step 1) and homopiperazine (250 mg, 2.5 mmol) with the exception that a final extraction step between EtOAc and 5% aqueous NaOH was carried out. This gave 141 mg (75%) of the title product. Anal. (C₁₇H₂₂N₄O₂ · 0.35EtOAc) C, H, N.

### EXAMPLE 26

### 2,3-Dihydro-1,4-benzodioxin-2-ylmethyl 3-(1,4-diazepan-1-yl)-2-pyrazinyl ether.

The title compound was prepared according to the procedure described in Example 4, Step 2, starting from 2-chloro-3-(2,3-dihydro-1,4-benzodioxin-2-ylmethoxy)pyrazine* (150 mg, 0.54 mmol) and homopiperazine (266 mg, 2.66 mmol) with the exception that a final extraction step between EtOAc and 5% aqueous NaOH was carried out. This gave 105 mg (57%) of the title product. Anal. (C₁₈H₂₂N₄O₃) H, N; C: calcd, 63.14; found, 62.70.
*Prepared according to the procedure described in Example 4, Step 1.

### EXAMPLE 27

### 2-(4-Chlorophenoxy)ethyl 3-(1,4-diazepan-1-yl)-2-pyrazinyl ether.

The title compound was prepared according to the procedure described in Example 4, Step 2, starting from 2-chloro-3-[2-(4-chlorophenoxy)ethoxy]pyrazine* (150 mg, 0.53 mmol) and homopiperazine (287 mg, 2.87 mmol) with the exception that a final extraction step between EtOAc and 5% aqueous NaOH was carried out. This gave 128 mg (69%) of the title product. HRMS *m*/*z* calcd for C₁₇H₂₁ClN₄O₂ (M)⁺ 348.1353, found 348.1353. Anal. (C₁₇H₂₁ClN₄O₂) C, H, N.

### EXAMPLE 28

### 2-(Phenoxy)ethyl 3-(3-methyl-1-piperazinyl)-2-pyrazinyl ether.

The title compound was prepared according to the procedure described in Example 4, Step 2, starting from 2-chloro-3-(2-phenoxyethoxy)pyrazine (150 mg, 0.54 mmol; from Example 1, Step 1) and 2-methylpiperazine (250 mg, 2.5 mmol) with the exception that a final extraction step between EtOAc and 5% aqueous NaOH was carried out. This gave 138 mg (73%) of the title product. Anal. (C₁₇H₂₂N₄O₂) C, H, N.

### EXAMPLE 29

### 2-(4-Chlorophenoxy)ethyl 3-(3-methyl-1-piperazinyl)-2-pyrazinyl ether.

The title compound was prepared according to the procedure described in Example 4, Step 2, starting from 2-chloro-3-[2-(4-chlorophenoxy)ethoxy]pyrazine* (150 mg, 0.53 mmol) and 2-methylpiperazine (256 mg, 2.56 mmol) with the exception that a final extraction step between EtOAc and 5% aqueous NaOH was carried out. This gave 143 mg (77%) of the title product. HRMS *m*/*z* calcd for C₁₇H₂₁ClN₄O₂ (M)⁺ 348.1353, found 348.1370. Anal. (C₁₇H₂₁ClN₄O₂) C, H, N.
*Prepared according to the procedure described in Example 4, Step 1.

### EXAMPLE 30

### 2-(2-Methoxyphenoxy)-1-methylethyl 3-(3-methyl-1-piperazinyl)-2-pyrazinyl ether.

The title compound was prepared according to the procedure described in Example 4, Step 2, starting from 2-chloro-3-[2-(2-methoxyphenoxy)-1-methylethoxy]pyrazine* (150 mg, 0.51 mmol) and 2-methylpiperazine (260 mg, 2.6 mmol) with the exception that a final extraction step between EtOAc and 5% aqueous NaOH was carried out. This gave 118 mg (65%) of the title product. HRMS *m*/*z* calcd for C₁₉H₂₆N₄O₃ (M)⁺ 358.2005, found 358.2018. Anal. (C₁₉H₂₆N₄O₃) C, H, N.

### EXAMPLE 31

### 2,3-Dihydro-1,4-benzodioxin-2-ylmethyl 3-(3-methyl-1-piperazinyl)-2-pyrazinyl ether.

The title compound was prepared according to the procedure described in Example 4, Step 2, starting from 2-chloro-3-(2,3-dihydro-1,4-benzodioxin-2-ylmethoxy)pyrazine* (150 mg, 0.54 mmol) and 2-methylpiperazine (293 mg, 2.92 mmol) with the exception that a final extraction step between EtOAc and 5% aqueous NaOH was carried out. This gave 118 mg (64%) of the title product. HRMS *m*/*z* calcd for C₁₈H₂₂N₄O₃ (M)⁺ 342.1692, found 342.1678. Anal. (C₁₈H₂₂N₄O₃) H, N; C: calcd, 63.14; found, 62.30.

### EXAMPLE 32

### 2-(Phenoxy)ethyl 3-(4-ethyl-1-piperazinyl)-2-pyrazinyl ether.

The title compound was prepared according to the procedure described in Example 4, Step 2, starting from 2-chloro-3-(2-phenoxyethoxy)pyrazine (150 mg, 0.60 mmol; from Example 1, Step 1) and *N*-ethylpiperazine (250 mg, 2.19 mmol) with the exception that a final extraction step between EtOAc and 5% aqueous NaOH was carried out. This gave 127 mg (64%) of the title product. Anal. (C₁₈H₂₄N₄O₂) C, H, N.

### EXAMPLE 33

### 2-(2-Chlorophenoxy)ethyl 3-(4-ethyl-1-piperazinyl)-2-pyrazinyl ether.

The title compound was prepared in an analogous manner to Example 4, Step 2, starting from 2-chloro-3-[2-(2-chlorophenoxy)ethoxy]pyrazine* (150 mg, 0.53 mmol) and *N*-ethylpiperazine (221 mg, 1.93 mmol) with the exception that a final extraction step between EtOAc and 5% aqueous NaOH was carried out. This gave 100 mg (52%) of the title product. Anal. (C₁₈H₂₃ClN₄O₂) C, H, N.
*Prepared according to the procedure described in Example 4, Step 1.

### EXAMPLE 34

### 2-(4-Chlorophenoxy)ethyl 3-(4-ethyl-1-piperazinyl)-2-pyrazinyl ether.

The title compound was prepared according to the procedure described in Example 4, Step 2, starting from 2-chloro-3-[2-(4-chlorophenoxy)ethoxy]pyrazine* (150 mg, 0.53 mmol) and *N*-ethylpiperazine (221 mg, 1.93 mmol) with the exception that a final extraction step between EtOAc and 5% aqueous NaOH was carried out. This gave 138 mg (72%) of the title product. HRMS *m*/*z* calcd for C₁₈H₂₃ClN₄O₂) (M)⁺ 362.1510, found 362.1526. Anal. (C₁₈H₂₃ClN₄O₂) C, H, N.
*Prepared according to the procedure described in Example 4, Step 1.

### EXAMPLE 35

### 2,3-Dihydro-1,4-benzodioxin-2-ylmethyl 3-(4-ethyl-1-piperazinyl)-2-pyrazinyl ether.

The title compound was prepared according to the procedure described in Example 4, Step 2, starting from 2-chloro-3-(2,3-dihydro-1,4-benzodioxin-2-ylmethoxy)pyrazine* (150 mg, 0.54 mmol) and *N*-ethylpiperazine (219 mg, 1.92 mmol) with the exception that a final extraction step between EtOAc and 5% aqueous NaOH was carried out. This gave 128 mg (66%) of the title product. HRMS *m*/*z* calcd for C₁₉H₂₄N₄O₃ (M)⁺ 356.1848, found 356.1848. Anal. (C₁₉H₂₄N₄O₃) C, H, N.

### EXAMPLE 36

### 2-(2-Methoxyphenoxy)-1-methylethyl 3-(4-ethyl-1-piperazinyl)-2-pyrazinyl ether.

The title compound was prepared according to the procedure described in Example 4, Step 2, starting from 2-chloro-3-[2-(2-methoxyphenoxy)-1-methylethoxy]pyrazine* (150 mg, 0.51 mmol) and *N*-ethylpiperazine (222 mg, 1.94 mmol) with the exception that a final extraction step between EtOAc and 5% aqueous NaOH was carried out. This gave 127 mg (67%) of the title product. HRMS *m*/*z* calcd for C₂₀H₂₈N₄O₃ (M)⁺ 372.2161, found 372.2174. Anal. (C₂₀H₂₈N₄O₃) C, H, N.

### EXAMPLE 37

### 2-(3-Bromophenoxy)ethyl 3-(1-piperazinyl)-2-pyrazinyl ether.

### Step 1: 2-(3-Bromophenoxy)ethyl 3-chloro-2-pyrazinyl ether.

A mixture of 3-bromophenol (1.73 g, 10.0 mmol), ethylene oxide (0.44 g, 10 mmol), Et₃N (3 drops) and dioxane (4 mL) was heated at 100 °C in a sealed tube for 2 d. The solution was cooled in an ice-bath and KO-*t*-Bu (1.07 g, 9.50 mmol) followed by 2,3-dichloropyrazine (1.34 g, 9 mmol) were added along with dioxane (1 mL). The mixture was stirred at room temperature for 90 min, diluted with CH₂Cl₂ and filtered. The filtrate was concentrated and the residue was purified by chromatography on silica gel (gradient: isohexane to 25% EtOAc/isohexane) to give 2.21 g (75%) of the title product as a white solid: mp 57-58 °C. Anal. (C₁₂H₁₀BrClN₂O₂) C, H, N.

### Step 2: 2-(3-Bromophenoxy)ethyl 3-(1-piperazinyl)-2-pyrazinyl ether.

Piperazine (1.29 g, 15.0 mmol) and K₂CO₃ (0.69 g, 5.0 mmol) were added to a solution of 2-(3-bromophenoxy)ethyl 3-chloro-2-pyrazinyl ether (1.65 g, 5.00 mmol) in acetonitrile (25 mL) at room temperature. The mixture was heated at reflux for 21 h, allowed to cool, concentrated and the residue was partitioned between water and EtOAc. The layers were separated and the organic phase was washed with water and brine, and dried over MgSO₄. Purification by chromatography on silica gel (gradient: PhMe to PhMe/MeOH/Et₃N, 8:1:1) gave 1.61 g (85%) of the title compound as a pale yellow oil that slowly solidified on standing: mp 62-63 °C. Anal. (C₁₆H₁₉BrN₄O₂) C, H, N.

### EXAMPLE 38

### 2-[2-(2-Chlorophenoxy)ethoxy]-3-(1-piperazinyl)quinoxaline.

### Step 1: 2-Chloro-3-[2-(2-chlorophenoxy)ethoxy]quinoxaline.

2-(2-Chlorophenoxy)ethanol (0.229 g, 1.33 mmol) was treated with NaH (55% dispersion in mineral oil; 0.058 g, 1.33 mmol) in dioxane (2 mL). After stirring at room temperature for 3 h, the mixture was added dropwise to a slurry of 2.3-dichloroquinoxaline (0.320 g, 1.60 mmol) in dioxane (1 mL). The resulting mixture was stirred at room temperature for 15 h to produce the intermediate 2-chloro-3-[2-(2-chlorophenoxy)ethoxy]quinoxaline which was used directly in the next step.

### Step 2: 2-[2-(2-Chlorophenoxy)ethoxy]-3-(1-piperazinyl)quinoxaline.

Piperazine (0.580 g, 6.65 mmol) was added to the crude material from Step I and the resulting mixture was stirred at room temperature for 5 h. The solvent was evaporated, and the crude mixture was dissolved in DMSO and precipitated with water. The precipitate was purified by chromatography on silica gel using
EtOAc/HOAc/MeOH/H₂O (40:3:3:2) as eluent to afford 0.100 g (22%) of the title compound as a yellow oil. HRMS *m*/*z* calcd for C₂₀H₂₁ClN₄O₂ (M)⁺ 384.1353, found 384.1334.

Examples 39-45 were prepared according to the procedure of Example 38, starting from 2,3-dichloroquinoxaline and the appropriate alcohol.

### EXAMPLE 39

### 2-[2-(4-Chlorophenoxy)ethoxy]-3-(1-piperazinyl)quinoxaline.

The title compound was prepared starting from 2-(4-chlorophenoxy)ethanol (0.229 g, 1.33 mmol) to give 0.250 g (56%) of a yellow solid: mp 108-112 °C; HRMS *m*/*z* calcd for C₂₀H₂₁ClN₄O₂ (M)⁺ 384.1353, found 384.1347.

### EXAMPLE 40

### 2-[2-(Phenylthio)ethoxy]-3-(1-piperazinyl)quinoxaline.

The title compound was prepared starting from 2-(phenylthio)ethanol (0.205 g, 1.33 mmol) to give 0.045 g (9%) of a yellow oil. HRMS *m*/*z* calcd for C₂₀H₂₂N₄OS (M)⁺ 366.1514, found 366.1509.

### EXAMPLE 41

### 2-(1-Methyl-2-phenoxyethoxy)-3-(1-piperazinyl)quinoxaline.

The title compound was prepared starting from 1-methyl-2-phenoxyethanol (0.202 g, 1.33 mmol) to give 0.084 g (17%) of a yellow oil. HRMS *m*/*z* calcd for C₂₁H₂₄N₄O₂ (M)⁺ 364.1899, found 364.1908.

### EXAMPLE 42

### 2-[1-Methyl-2-(4-methylphenoxy)ethoxy]-3-(1-piperazinyl)quinoxaline.

The title compound was prepared starting from 1-methyl-2-(4-methylphenoxy)ethanol (0.221 g, 1.33 mmol) to give 0.047 g (9%) of a yellow oil. Anal. (C₂₂H₂₆N₄O₂) C, H, N.

### EXAMPLE 43

### 2-[2-(2-Methoxyphenoxy)-1-methylethoxy]-3-(1-piperazinyl)quinoxaline.

The title compound was prepared starting from 1-methyl-2-(2-methoxyphenoxy)ethanol (0.242 g, 1.33 mmol) to give 0.072 g (14%) of a yellow oil. Anal. (C₂₂H₂₆N₄O₃) C, H, N: calcd, 14.20; found, 13.70.

### EXAMPLE 44

### 2-(2,3-Dihydro-1,4-benzodioxin-2-ylmethoxy)-3-(1-piperazinyl)quinoxaline.

The title compound was prepared starting from 2,3-dihydro-1,4-benzodioxin-2-ylmethanol (0.221 g, 1.33 mmol) to give 0.18 g (35%) of a yellow oil. Anal. (C₂₁H₂₂N₄O) C, H, N.

### EXAMPLE 45

### 2-[2-(2-Naphthyloxy)ethoxy]-3-(1-piperazinyl)quinoxaline.

The title compound was prepared starting from 2-(2-naphthyloxy)ethanol (0.250 g, 1.33 mmol) to give 0.22 g (41 %) of a yellow solid. HRMS *m*/*z* calcd for C₂₄H₂₄N₄O₂ (M)⁺ 400.1899, found 400.1902.

### EXAMPLE 46

### 2-(2-Phenoxyethylamino)-3-(1-piperazinyl)pyrazine.

### Step 1: 2-Chloro-3-(2-phenoxyethylamino)pyrazine.

A mixture of 2-phenoxyethylamine (2.65 g, 19.3 mmol), 2,3-dichloropyrazine (2.88 g, 19.3 mmol) and K₂CO₃ (2.67 g, 19.3 mmol) in acetonitrile (8 mL) was stirred in a sealed tube for 12 h at room temperature, and for a further 9.5 h at 80 °C. The reaction mixture was diluted with ether, filtered, and concentrated. The residue was purified by chromatography on silica gel using n-hexane/EtOAc (7:3) as eluent to give 2.36 g (49%) of the title compound as a yellowish oil that solidified on standing: mp 51-53 °C; HRMS *m*/*z* calcd for C₁₂H₁₂ClN₃O (M)⁺ 249.0669, found 249.0659. Anal. (C₁₂H₁₂ClN₃O) C, H, N.

### Step 2: 2-(2-Phenoxyethylamino)-3-(1-piperazinyl)pyrazine.

A mixture of the product from Step 1 above (1.59 g, 6.37 mmol), piperazine (1.56 g, 18.2 mmol), and K₂CO₃ (0.88 g, 6.4 mmol) in acetonitrile (10 mL) was stirred at 140 °C for 12 h in a sealed tube. After cooling, the reaction mixture was diluted with CH₂Cl₂, filtered, and concentrated. The semisolid residue was purified by chromatography on silica gel using CHCl₃/MeOH (9:1) as eluent to give a beige oil. This oil was redissolved in CHCl₃, and filtered through a short (3 cm) plug of alumina covered with K₂CO₃. The pad was washed with several portions of CHCl₃ and the resulting filtrate was concentrated to afford 1.48 g (78%) of the title product as a pale yellow oil. HRMS *m*/*z* calcd for C₁₆H₂₁N₅O (M)⁺ 299.1746, found 299.1753. Anal. (C₁₆H₂₁N₅O · 1/3H₂O) C, H, N.

### EXAMPLE 47

### N-Methyl-N-(2-phenoxyethyl)-3-(1-piperazinyl)-2-pyrazinamine, Fumarate.

### Step 1: 3-Chloro-N-methyl-N-(2-phenoxyethyl)-2-pyrazinamine.

A mixture of 2,3-dichloropyrazine (6.64 g, 44.6mmol) and N-methyl-N-(2-phenoxyethyl)amine* (4.5 g, 29.8 mmol) in DMF (5 mL) was placed in a sealed Pyrex tube and heated in a microwave oven (Labwell MW10) for 1 x 2 min followed by 2 x 1 min at 75W. The mixture was concentrated and the resulting oil was purified by column chromatography on silica using hexane/EtOAc (95:5 followed by 93:7) to give 5.56 g (71%) of the title product as a colorless oil. HRMS *m*/*z* calcd for C₁₃H₁₄ClN₃O (M)⁺ 263.0825, found 263.0824.
*Previously described in *J*. *Chem. Soc.* **1963,** 1385-1400.

### Step 2: N-Methyl-N-(2-phenoxyethyl)-3-(1-piperazinyl)-2-pyrazinamine, Fumarate.

A mixture of the product obtained in Step 1 (5.0 g, 19 mmol) and piperazine (5.0 g, 59.4 mmol) in DMF (10 mL) was placed in a sealed Pyrex tube and heated in a microwave oven (Labwell MW10) for 2.5 min at 75W. The mixture was concentrated and the remaining yellow oil was partitioned between water and CHCl₃. The water phase was extracted with CHCl₃ (x 2) and the combined organic phases were extracted with 0.5 M aqueous HCl (x 2). The combined aqueous phases were made alkaline (11M NaOH) and extracted with CHCl₃ (x 3). The combined organic phases were washed with water and brine, dried (MgSO₄), and concentrated to give the free base of the title product as a light yellow oil. The free base was converted to its fumarate salt. Yield 1.38 g (17%) of off-white crystals from methanol; mp 154°C. Anal. (C₁₇H₂₃N₅O · C₄H₄O₄) H, N; C: calcd, 58.7; found, 58.05.

### EXAMPLE 48

### 2-(Phenoxy)ethyl 3-(3-pyrrolidinyloxy)-2-pyrazinyl ether.

2-Chloro-3-(2-phenoxyethoxy)pyrazine (150 mg, 0.60 mmol; from Example 1, Step 1) was added to a stirred mixture of 3-pyrrolidinol (260 mg, 2.99 mmol) and NaH (55% dispersion in mineral oil; 110 mg, 2.50 mmol) in dioxane (2 mL), and the reaction was stirred at room temperature for 2 h. EtOAc was added and the mixture was washed with water, dried (MgSO₄), and concentrated. The residue was purified by chromatography on silica gel using EtOAc/MeOH/HOAc/H₂O (50:40:9:1) as eluent to give 38 mg (22%) of the title compound. Anal. (C₁₆H₁₉N₃O₃) H; C: calcd, 63.77; found, 62.20; N: calcd, 13.94; found, 13.0.

### EXAMPLE 49

### 2-(Phenoxy)ethyl 3-[(3R)-pyrrolidinyloxy]-2-pyrazinyl ether.

### Step 1: 2-(Phenoxy)ethyl 3-[N-tert-butoxycarbonyl (3R)-pyrrolidinyloxy]-2-pyrazinyl ether.

2-Chloro-3-(2-phenoxyethoxy)pyrazine (250 mg, 1.00 mmol; from Example 1, Step 1) was added to a mixture of *N*-Boc-(*R*)-3-pyrrolidinol (225 mg, 1.20 mmol) and KO-*t*-Bu (140 mg, 1.25 mmol) in toluene (5 mL), and the reaction was stirred at 95 °C for 30 min. The reaction mixture was diluted with toluene, washed with water, dried (MgSO₄), and concentrated under reduced pressure. The residue was purified by chromatography on silica gel using toluene/EtOAc (9:1) as eluent to give 261 mg (65%) of the title product which was used in the next step without further characterization.

### Step 2: 2-(Phenoxy)ethyl 3-[(3R)-pyrrolidinyloxy]-2-pyrazinyl ether.

The product from Step 1 above was treated with CH₂Cl₂/trifluoroacetic acid/H₂O (50:45:5; 4 mL) for 30 min. The mixture was concentrated and the residue was partitioned between 5% aqueous NaOH and CH₂Cl₂. After separation, the organic layer was dried (MgSO₄) and concentrated. The residue was purified by chromatography on silica gel using CH₂Cl₂/MeOH (8:2) as eluent to give 44 mg (15%) of the title product: [α]_{D} = - 4.6° (c 0.017, MeOH); HRMS *m*/*z* calcd for C₁₆H₁₉N₃O₃ (M)⁺ 301.1426, found 301.1427.

### EXAMPLE 50

### 2-(Phenoxy)etbyl 3-(4-piperidinyloxy)-2-pyrazinyl ether.

The title compound was prepared according to the procedure described in Example 49 starting from 2-chloro-3-(2-phenoxyethoxy)pyrazine (250 mg, 1.00 mmol; from Example 1, Step 1) and *N*-Boc-4-hydroxypiperidine (234 mg, 1.16 mmol) to give 112 mg (35%) of the title product. HRMS *m*/*z* calcd for C₁₇H₂₃N₃O₃ (M)⁺ 315.1583, found 315.1570.

### EXAMPLE 51

### 2-(Phenoxy)ethyl 3-[(2S)-pyrrolidinylmethoxy]-2-pyrazinyl ether.

The title compound was prepared according to the procedure described in Example 49, starting from 2-chloro-3-(2-phenoxyethoxy)pyrazine (250 mg, 1.00 mmol; from Example 1, Step 1) and *N*-Boc-*L*-prolinol (250 mg, 1.24 mmol). The intermediate product 2-(phenoxy)ethyl 3-[*N-tert*-butoxycarbonyl (2S)-pyrrolidinylrnethoxy]-2-pyrazinyl ether was not characterized. Yield 23 mg (7%); [α]_{D} = + 13.2° (c 0.016, MeOH). HRMS *m*/*z* calcd for C₁₇H₂₁N₃O₃ (M)⁺ 315.1583, found 315.1598.

### EXAMPLE 52

### 2-(2-Methoxypbenoxy)ethyl 3-(1-piperazinyl)-2-pyrazinyl ether.

### Step 1: 2-Cbloro-3-(4-tert-butoxycarbonyl-1-piperazinyl)pyrazine.

A mixture of N-Boc-piperazine (11.47 g, 61.5 mmol), K₂CO₃ (8.5 g, 61 mmol) and 2,3-dichloropyrazine (9.20 g, 61.7 mmol) in acetonitrile (100 mL) was stirred at 100°C for 40 h. The reaction mixture was concentrated, dissolved in toluene, washed with water, dried (MgSO₄), and concentrated. The residue was purified by chromatography on silica gel using toluene/EtOAc (7:3) as eluent to give 18.3 g (100%) of the title product. HRMS *m*/*z* calcd for C₁₃H₁₉ClN₄O₂ (M)⁺ 298.1197, found 298.1206.

### Step 2: 2-[3-(4-tert-Butoxycarbonyl-1-piperazinyl)-2-pyrazinyloxy]ethanol.

KO-*t*-Bu (9.92 g, 103 mmol) was added to a mixture of the product obtained in Step 1 (18.14 g, 60.7 mmol) and ethylene glycol (25 mL, 448 mmol) in pyridine (125 mL) at 85 °C. The reaction mixture was stirred for 15 h and then poured into ice-water and extracted with toluene. The organic phase was dried (MgSO₄) and concentrated. The residue was purified by chromatography on silica gel using toluene/EtOAc (1:1) as eluent to give 16.9 g (85%) of the title product. HRMS *m*/*z* calcd for C₁₅H₂₄N₄O₄ (M)⁺ 324.1798, found 324.1784.

### Step 3: 2-(2-Methoxyphenoxy)ethyl 3-(1-piperazinyl)-2-pyrazinyl ether.

1,1'-Azobis(*N,N*-dimethylformamide) (TMAD; 60 mg, 0.35 mmol) was dissolved in THF (1 mL) and DMF (0.5 mL) and added to a stirred solution of the product from Step 2 (100 mg, 0.31 mmol), 2-methoxyphenol (124 mg, 1.00 mmol) and triphenylphosphine (92 mg, 0.35 mmol) in THF (1 mL). The reaction mixture was stirred for 15 h and then concentrated under reduced pressure. The residue was passed through a bed of silica gel using toluene/EtOAc/MeOH (45:45:10) as eluent, and the pure fractions were combined, concentrated and treated with CH₂Cl₂/TFA/H₂O (50:45:5; 5 mL) for 30 min. The mixture was concentrated and the residue was purified by chromatography on silica gel using EtOAc/HOAc/MeOH/H₂O (20:3:3:2) as eluent. The combined product-containing fractions were concentrated and partitioned between 5% aqueous NaOH and CH₂Cl₂. The organic phase was concentrated, and the residue was purified by chromatography on silica gel using CH₂Cl₂/MeOH (8:2) as eluent to give 11.2 mg (11%) of the title product. HRMS *m*/*z* calcd for C₁₇H₂₂N₄O₃ (M)⁺ 330.1692, found 330.1707.

Examples 53-68 were prepared according to the procedure described in Example 52, Step 3, starting from 2-[3-(4-*tert*-butoxycarbonyl-1-piperazinyl)-2-pyrazinyloxy]ethanol and the requisite phenolic compound (1 mmol unless otherwise noted).

### EXAMPLE 53

### 2-(4-n-Butoxyphenoxy)ethyl 3-(1-piperazinyl)-2-pyrazinyl ether, Trifluoroacetate.

The title compound was prepared starting from *p*-butoxyphenol, but no extraction with NaOH and without the third chromatographic step, to give 92 mg (19%) as the trifluoroacetate salt. HRMS *m*/*z* calcd for C₂₀H₂₈N₄O₃ (M)⁺ 372.2161, found 372.2162.

### EXAMPLE 54

### 2-(2-Isopropoxyphenoxy)ethyl 3-(1-piperazinyl)-2-pyrazinyl ether.

The title compound was prepared starting from 2-isopropoxyphenol to give 216 mg (60%). HRMS *m*/*z* calcd for C₁₉H₂₆N₄O₃ (M)⁺ 358.2005, found 358.2006.

### EXAMPLE 55

### 2-(1-Naphthyloxy)ethyl 3-(1-piperazinyl)-2-pyrazinyl ether.

The title compound was prepared starting from 1-naphthol to give 142 mg (40%). HRMS *m*/*z* calcd for C₂₀H₂₂N₄O₂ (M)⁺ 350.1743, found 350.1758.

### EXAMPLE 56

### 2-(2,5-Difluorophenoxy)ethyl 3-(1-piperazinyl)-2-pyrazinyl ether, Trifluoroacetate.

The title compound was prepared starting from 2,5-difluorophenol, but no extraction with NaOH and without the third chromatographic step, to give 21 mg (6%) as the trifluoroacetate salt. HRMS *m*/*z* calcd for C₁₆H₁₈F₂N₄O₂ (M)⁺ 336.1398. Found 336.1407.

### EXAMPLE 57

### 2-(3,5-Dimethoxyphenoxy)ethyl 3-(1-piperazinyl)-2-pyrazinyl ether.

The title compound was prepared starting from 3,5-dimethoxyphenol to give 52 mg (14%). HRMS *m*/*z* calcd for C₁₈H₂₄N₄O₄ (M)⁺ 360.1798, found 360.1808.

### EXAMPLE 58

### 2-[2-(2-Methoxy-5-nitrophenoxy)ethoxy]-3-(1-piperazinyl)pyrazine, Hydrochloride.

The title compound was prepared starting from 2-methoxy-5-nitrophenol (0.524 g, 3.08 mmol) to give 0.53 g (42 %). Pos-EI-MS shows M⁺ and 10 ions supporting the stated structure. HRMS *m*/*z* calcd for C₁₇H₂₁N₅O₅ (M)⁺ 375.1543, found 375.1561. Anal. (C₁₇H₂)N₅O₅ · 0.5 HCl · 1.3 H₂O) C, H, N.

### EXAMPLE 59

### 2-(2,6-Dimethoxyphenoxy)ethyl 3-(1-piperazinyl)-2-pyrazinyl ether, Trifluoroacetate.

The title compound was prepared starting from 2,6-dimethoxyphenol to give 142 mg (30%). HRMS *m*/*z* calcd for C₁₈H₂₄N₄O₄ (M)⁺ 360.1798, found 360.1797.

### EXAMPLE 60

### 2-(2,3-Dimethoxyphenoxy)ethyl 3-(1-piperazinyl)-2-pyrazinyl ether.

The title compound was prepared starting from 2,3-dimethoxyphenol to give 110 mg (31%). HRMS *m*/*z* calcd for C₁₈H₂₄N₄O₄ (M)⁺ 360.1798, found 360.1800.

### EXAMPLE 61

### 2-(2-Acetylphenoxy)ethyl 3-(1-piperazinyl)-2-pyrazinyl ether, Trifluoroacetate.

The title compound was prepared starting from 2-hydroxyacetophenone (170 mg, 1.25 mmol) and 2-[3-(4-*tert*-butoxycarbonyl-1-piperazinyl)-2-pyrazinyloxy]ethanol (325 mg, 1 mmol; prepared in Example 52, Step 2), but no extraction with NaOH and without the third chromatographic step, to give 170 mg (37%) of the trifluoroacetate. HRMS *m*/*z* calcd for C₁₈H₂₂N₄O₃ (M)⁺ 342.1692, found 342.1704.

### EXAMPLE 62

### 2-(2-Acetyl-5-methoxyphenoxy)ethyl 3-(1-piperazinyl)-2-pyrazinyl ether, Trifluoroacetate.

The title compound was prepared starting from 2-hydroxy-4-methoxy-acetophenone (208 mg, 1.25 mmol) and 2-[3-(4-*tert*-butoxycarbonyl-1-piperazinyl)-2-pyrazinyloxy]ethanol (325 mg, 1 mmol; prepared in Example 52, Step 2), but no extraction with NaOH and without the third chromatographic step, to give 128 mg (26%) of the trifluoroacetate. HRMS *m*/*z* calcd for C₁₉H₂₄N₄O₄ (M)⁺ 372.1798, found 372.1810.

### EXAMPLE 63

### 2-(2-Acetyl-3,5-dimethoxyphenoxy)ethyl 3-(1-piperazinyl)-2-pyrazinyl ether, Trifluoroacetate.

The title compound was prepared starting from 2-hydroxy-4,6-dimethoxyacetophenone (245 mg, 1.25 mmol) and 2-[3-(4-*tert*-butoxycarbonyl-1-piperazinyl)-2-pyrazinyloxy]ethanol (325 mg, 1.00 mmol; prepared in Example 52, Step 2), but no extraction with NaOH and without the third chromatographic step, to give 216 mg (42%) of the trifluoroacetate. HRMS *m*/*z* calcd for C₂₀H₂₆N₄O₅ (M)⁺ 402.1903, found 402.1886.

### EXAMPLE 64

### 2-(5,6,7,8-Tetrahydro-2-naphthyloxy)ethyl 3-(1-piperazinyl)-2-pyrazinyl ether, Trifluoroacetate.

The title compound was prepared starting from 5,6,7,8-tetrahydro-2-naphthol (185 mg, 1.25 mmol) and 2-[3-(4-*tert*-butoxycarbonyl-1-piperazinyl)-2-pyrazinyloxy]ethanol (325 mg, 1.00 mmol; prepared in Example 52, Step 2), but no extraction with NaOH and without the third chromatographic step, to give 155 mg (33%) of the trifluoroacetate. HRMS *m*/*z* calcd for C₂₀H₂₆N₄O₂ (M)⁺ 354.2056, found 354.2068.

### EXAMPLE 65

### 2-(2-Fluoro-6-methoxyphenoxy)ethyl 3-(1-piperazinyl)-2-pyrazinyl ether.

The title compound was prepared starting from 2-fluoro-6-methoxyphenol (178 mg, 1.25 mmol) and 2-[3-(4-*tert*-butoxycarbonyl-1-piperazinyl)-2-pyrazinyloxy]ethanol (1.17 mmol; prepared in Example 52, Step 2) to give 230 mg (66%) of a yellow oil. HRMS *m*/*z* calcd for C₁₇H₂₁FN₄O₃ (M)⁺ 348.1598, found 348.1602.

### EXAMPLE 66

### 2-(2-Methoxy-4-methylphenoxy)ethyl 3-(1-piperazinyl)-2-pyrazinyl ether.

The title compound was prepared starting from 2-methoxy-4-methylphenol (162 mg, 1.25 mmol) and 2-[3-(4-*tert*-butoxycarbonyl-1-piperazinyl)-2-pyrazinyloxy]ethanol (1.00 mmol; prepared in Example 52, Step 2) to give 233 mg (67%) of a yellow solid. HRMS *m*/*z* calcd for C₁₈H₂₄N₄O₃ (M)⁺ 344.1848, found 344.1839.

### EXAMPLE 67

### 7-Isoquinolinyl 2-{[3-(1-piperazinyl)]-2-pyrazinyl}oxy)ethyl ether, Difumarate

The title compound was prepared starting from 7-hydroxyisoquinoline (435 mg, 3.00 mmol) and 2-[3-(4-*tert*-butoxycarbonyl-1-piperazinyl)-2-pyrazinyloxy]ethanol (1.00 mmol; prepared in Example 52, Step 2). The free base of the title compound was converted to its fumarate salt. Yield 45 mg (2%); mp 157 °C (dec). HRMS *m*/*z* calcd for C₁₉H₂₁N₅O₂ (M)⁺ 351.1695, found 351.1695. Anal. (C₁₉H₂₁N₅O₂ · 2.3 C₄H₄O₄).

### EXAMPLE 68

### 2-(2-Chloro-4-methoxyphenoxy)ethyl 3-(1-piperazinyl)-2-pyrazinyl ether.

The title compound was prepared starting from 2-chloro-4-methoxyphenol (103 mg, 0.65 mmol) and 2-[3-(4-*tert*-butoxycarbonyl-1-piperazinyl)-2-pyrazinyloxy]ethanol (0.61 mmol; prepared in Example 52, Step 2) according to the procedure described in Example 52, Step 3, but utilizing diethyl azodicarboxylate (DEAD) instead of TMAD. Yield 50 mg (21%). HRMS *m*/*z* calcd for C₁₇H₂₁ClN₄O₃ (M)⁺ 364.1302, found 364.1307.

### EXAMPLE 69

### 2-{2-[2-Fluoro-5-(trifluoromethyl)phenoxy]ethoxy}-3-(1-piperazinyl)pyrazine.

2-Fluoro-5-(trifluoromethyl)phenol (0.54 g, 3.0 mmol) was added to a stirred solution of DEAD (0.52 g, 3.0 mmol), PPh₃ (0.79 g, 3.0 mmol) and 2-[3-(4-*tert*-butoxycarbonyl-1-piperazinyl)-2-pyrazinyloxy]ethanol (0.49 g, 1.5 mmol; prepared in Example 52, Step 2) in THF (50 mL). The reaction mixture was stirred at room temperature overnight. The solvent was evaporated and the residue was purified by column chromatography on silica using isohexane/EtOAc (80:20) as eluent. The *N*-Boc protected intermediate obtained was treated with CH₂Cl₂/TFA (75:25; 5 mL) for 1 h at room temperature and concentrated. The residue was purified by column chromatography on silica using EtOAc/MeOH/Et₃N (80:15:5) as eluent to give 0.22 g (38%) of the title compound. MS *m*/*z* 387 (M+H)⁺. HRMS *m*/*z* calcd for C₁₇H₁₈F₄N₄O₂ (M)⁺ 386.1366, found 386.1367.

### EXAMPLE 70

### 8-(2-{[3-(1-Piperazinyl)-2-pyrazinyl]oxy}ethoxy)-2H-1,4-benzoxazin-3(4H)-one, Maleate.

### Step 1: 8-Hydroxy-2H-1,4-benzoxazin-3(4H)-one.

The title compound was prepared according to procedure described in *J. Med. Chem.* **1996,** *39*, 3533-3538 by replacing 2-amino-*m*-cresol with 3-amino-pyrocatechol.* The crude product was purified by column chromatography on silica gel using EtOAc/isohexane (1:2 to 2:1) as eluent. Solid; yield 70%. MS *m*/*z* 165 (M)⁺. Anal. (C₈H₇NO₃) C, H, N.
*Previously reported in *Liebigs Ann. Chem.* **1957***, 608,* 128 and *Synth. Commun.* **1997**, *27,* 1661-1668.

### Step 2: 8-(2-{[3-(1-Piperazinyl)-2-pyrazinyl]oxy}ethoxy)-2H-1,4-benzoxazin-3(4H)-one, Maleate.

The title compound was prepared according to the procedure of Example 69 starting from the product of Step 1 above. Yield 42%. MS *m*/*z* 372 (M+H)⁺. Anal. (C₁₈H₂₁N₅O₄ · C₄H₄O₄) C, H, N.

### EXAMPLE 71

### 2-{2-[3-(Methylsulfonyl)phenoxy]ethoxy}-3-(1-piperazinyl)pyrazine.

### Step 1: 3-(Methylsulfonyl)phenol.

The title compound was prepared by a slightly modified literature procedure.* 3-aminophenyl methyl sulfone hydrochloride (1.18 g, 5.70 mmol) was suspended in water/CH₂Cl₂. The mixture was neutralized with 25% aqueous NaOH. The CH₂Cl₂ layer was isolated and evaporated to dryness. Aqueous H₂SO₄ (60%; 10 mL) was added to the residue and the solution was cooled to 0 °C. A solution of NaNO₂ (0.345 g, 5.00 mmol) in water (5 mL) was added dropwise. The solution was stirred at 0 °C for 30 min. and at 90 °C for 30 min. The reaction mixture was allowed to come to room temperature. Extraction with CH₂Cl₂, drying (MgSO₄), and concentration gave 0. 39 g (40%) of the title compound as a solid. MS *m*/*z* 172 (M)⁺. HRMS *m*/*z* calcd for C₇H₈O₃S (M)⁺ 172.0194, found 172.0198.
* *J. Chem. Soc.* **1938**, 899-905.

### Step 2. 2-{2-[3-(Methylsulfonyl)phenoxy]ethoxy}-3-(1-piperazinyl)pyrazine.

The title compound was prepared according the procedure of Example 69 starting from the product of Step 1. Yield 26%. MS *m*/*z* 379 (M+H)⁺. HRMS *m*/*z* calcd for C₁₇H₂₂N₄O₄S (M)⁺ 378.1362, found 378.1343.

### EXAMPLE 72

### 7-(2-{[3-(1-Piperazinyl)-2-pyrazinyl]oxy}ethoxy)-2H-chromen-2-one.

The title compound was prepared according to the procedure of Example 69 starting from 7-hydroxy-2*H*-chromen-2-one. Yield 29%. MS *m*/*z* 369 (M+H)⁺. HRMS *m*/*z* calcd for C₁₉H₂₀N₄O₄ (M)⁺ 368.1485, found 368.1478

### EXAMPLE 73

### 2-(2,3,6-Trifluorophenoxy)ethyl 3-(1-piperazinyl)-2-pyrazinyl ether.

The title compound was prepared according to the procedure of Example 69 starting from 2,3,6-trifluorophenol. Yield 46%. MS *m*/*z* 355 (M+H)⁺. HRMS *m*/*z* calcd for C₁₆H₁₇F₃N₄O₂ (M)⁺ 354.1304, found 354.1288.

### EXAMPLE 74

### 2-(2,4,5-Trifluorophenoxy)ethyl 3-(1-piperazinyl)-2-pyrazinyl ether.

The title compound was prepared according to the procedure of Example 69 starting from 2,4,5-trifluorophenol. Yield 43%. MS *m*/*z* 355 (M+H)⁺. HRMS *m*/*z* calcd for C₁₆H₁₇F₃N₄O₂ (M)⁺ 354.1304, found 354.1304.

### EXAMPLE 75

### 2-(2-Hydroxyphenoxy)ethyl 3-(1-piperazinyl)-2-pyrazinyl ether.

The title compound was prepared according to the procedure of Example 69 starting from catechol. Yield 37%. MS *m*/*z* 317 (M+H)⁺. HRMS *m*/*z* calcd for C₁₆H₂₀N₄O₃ (M)⁺ 316.1535, found 316.1535.

### EXAMPLE 76

### 2-(3-Hydroxyphenoxy)ethyl 3-(1-piperazinyl)-2-pyrazinyl ether.

The title compound was prepared according to the procedure of Example 69 starting from resorcinol. Yield 36%. MS *m*/*z* 317 (M+H)⁺. HRMS *m*/*z* calcd for C₁₆H₂₀N₄O₃ (M)⁺ 316.1535, found 316.1525.

### EXAMPLE 77

### 2-[3-(4-Morpholinyl)phenoxy]ethyl 3-(1-piperazinyl)-2-pyrazinyl ether.

1,1'-Azobis(*N,N*-dimethylformamide) (TMAD; 256 mg, 1.50 mmol) was added to a solution of 2-[3-(4*-tert*-butoxycarbonyl-1-piperazinyl)-2-pyrazinyloxy]ethanol (400 mg, 1.24 mmol; prepared in Example 52, Step 2), 3-(4-morpholinyl)phenol (441 mg, 1.23 mmol) and triphenylphosphine (646 mg, 2.46 mmol) in THF (3 mL). The reaction mixture was stirred at room temperature for 2.5 h and then concentrated. The residue was purified by repeated chromatography on silica gel using toluene and toluene/EtOAc (8:2) as eluents. Solvents were evaporated and the residue was treated with CH₂Cl₂/TFA/H₂O (1:0.9:0.1; 5 mL) at room temperature for 0.5 h. After concentration, 5 M NaOH was added followed by extraction with CH₂Cl₂. The organic phase was dried (K₂CO₃), filtered, and concentrated. The residue was purified by chromatography on silica gel using CHCl₃/MeOH (9:1) as eluent to give 50 mg (10%) of the title product as on oil. HRMS *m*/*z* calcd for C₂₀H₂₇N₅O₃ (M)⁺ 385.2114, found 385.2100.

Examples 78 - 82 were prepared according to the procedure of Example 77 starting from 2-[3-(4-*tert*-butoxycarbonyl-1-piperazinyl)-2-pyrazinyloxy]ethanol and the requisite phenolic compound.

### EXAMPLE 78

### 2-(1,3-Benzodioxol-4-yloxy)ethyl 3-(1-piperazinyl)-2-pyrazinyl ether.

The title compound was prepared starting from 1,3-benzodioxol-4-ol* (340 mg, 1.24 mmol) to give 125 mg (29%) of an oil. HRMS *m*/*z* calcd for C₁₇H₂₀N₄O₄ (M)⁺ 344.1485, found 344.1487.
*Prepared as described in *Chem. Pharm. Bull.* **1980,** *28*, 2414-2421.

### EXAMPLE 79

### 2-(2,3-Dihydro-1,4-benzodioxin-5-yloxy)ethyl 3-(1-piperazinyl)-2-pyrazinyl ether.

The title compound was prepared starting from 2,3-dihydro-1,4-benzodioxin-5-ol* (374 mg, 1.24 mmol) to give 260 mg (59%) of a solid: mp 98-99 °C. HRMS *m*/*z* calcd for C₁₈H₂₂N₄O₄ (M)⁺ 358.1641, found 358.1648.
*Prepared as described in *J. Am. Chem Soc.* **1959**, *81*, 5199-5201.

### EXAMPLE 80

### 2-(2-Allylphenoxy)ethyl 3-(1-piperazinyl)-2-pyrazinyl ether.

The title compound was prepared starting from 2-allylphenol (330 mg, 1.24 mmol) to give 200 mg (47%) of an oil. HRMS *m*/*z* calcd for C₁₉H₂₄N₄O₂ (M)⁺ 340.1899, found 340.1888.

### EXAMPLE 81

### 2-(3-Aminophenoxy)ethyl 3-(1-piperazinyl)-2-pyrazinyl ether.

The title compound was prepared starting from 3-aminophenol (268 mg, 1.24 mmol) to give 49 mg (13%) of an oil. HRMS *m*/*z* calcd for C₁₆H₂₁N₅O₂ (M)⁺ 315.1695, found 315.1705.

### EXAMPLE 82

### 2-(1-Oxoindanyl-4-yloxy)ethyl 3-(1-piperazinyl)-2-pyrazinyl ether.

The title compound was prepared starting from 4-hydroxy-1-indanone (365 mg, 1.24 mmol) to give 19 mg (4%) of an oil. HRMS *m*/*z* calcd for C₁₉H₂₂N₄O₃ (M)⁺ 354.1692, found 354.1705.

### EXAMPLE 83

### 2-(2,6-Difluorophenoxy)ethyl 3-(1-piperazinyl)-2-pyrazinyl ether.

The title compound was prepared according to the procedure of Example 77 starting from 2,6-difluorophenol (239 mg, 1.84 mmol), TMAD (384 mg, 2.25 mmol), 2-[3-(4-*tert*-butoxycarbonyl-1-piperazinyl)-2-pyrazinyloxy]ethanol (600 mg, 1.86 mmol; prepared in Example 52, Step 2), and triphenylphosphine (969 mg, 3.69 mmol) to give 279 mg (45%) of a solid: mp 101-102 °C. HRMS *m*/*z* calcd for C₁₆H₁₈F₂N₄O₂ (M)⁺ 336.1398, found 336.1403.

### EXAMPLE 84

### 5-Nitro-8-(2-{[3-(1-piperazinyl)-2-pyrazinyl]oxy}ethoxy)quinoline, Hydrochloride.

DEAD (0.485 mL, 3.08 mmol) was added to a stirred solution of 2-[3-(4-*tert*-butoxycarbonyl-1-piperazinyl)-2-pyrazinyloxy]ethanol (1.00 g, 3.08 mmol; prepared in Example 52, Step 2), 8-hydroxy-5-nitroquinoline (0.589 g, 3.08 mmol) and PPh₃ (0.85 g, 3.24 mmol) in THF (10 mL). The mixture was stirred at room temperature for 2 h, and concentrated. The residue was passed through a silica column using toluene/ EtOAc (1:1) as eluent. The *N*-Boc protected intermediate obtained was treated with CH₂Cl₂/TFA/H₂O (50:45:5; 15 mL) for 30 min at room temperature and concentrated. The residue was dissolved in 0.1 M aqueous HCl and washed with toluene. The water phase was concentrated to give 0.206 g (15%) of the title product. Pos-EI-MS shows M⁺ and 7 ions supporting the stated structure; HRMS *m*/*z* calcd for C₁₉H₂₀N₆O₄ (M)⁺ 396.1546, found 396.1557.

### EXAMPLE 85

### 6-Methoxy-7-(2-{[3-(1-piperazinyl)-2-pyrazinyl]oxy}ethoxy)-2H-chromen-2-one, Dihydrochloride.

DEAD (0.50 mL, 3.2 mmol) was added dropwise to a stirred mixture of 2-[3-(4-*tert*-butoxycarbonyl-1-piperazinyl)-2-pyrazinyloxy]ethanol (1.00 g, 3.08 mmol; prepared in Example 52, Step 2), scopoletin (0.60 g, 3.1 mmol) and triphenylphosphine (0.87 g, 3.3 mmol) in THF (10 mL) at room temperature. After 1 h, the reaction mixture was diluted with EtOAc and washed with water. The organic phase was dried, (MgSO₄), concentrated and the residue was purified by column chromatography on silica using toluene/EtOAc (7:3) as eluent. The pure fractions were combined and treated with CH₂Cl₂/TFA/H₂O (50:45:5; 5 mL) for 30 min at room temperature. The mixture was diluted with 0.2 M aqueous HCl and washed with EtOAc (x 3). The aqueous layer was concentrated to give 0.075 g (5%) of the title product. Pos-EI-MS shows M⁺ and 15 ions supporting the stated structure. Anal (C₂₀H₂₂N₄O₅ · 1.3 HCl · 1.4 H₂O) C, H, N.

### EXAMPLE 86

### 4-(2-{[3-(1-Piperazinyt)-2-pyrazinyl]oxy}ethoxy)-2,1,3-benzothiadiazole, Dihydrochloride.

### Step 1: tert-Butyl 4-{3-[2-(2,1,3-benzothiadiazol-4-yloxy)ethoxy]-2-pyrazinyl}-1-piperazinecarboxylate.

DEAD (0.52 mL, 3.3 mmol) was added to a slurry of 2-[3-(4-*tert*-butoxycarbonyl-1-piperazinyl)-2-pyrazinyloxy]ethanol (1.00 g, 3.08 mmol; prepared in Example 52, Step 2), 4-hydroxy-2,1,3-benzothiadiazole* (0.46 g, 3.0 mmol) and resin bound PPh₃ (1.1 g, 3.3 mmol) and shaken until HPLC showed no starting material. The mixture was filtered to remove the resin, concentrated and purified by column chromatography on silica to give 0.163 g (12%) of the title compound. Pos-EI-MS shows M⁺ and 11 ions supporting the stated structure. HRMS *m*/*z* calcd for C₂₁H₂₆N₆O₄S (M)⁺ 458.1736, found 458.1716.
*The starting material 4-hydroxy-2,1,3-benzothiadiazole was obtained as described in *Khim*. *Geterotsikl. Soedin* **1973**, *7*, 926-9.

### Step 2: 4-(2-{[3-(1-piperazinyl)-2-pyrazinyl]oxy}ethoxy)-2,1,3-benzothiadiazole, Dihydrochloride.

The product from Step 1 above (0.163 g, 0.356 mmol) was treated with CH₂Cl₂/TFA/H₂O (50:45:5; 5 mL) at room temperature for 30 min. The reaction mixture was concentrated and diluted with 0.1 M aqueous HCl and washed with toluene. The water phase was concentrated and the residue crystallized from MeOH/diethyl ether to give 0.088 g (57 %) of the title product. Pos-EI-MS shows M⁺ and 7 ions supporting the stated structure; HRMS *m*/*z* calcd for C₁₆H₁₈N₆O₂S (M)⁺ 358.1212, found 358.1207. Anal (C₁₆H₁₈N₆O₂S · 2HCl · H₂O) C, H, N.

### EXAMPLE 87

### 8-(2-{[3-(1-Piperazinyl)-2-pyrazinyl]oxy}ethoxy)quinazoline, Maleate.

DEAD (0.657 mL, 4.00 mmol) was added to a stirred solution of 8-quinazolinol* (0.55 g, 3.76 mmol), 2-[3-(4-*tert*-butoxycarbonyl-1-piperazinyl)-2-pyrazinyloxy]ethanol (1.216 g, 3.75 mmol; prepared in Example 52, Step 2) and PPh₃ (1.048 g, 4.00 mmol) in THF (12 mL). The reaction was stirred at room temperature for 2 h and then poured into 5% aqueous NaOH and extracted with EtOAc. The organic layer was dried (MgSO₄) and concentrated. The residue was purified by column chromatography on silica using toluene/EtOAc/MeOU (49:50:1). The pure fractions were combined and treated with CH₂Cl₂/TFA/H₂O (50:45:5; 5 mL) at room temperature for 30 minutes, poured into 0.5 M aqueous HCl and washed with EtOAc. The water phase was alkalinized to pH 12 and extracted with EtOAc, dried (MgSO₄), and concentrated to give 0.499 g (1.40 mmol) of the free base of the title compound. The free base was dissolved in dioxane (10 mL), and maleic acid (0.162 g, 1.40 mmol) in dioxane (2 mL) was added and the solution concentrated to give 0.65 g (37%) of the title product. Pos-EI-MS shows M⁺ and 9 ions supporting the stated structure. Anal (C₁₈H₂₀N₆O₂ · 1.3 C₄H₄O₄) C, H, N.
*The starting material 8-quinazolinol was obtained as described in *J Chem. Soc.* **1952,** 4985-4993.

### EXAMPLE 88

### 5-(2-{[3-(1-Piperazinyl)-2-pyrazinyl]oxy}ethoxy)quinoxaline, Hydrochloride.

TMAD (0.55 g, 3.20 mmol) was added to a stirred solution of 2-[3-(4-*tert*-butoxycarbonyl-1-piperazinyl)-2-pyrazinyloxy]ethanol (1.00 g, 3.08 mmol; prepared in Example 52, Step 2), 5-hydroxyquinoxaline* (0.45 g, 3.08 mmol) and PPh₃ (0.85 g, 3.24 mmol) in THF (20 mL). The reaction was stirred at room temperature for 1.5 h and at 65 °C for 2 h. The reaction mixture was concentrated and the residue was purified by column chromatography on silica using toluene/EtOAc (1:1) as eluent. The resulting solid was crystallized twice from diethyl ether/petroleum ether to remove PPh₃O. The recrystallized material (0.50 g) was dissolved in MeOH (15 mL) and 1 M HCl (3.5 mL) was added. The resulting mixture was stirred at room temperature for 4 h (only 10% *N*-Boc-deprotection had occurred according to HPLC/MS). The mixture was concentrated and the residue was treated with CH₂Cl₂/TFA/H₂O (50:45:5; 20 mL) for 1 h. After concentration *in vacuo,* the residue was dissolved in 0.1 M aqueous HCl and washed with toluene. The water phase was lyophilized to give a yellow oil that was dissolved in 1 M HCl. The solution was concentrated and MeOH and diethyl ether was added. The precipitate formed was collected and dried in vacuum to give 0.32 g (76%) of the title product. Pos-EI-MS shows M⁺ and 8 ions supporting the stated structure. HRMS *m*/*z* calcd for C₁₈H₂₀N₆O₂ (M)⁺ 352.1648, found 352.1662.
*The starting material 5-hydroxyquinoxaline was obtained as described in *J. Org. Chem.* **1951,** *16,* 438-442.

### EXAMPLE 89

### 1-[7-(2-{[3-(1-Piperazinyl)-2-pyrazinyl]oxy}ethory)-1-benzofuran-2-yl]-1-ethanone, Hydrochloride.

DEAD (0.787 mL, 5.00 mmol) was added to a stirred solution of 2-[3-(4-*tert*-butoxycarbonyl-1-piperazinyl)-2-pyrazinyloxy]ethanol (1.50 g, 4.63 mmol; prepared in Example 52, Step 2), 2-acetyl-7-hydroxybenzofuran (0.88 g, 5.0 mmol) and PPh₃ (1.31 g, 5.0 mmol) in THF (3 mL) under gentle heating. After being stirred for 30 min at room temperature, the reaction mixture was poured into water, extracted with EtOAc, dried (MgSO₄) and concentrated. The residue was purified by column chromatography on silica using toluene/EtOAc (7:3) as eluent. The pure fractions were combined and treated with CH₂Cl₂/TFA/H₂O (50:45:5; 5 mL) for 30 min at room temperature. The mixture was poured into 0.5 M aqueous HCl and washed with EtOAc. The aqueous layer was alkalinized, by addition of NaOH, to pH 12 and extracted with EtOAc. The organic layer was dried (MgSO₄), filtered and concentrated. The residue (0.250 g, 0.65 mmol) and pyridinium hydrochloride (0.075 g, 0.65 mmol) was dissolved in MeOH. The mixture was and concentrated and the residue was re-dissolved in EtOH and concentrated three more times to give 0.27 g (14 %) of the title product. Pos-EI-MS shows M⁺ and 19 ions supporting the stated structure. HRMS *m*/*z* calcd for C₂₀H₂₂N₄O₄ (M)⁺ 382.1641, found 382.1631.
*Previously reported in *J*. *Med. Chem.* **1978**, *21*, 536-542.

### EXAMPLE 90

### 3-(Phenoxy)propyl 3-(1-piperazinyl)-2-pyrazinyl ether, Dihydrochloride. Step 1: 2-Chloro-3-(1-piperazinyl)pyrazine.*

A mixture of 2,3-dichloropyrazine (1.35 g, 15.32 mmol), piperazine (2.34 g, 27.2 mmol) and K₂CO₃ (1.25 g, 9.04 mmol) in acetonitrile (5.5 mL) was stirred at 110 °C for 1.25 h in a sealed tube. The reaction mixture was diluted with CH₂Cl₂, filtered, and concentrated to give a yellowish semisolid residue which was purified by chromatography on silica gel using CHCl₃/MeOH (9: 1) as eluent. The obtained solid was redissolved in CHCl₃ and applied to a short (3 cm) plug of alumina. Elution with ether/CHCl₃ (9: 1) afforded 1.24 g (69%) of the title product as a white solid: mp 47-53 °C. HRMS *m*/*z* calcd for C₈H₁₁ClN₄ (M)⁺ 198.0672, found 198.0673.

### Step 2: 3-(Phenoxy)propyl 3-(1-piperazinyl)-2-pyrazinyl ether, Dihydrochloride.

2-Chloro-3-(1-piperazinyl)pyrazine (608 mg, 3.1 mmol; from Step 1 above) and 3-phenoxypropanol (570 mg, 3.7 mmol) was dissolved in dioxane (10 mL). KO-*t*-Bu (870 mg, 7.7 mmol) was added and the mixture was stirred at 95 °C for 6 h. The solvent was evaporated and the residue was purified by chromatography on silica gel using CH₂Cl₂/MeOH (9:1) as eluent to give the free base of the title compound as a yellow oil. The free base was converted to the dihydrochloride salt: yield 0.380 g (32%); mp 146-146.5 °C. Anal. (C₁₇H₂₂N₄O₂ · 2HCl) C, H, N.

Examples 91-161 were prepared according to the procedure of Example 90, Step 2, starting from 2-chloro-3-(1-piperazinyl)pyrazine and the appropriate alcohol.
Purification was by column chromatography on silica gel using CHC₃/MeOH (9:1) or CHCl₃/MeOH/NH₄OH (95:5:0.5) or other appropriate solvent system.

### EXAMPLE 91

### 2-(2-Trifluoromethylphenoxy)ethyl 3-(1-piperazinyl)-2-pyrazinyl ether.

### Step 1: 2-(2-Trifluoromethylphenoxy)ethanol.

A mixture of 2-trifluoromethylphenol (2.00 g, 12.3 mmol), K₂CO₃ (1.71 g, 12.3 mmol) and ethylene carbonate (1.20 g, 13.6 mmol) in dry DMF (30 mL) was heated at 150 °C for 1 h. After cooling, the reaction was quenched by addition of water (10 mL). The mixture was concentrated *in vacuo* and the residue partitioned between water (30 mL) and EtOAc (30 mL). The aqueous phase was extracted with additional portions of EtOAc (3 x 30 mL). The combined organic layers were treated with charcoal, dried over MgSO₄, and filtered through Celite. The filtrate was concentrated *in vacuo* and the remaining oil was purified by chromatography on silica gel using *n*-hexane/EtOAc (80:20) as eluent to give 0.50 g (20%) of the title product as white crystals: mp 74-77 °C. Anal. (C₉H₉F₃O₂) C, H.

### Step 2: 2-(2-Trifluoromethylphenoxy)ethyl 3-(1-piperazinyl)-2-pyrazinyl ether.

Yield 48%; mp 79-80 °C. Anal. (C₁₇H₁₉F₃N₄O₂) C, H, N.
*Previously described in US Patent No. 3,932,498.

### EXAMPLE 92

### 2-(2-Methylthiophenoxy)ethyl 3-(1-piperazinyl)-2-pyrazinyl ether, Maleate.

### Step 1: 2-(2-Methylthiophenoxy)ethanol.*

The title compound was prepared according to the procedure described in Example 91, Step 1, starting from 2-(methylthio)phenol. Yield 63%; mp 47-49 °C. Anal. (C₉H₁₂O₂S) C, H.

### Step 2: 2-(2-Methylthiophenoxy)ethyl 3-(1-piperazinyl)-2-pyrazinyl ether, Maleate.

Yield 68%; mp 160-162 °C. Anal. (C₁₇H₂₂N₄O₂S · C₄H₄O₄ · 0.1 THF) C, H, N.
*Previously described in *Tetrahedron* **1996***, 52,* 177-184.

### EXAMPLE 93

### 2-(2-Methylphenoxy)ethyl 3-(1-piperazinyl)-2-pyrazinyl ether, Maleate.

### Step 1: 2-(2-Methylphenoxy)ethanol.*

The title compound was prepared according to the procedure described in Example 91, Step 1, starting from *o*-cresol. Oil; yield 59%. HRMS *m*/*z* calcd for C₉H₁₂O₂ (M)⁺ 152.0837, found 152.0840.

### Step 2: 2-(2-Methylphenoxy)ethyl 3-(1-piperazinyl)-2-pyrazinyl ether, Maleate.

Yield 33%; mp 151-152 °C. Anal. (C₁₇H₂₂N₄O₂ · C₄H₄O₄ · 0.5 H₂O) C, H, N.
*Previously described in *J*. *Chem . Soc*. **1914**, 2117-2139.

### EXAMPLE 94

### 2-(2,5-Dimethylphenoxy)ethyl 3-(1-piperazinyl)-2-pyrazinyl ether.

### Step 1: 2-(2,5-Dimethylphenoxy)ethanol.*

The title compound was prepared according to the procedure described in Example 91, Step 1, starting from 2,5-dimethylphenol. Yield 60%; mp 45-48 °C. Anal. (C₁₀H₁₄O₂) C, H.

### Step 2: 2-(2,5-Dimethylphenoxy)ethyl 3-(1-piperazinyl)-2-pyrazinyl ether.

Oil; yield 65%. Anal. (C₁₈H₂₄N₄O₂) C, H, N.
*Previously described in *J*. *Indian Chem*. *Soc*. 1**962**, *39*, 5-8.

### EXAMPLE 95

### 2-(2-Fluorophenoxy)ethyl 3-(1-piperazinyl)-2-pyrazinyl ether, Maleate.

### Step 1: 2-(2-Fluorophenoxy)ethanol.*

The title compound was prepared according to the procedure described in Example 91, Step 1, starting from 2-fluorophenol. Oil; yield 71%. MS *m*/*z* 156 (M)⁺.

### Step 2: 2-(2-Fluorophenoxy)ethyl 3-(1-piperazinyl)-2-pyrazinyl ether, Maleate.

Yield 46%; mp 171-173 °C. Anal. (C₁₆H₁₉FN₄O₂ · C₄H₄O₄) C, H, N.
*Previously described in *J*. *Chem*. *Soc.* **1914**, 2117-2139.

### EXAMPLE 96

### 2-(2-Cyanophenoxy)ethyl 3-(1-piperazinyl)-2-pyrazinyl ether, Maleate.

### Step 1: 2-(2-Cyanophenoxy)ethanol.*

The title compound was prepared according to the procedure described in Example 91, Step 1, starting from 2-cyanophenol. Oil; yield 26%. MS *m*/*z* 163 (M)⁺.

### Step 2: 2-(2-Cyanophenoxy)ethyl 3-(1-piperazinyl)-2-pyrazinyl ether, Maleate.

Yield 40%; mp 165-166 °C. Anal. (C₁₇H₁₉N₅O₂ · C₄H₄O₄) C, H, N.
*Previously described in *J. Med.* Chem. **1987,** *30*, 939-943.

### EXAMPLE 97

### 2-[(1,1'Biphenyl)-2-yloxy]ethyl 3-(1-piperazinyl)-2-pyrazinyl ether.

### Step 1: 2-[(1,1'Biphenyl)-2-yloxy]ethanol.*

The title compound was prepared according to the procedure described in Example 91, Step 1, starting from 2-phenylphenol. Yield 52%; mp 71-75 °C. Anal. (C₁₄H₁₄O₂) C, H.

### Step 2: 2-[(1,1'Biphenyl)-2-yloxy]ethyl 3-(1-piperazinyl)-2-pyrazinyl ether.

Yield 53%; mp 101-103 °C. Anal. (C₂₂H₂₄N₄O₂) C, H, N.
*The starting material 4-phenoxybutanol was obtained as described in *J. Org. Chem.* **1965**, *30*, 2441-2447.

### EXAMPLE 98

### 4-(Phenoxy)butyl 3-(1-piperazinyl)-2-pyrazinyl ether, Dihydrochloride.*

Yield 49%; mp 129-131 °C. Anal. (C₁₈H₂₄N₄O₂) · 2HCl) C, H, N.

### EXAMPLE 99

### 2-[2-(2-Fluoro-5-methylphenoxy)ethoxy]-3-(1-piperazinyl)pyrazine, Fumarate. Step 1: 2-(2-Fluoro-5-methylphenoxy)ethanol.

The title compound was prepared according to the procedure described in Example 91, Step 1, starting from 2-fluoro-5-methylphenol. Oil; yield 94%. MS *m*/*z* 170 (M)⁺.

### Step 2: 2-[2-(2-Fluoro-5-methylphenoxy)ethoxy]-3-(1-piperazinyl)pyrazine, Fumarate.

Yield 71%; mp 145-146 °C. Anal. (C₂₁H₂₅FN₄O₆ · 0.3H₂O) C, H, N.
*Previously described in EP 331943.

### EXAMPLE 100

### 2-[2-(5-Fluoro-2-methoxyphenoxy)ethoxy]-3-(1-piperazinyl)pyrazine, Fumarate. Step 1: 2-(5-Fluoro-2-methoxyphenoxy)ethanol*.

The title compound was prepared according to the procedure described in Example 91, Step 1, starting from 5-fluoro-2-methoxyphenol**. Yield 89%. MS *m*/*z* 186 (M)⁺. Anal. (C₉H₁₁FO₃) C, H.
** Prepared as described in *Bull. Soc. Chim. Belg.* **1993,** *102,* 217-226.

### Step 2: 2-[2-(5-Fluoro-2-methoxyphenoxy)ethoxy]-3-(1-piperazinyl)pyrazine, Fumarate.

Yield 76%; mp 172-176 °C. Anal. (C₁₇H₂₁FN₄O₃ · C₄H₄O₄ · 0.3H₂O) C, H, N.
*Previously described in *Magn. Reson. Chem.* **1992,** *30*, 723-732.

### EXAMPLE 101

### 2-[2-(3-Fluorophenoxy)ethoxy]-3-(1-piperazinyl)pyrazine, Maleate.

### Step 1: 2-(3-Fluorophenoxy)ethanol*.

The title compound was prepared according to the procedure described in Example 91, Step 1, starting from 3-fluorophenol. Oil; yield 83%. MS *m*/*z* 156 (M)⁺.

### Step 2: 2-[2-(3-Fluorophenoxy)ethoxy]-3-(1-piperazinyl)pyrazine, Maleate.

Solid, yield 51%; mp 141-143 °C. Anal. (C₁₆H₁₉FN₄O₂ · C₄H₄O₄) C, H, N.
*Previously described in *J. Chem. Soc.* **1935**, 1098-1101 and in US 5025031.

### EXAMPLE 102

### 2-[2-(3-Methoxyphenoxy)ethoxy]-3-(1-piperazinyl)pyrazine, p-Toluenesulfonate.

### Step 1: 2-(3-Methoxyphenoxy)ethanol*.

The title compound was prepared according to the procedure described in Example 91, Step 1, starting from 3-methoxyphenol. Oil; yield 64%; MS *m*/*z* 168 (M)⁺. Anal. (C₉H₁₂O₃) C, H.

### Step 2: 2-[2-(3-Methoxyphenoxy)ethoxy]-3-(1-piperazinyl)pyrazine, p-Toluenesulfonate.

Solid; yield 20%; mp 131-133 °C. Anal. (C₁₇H₂₂N₄O₃ · C₇H₈O₃S) C, H, N.
*Previously described in *Helv. Chim Acta* **1989**, *72*, 1216-1224.

### EXAMPLE 103

### 2-[2-(3-Acetylphenoxy)ethoxy]-3-(1-piperazinyl)pyrazine.

### Step 1: 2-(3-Aeetylphenoxy)-1-ethanol.*

The title compound was prepared according to the procedure described in Example 91, Step 1, starting from 3-hydroxyacetophenone. Oil; yield 82%. MS *m*/*z* 180 (M)⁺. HRMS *m*/*z* calcd for C₁₀H₁₂O₃ (M)⁺ 180.0786, found 180.0787.

### Step 2: 2-[2-(3-Acetylphenoxy)ethoxy]-3-(1-piperazinyl)pyrazine, Maleate.

Yield of free base 68%. Part of the free base was converted to the maleate salt. MS *m*/*z* 343 (M+H)⁺. Anal. (C₁₈H₂₂N₄O₃ · C₄H₄O₄) C, H, N.
*Previously described in *J. Chem. Soc.* **1914**, 2117-2139 and *Pharmazie* **1975,** *30*, 353-357.

### EXAMPLE 104

### 2-[2-(3-Cyanophenoxy)ethoxy]-3-(1-piperazinyl)pyrazine.

### Step 1: 2-(3-Cyanophenoxy)-1-ethanol.*

The title compound was prepared according to the procedure described in Example 91, Step 1, starting from 3-cyanophenol. Oil; yield 63%. MS *m*/*z* 163 (M)⁺. Anal. (C₉H₉NO₂) C, H, N.

### Step 2: 2-[2-(3-Cyanophenoxy)ethoxy]-3-(1-piperazinyl)pyrazine.

Solid; yield 74%. MS *m*/*z* 326 (M+H)⁺. Anal. (C₁₇H₁₉N₅O₂) C, H, N.

### EXAMPLE 105

### 2-[2-(2,3-Difluorophenoxy)ethoxy]-3-(1-piperazinyl)pyrazine, Fumarate.

### Step 1: 2-(2,3-Difluorophenoxy)-1-ethanol.

The title product was prepared according to the procedure described in Example 91, Step 1, starting from 2,3-difluorophenol. Oil; yield 92%. MS *m*/*z* 174 (M)⁺. Anal (C₈H₈F₂O₂) C, H.

### Step 2: 2-[2-(2,3-Difluorophenoxy)ethoxy]-3-(1-piperazinyl)pyrazine, Fumarate.

Yield 35%; mp 125-130 °C. Anal. (C₁₆H₁₈F₂N₄O₂ · C₄H₄O₄) C, H, N.

### EXAMPLE 106

### 2-[2-(2,3,5-Trifluorophenoxy)ethoxy]-3-(1-piperazinyl)pyrazine, Fumarate.

### Step 1: 2-(2,3,5-Trifluorophenoxy)-1-ethanol.

The title product was prepared according to the procedure described in Example 91, Step 1, starting from 2,3,5-trifluorophenol. Oil; yield 83%. MS *m*/*z* 192 (M)⁺. Anal. (C₈H₇F₃O₂) C, H.

### Step 2: 2-[2-(2,3,5-Trifluorophenoxy)ethoxy]-3-(1-piperazinyl)pyrazine, Fumarate.

Yield 35%; mp 158-159 °C. MS *m*/*z* 354 (M)⁺; HRMS *m*/*z* calcd for C₁₆H₁₇F₃N₄O₂ (M)⁺ 354.1304, found 354.1303. Anal. (C₁₆H₁₇F₃N₄O₂ · C₄H₄O₄) C, H, N.
*Previously described in US 2077322 and EP 585500.

### EXAMPLE 107

### N-Phenyl-3-(2-{[3-(1-piperazinyl)-2-pyrazinyl]oxy}ethoxy)aniline, Fumarate.

### Step 1: 2-(3-Anilinophenoxy)-1-ethanol.*

The title product was prepared according to the procedure described in Example 91, Step 1, starting from 3-anilinophenol. Oil; yield 75% HRMS *m*/*z* calcd for C₁₄H₁₅NO₂ (M)⁺ 229.1103, found 229.1111.

### Step 2: N-Phenyl-3-(2-{[3-(1-piperazinyl)-2-pyrazinyl]oxy}ethoxy)aniline, Fumarate.

Yield of free base 24%. Part of the base was converted into its fumarate salt: mp 78-81 °C; HRMS *m*/*z* calcd for C₂₂H₂₅N₅O₂ (M)⁺ 391.2008, found 391.2001. Anal (C₂₂H₂₅N₅O · C₄H₄O₄) C, H, N.
*Previously described in WO 9733202.

### EXAMPLE 108

### [3-(2-{[3-(1-Piperazinyl)-2-pyrazinyl]oxy}ethoxy)phenyl]methanol, Maleate.

### Step 1: 3-(2-Hydroxyethoxy)benzaldehyde*

The title product was prepared according to the procedure described in Example 91, Step 1, starting from 3-hydroxybenzaldehyde. Oil; yield 88%; MS *m*/*z* 166 (M)⁺. Anal. (C₉H₁₀O₃ · 0.2 H₂O) C, H, N.

### Step 2: 2-[3-(Dimethoxymethyl)phenoxy]-1-ethanol.

*p*-TsOH (165 mg, 0.87 mmol) was added to a solution of the product obtained in Step 1 (1.45 g, 8.73 mmol) in dry MeOH (60 mL). After being stirred for 23 h at room temperature, more *p*-TsOH (170 mg, 0.89 mmol) was added. After additional 4 h of stirring, the reaction was quenched by the addition of NaHCO₃ (saturated aqueous solution, 70 mL) and H₂O (50 mL). The aqueous phase was extracted with EtOAc (3 x 100 mL). The combined organic phases were washed with brine and dried (MgSO₄). Evaporation of the solvents afforded a yellow oil that was used in the next step without purification. Yield 1.59 g (86 %). The product contained about 5% of starting material (¹H NMR).

### Step 3: 2-{2-[3-(Dimethoxymethyl)phenoxy]ethoxy}-3-(1-piperazinyl)pyrazine.

Oil; yield 63 %; MS *m*/*z* 344 (M-CH₂O)⁺. HRMS *m*/*z* calcd for C₁₈H₂₄N₄O₃ (M-CH₂O)⁺ 344.1848, found 344.1847.

### Step 4: 3-(2-{[3-(1-Piperazinyl)-2-pyrazinyl]oxy}ethoxy)benzaldehyde.

HOAc (12 mL) and H₂O (4 mL) was added to a solution of the product obtained in Step 3 (858 mg, 2.29 mmol) in THF (4 mL). The reaction mixture was stirred at 45 °C for 1.5 h and then diluted with EtOAc (150 mL). The organic phase was washed with brine (x 2), aqueous saturated NaHCO₃ and brine. Drying (MgSO₄) followed by evaporation of the solvents afforded the title product (0.41 g) as an acetate salt (1.7 · HOAc as determined by ¹H NMR). This product was used in the next step without purification.

### Step 5: [3-(2-{[3-(1-Piperazinyl)-2-pyrazinyl]oxy}ethoxy)phenyl]methanol, Maleate.

The product obtained in Step 4 (408 mg, 1.24 mmol) was dissolved in EtOH (99%, 35 mL). Immediately upon addition of NaBH₄ (293 mg, 7.75 mmol) the solution turned white. The reaction mixture was stirred at room temperature for 5 h and then quenched by addition of H₂O (7 mL). The mixture was filtered, and the filtrate diluted with aqueous saturated NaCl (30 mL) and H₂O (20 mL). The aqueous solution was extracted with EtOAc (x 4). The combined organic phases were washed with brine and dried (MgSO₄). Evaporation of the solvents gave a semi-solid residue (330 mg) that was purified by column chromatography on silica using gradient elution with EtOAc/MeOH (9:1) + 1 % NH₃ to EtOAc/MeOH (8:2) + 1 % NH₃. This afforded 76 mg (16%) of the free base of the title product as a semi-solid which was converted to its maleate salt: mp 123.2-123.5 °C; MS *m*/*z* 331 (M+H)⁺. HRMS *m*/*z* calcd for C₁₇H₂₂N₄O₃ (M)⁺ 330.1692, found 330.1692.

### EXAMPLE 109

### 2-{2-[3-(Methoxymethyl)phenoxy]ethoxy}-3-(1-piperazinyl)pyrazine, Maleate.

### Step 1: 2-[3-(Methoxymethyl)phenoxy]-1-ethanol.

Et₃SiH (4.0 mL, 25.0 mmol) was added to a solution of the product of Example 108, Step 1 (1.33 g, 8.00 mmol) in MeOH (14 mL). The mixture was cooled to 0 °C, and concentrated H₂SO₄ (1.5 mL) was added dropwise over 1.5 min. The reaction mixture was stirred at room temperature for 4.5 h. More Et₃SiH (1.5 mL, 9.4 mmol) was added, and the stirring was continued for 1 h. After solvent removal *in vacuo,* H₂O was added to the residue and pH was adjusted to pH 4 by addition of 2 M aqueous NaOH. The aqueous solution was extracted with CH₂Cl₂ (x 3). The combined organic phases were washed with brine, dried (MgSO₄) and concentrated to give an oil (1.8 g). NMR analysis of the crude product showed only 40% conversion. Therefore, the procedure was repeated by treating the residue with Et₃SiH (3 mL), MeOH (8 mL) and concentrated H₂SO₄ (1.2 mL). After being stirred for 4 h at room temperature more H₂SO₄ (1.2 mL) was added. The stirring was continued for a further hour and worked up as above. The crude product was purified by column chromatography on silica [gradient 100 % isohexane to isohexane/EtOAc (1:1)] to give the title compound as a colorless oil. Yield 0.98 g (67 %); MS *m*/*z* 182 (M)⁺. Anal. (C₁₀H₁₄O₃ · 0.1 H₂O) C, H.

### Step 2: 2-{2-[3-(Methoxymethyl)phenoxy]ethoxy}-3-(1-piperazinyl)pyrazine, Maleate.

Yield of free base of the title compound 0.61 g (80 %). Part of this material was converted to the maleate salt: mp 114-115 °C; MS *m*/*z* 345 (M+H)⁺.
Anal. (C₁₈H₂₄N₄O₃ · C₄H₄O₄) C, H, N.
*Previously reported in WO 8606628.

### EXAMPLE 110

### 3-(2-{[3-(1-Piperazinyl)-2-pyrazinyl]oxy}ethoxy)benzamide, Maleate.

### Step 1: 3-(2-Hydroxyethoxy)benzamide.*

The title compound was prepared according to the procedure described in Example 91, Step 1, starting from 3-hydroxybenzamide. Yield 62 %; MS *m*/*z* 181 (M)⁺. HRMS *m*/*z* calcd for C₉H₁₁NO₃ (M)⁺ 181.0739, found 181.0739.

### Step 2: 3-(2-{[3-(1-Piperazinyl)-2-pyrazinyl]oxy}ethoxy)benzamide, Maleate.

Yield of free base of the title compound 43%. The maleate salt was prepared: mp 134-137 °C; MS *m*/*z* 344 (M+H)⁺. Anal. (C₁₇H₂₁N₅O₃ · C₄H₄O₄) C, H, N.
*Previously described in EP 420790.

### EXAMPLE 111

### N-Phenyl-4-(2-{[3-(1-piperazinyl)-2-pyrazinyl]oxy}ethoxy)aniline, Maleate.

### Step 1: 2-(4-Anilinophenoxy)ethanol.*

The title product was prepared according to the procedure described in Example 91, Step 1, starting from 4-anilinophenol. Solid; yield 0.85 g (75%); MS *m*/*z* 229 (M)⁺. Anal. (C₁₄H₁₅NO₂) C, H, N.

### Step 2: N-Phenyl-4-(2-{[3-(1-piperazinyl)-2-pyrazinyl]oxy}ethoxy)aniline, Maleate.

Yield of free base of the title compound 44 %. The maleate salt was prepared: mp 132-133 °C; MS *m*/*z* 392 (M+H)⁺. Anal. (C₂₂H₂₅N₅O₂ · C₄H₄O₄) C, H, N.
*Previously described in *J*. *Heterocycl. Chem.* **1994,** *31*, 457-480.

### EXAMPLE 112

### Step 1: N-[4-(2-Hydroxyethoxy)phenyl]acetamide.*

The title product was prepared according to the procedure described in Example 91, Step 1, starting from 4-acetamidophenol. Solid; yield 1.38 g (52 %); MS *m*/*z* 195 (M)⁺. Anal. (C₁₀H₁₃NO₃ · 0.1 H₂O) C, H, N.

### Step 2: N-[4-(2-{[3-(1-Piperazinyl)-2-pyrazinyl]oxy}ethoxy)phenyl]acetamide.

Solid; yield 0.47 g (53 %); MS *m*/*z* 357 (M)⁺. HRMS *m*/*z* calcd for C₁₈H₂₃N₅O₃ (M)⁺ 357.1801, found 357.1810.

### EXAMPLE 113

### 2-(1-Piperazinyl)-3-{2-[3-(trifluoromethoxy)phenoxy]ethoxy}pyrazine, Maleate. Step 1: 2-[3-(Trifluoromethoxy)phenoxy]ethanol.

The title product was prepared according to the procedure described in Example 91, Step 1, starting from 3-trifluoromethoxyphenol. Oil; yield 0.75 g (60 %); MS *m*/*z* 222 (M)⁺. Anal. (C₉H₉F₃O₃) C, H.

### Step 2: 2-(1-Piperazinyl)-3-{2-[3-(trinuoromethoxy)phenoxy]ethoxy}pyrazine, Maleate.

Yield of free base of the title compound 85 %. The maleate salt was prepared. MS *m*/*z* 385 (M+H)⁺. Anal. (C₁₇H₁₉F₃N₄O₃ · C₄H₄O₄ 0.03 Et₂O) C, H, N.

### EXAMPLE 114

### 2-[2-(3,5-Difluorophenoxy)ethoxy]-3-(1-piperazinyl)pyrazine, p-Toluenesulfonate,

### Step 1: 2-(3,5-Difluorophenoxy)-1-ethanol.

In a sealed Pyrex tube, a mixture of 3,5-difluorophenol (2.19 g, 16.8 mmol), ethylene carbonate (1.0 g, 11.4 mmol), a catalytic amount ofNaH (60% in mineral oil) in DMF (1 mL) was reacted in a Labwell MW-10 microwave reactor at 50W for 3 min. The reaction mixture was diluted with toluene and washed with 5% aqueous NaOH. The organic phase was dried (MgSO₄) and concentrated. This gave 1.59 g (80%) of the title compound as a light red oil. MS *m*/*z* 174 (M)⁺.

### Step 2: 2-[2-(3,5-Difluorophenoxy)ethoxy]-3-(1-piperazinyl)pyrazine, p-Toluenesulfonate.

Yield 14%; mp 118-119 °C. Anal. (C₁₆H₁₈F₂N₄O₂ · C₇H₈O₃S) C, H, N.
*Previously described in *J. Org. Chem.* **1960,** *25,* 626-632.

### EXAMPLE 115

### 2-[2-(1,3-Benzodioxol-5-yloxy)ethoxy]-3-(1-piperazinyl)pyrazine.

### Step 1: 2-(1,3-Benzodioxol-5-yloxy)ethanol.*

The title compund was prepared according to the procedure of Example 114, Step 1 starting from sesamol (2.0 g, 14.5 mmol) and ethylene carbonate (1.0 g, 11.4 mmol): solid; yield 99%. MS *m*/*z* 182 (M)⁺.

### Step 2: 2-[2-(1,3-Benzodioxol-5-yloxy)ethoxy]-3-(1-piperazinyl)pyrazine.

Solid; yield 71%. MS *m*/*z* 345 (M+H)⁺. Anal. (C₁₇H₂₀N₄O₄) C, H, N.

### EXAMPLE 116

### 2-[2-(3-Butoxyphenoxy)ethoxy]-3-(1-piperazinyl)pyrazine, Maleate.

### Step 1: 2-(3-Butoxyphenoxy)ethanol.

The title compund was prepared according to the procedure of Example 114, Step 1 starting from 3-n-butoxyphenol (2.0 g, 12 mmol) and ethylene carbonate (0.88 g, 10 mmol). Oil; yield 85%. MS *m*/*z* 210 (M)⁺.

### Step 2: 2-[2-(3-Butoxyphenoxy)ethoxy]-3-(1-piperazinyl)pyrazine, Maleate.

Yield of free base of the title compound 77%. Part of the free base was converted to the maleate salt. MS *m*/*z* 373 (M+H)⁺. Anal. (C₂₀H₂₈N₄O₃ · C₄H₄O₄) C, H, N.

### EXAMPLE 117

### 2-[2-(3-Trifluoromethylphenoxy)ethoxy]-3-(1-piperazinyl)pyrazine.

### Step 1: 2-(3-Trifluoromethylphenoxy)ethanol.

The title compound was prepared according to the procedure of Example 114, Step 1 starting from 3-trifluoromethylphenol (3.89 g, 24 mmol) and ethylene carbonate (1.76 g, 20 mmol) with the exception that no DMF was used. Oil; yield 64%. MS *m*/*z* 206 (M)⁺.

### Step 2: 2-[2-(3-Trifluoromethylphenoxy)ethoxy]-3-(1-piperazinyl)pyrazine, Maleate.

Yield of free base of the title compound 65%. Part of the free base was converted to the maleate salt. MS *m*/*z* 369 (M+H)⁺. HRMS *m*/*z* calcd for C₁₇H₁₉F₃N₄O₂ (M)⁺ 368.1460, found 368.1460.
*Previously described in US 2140824.

### EXAMPLE 118

### 2-[2-([1,1'-Biphenyl]-3-yloxy)ethoxy]-3-(1-piperazinyl)pyrazine, Maleate.

### Step 1: 2-(3-Phenylphenoxy)ethanol.*

The title compund was prepared according to the procedure of Example 114, Step 1 starting from 3-phenylphenol (1.72 g, 10.1 mmol) and ethylene carbonate (0.80 g, 9.1 mmol) and DMF (3 mL). Semi-solid; yield 86%. MS *m*/*z* 214 (M)⁺.

### Step 2: 2-[2-([1,1'-Biphenyl]-3-yloxy)ethoxy]-3-(1-piperazinyl)pyrazine, Maleate.

Yield of free base 70%. Part of the free base was converted to the maleate salt.
MS *m*/*z* 377 (M+H)⁺. Anal. (C₂₂H₂₄N₄O₂ · C₄H₄O₄) C, H, N.
*Previously described in *J. Am. Chem. Soc.* **1939**, *61*, 355-357.

### EXAMPLE 119

### 2-[2-(3-Acetamidophenoxy)ethoxy]-3-(1-piperazinyl)pyrazine, Maleate.

### Step 1: 2-(3-Acetamidophenoxy)-1-ethanol*.

The title product was prepared according to the procedure described in Example 114, Step 1, starting from 3-acetamidophenol (2.10 g, 13.9 mmol) and ethylene carbonate (0.88 g, 10 mmol). The crude product was purified by column chromatography on silica using toluene/EtOAc/MeOH (49:49:2). Oil; yield 99%. MS *m*/*z* 195 (M)⁺.

### Step 2: 2-[2-(3-Acetamidophenoxy)ethoxy]-3-(1-piperazinyl)pyrazine, Maleate.

Yield 38%; mp 154-155 °C. Anal. (C₁₈H₂₃N₅O₃ · C4H404) C, H, N.

### EXAMPLE 120

### 2-[4-(2-{[3-(1-Piperazinyl)-2-pyrazinyl]oxy}ethoxy)phenoxy]ethanol, Maleate.

The title product was prepared from 1,4-bis(2-hydroxyethoxy)benzene. Yield of free base of the title compound 51%. The maleate salt was prepared: mp 127-129 °C. MS *m*/*z* 361 (M+H). Anal. (C₁₈H₂₄N₄O₄ · C₄H₄O₄) C, H, N.

### EXAMPLE 121

### 2-[3-(2-{[3-(1-Piperazinyl)-2-pyrazinyl]oxy}ethoxy)phenoxy]ethanol, Maleate.

The title product wasp prepared from 1,3-bis(2-hydroxyethoxy)benzene. Yield of free base of the title compound 45%. The maleate salt was prepared: mp 111-114 °C; MS *m*/*z* 361 (M+H)⁺. Anal. (C₁₈H₂₄N₄O₄ · C₄H₄O₄ · 0.1 H₂O) C, H, N.
*The starting material benzofuran-2-ylmethanol was obtained from benzofuran-2-carboxaldehyde by reduction with sodium borohydride.

### EXAMPLE 122

### 2-(Benzofuran-2-ylmethoxy)-3-(1-piperazinyl)pyrazine, Hydrochloride.*

Yield 44%; mp 160-161 °C. Anal. (C₁₇H₁₈N₄O₂ · HCl) C, H, N.

### EXAMPLE 123

### 2-(2,3-Dihydrobenzofuranyl-2-ylmethoxy)-3-(1-piperazinyl)pyrazine, Dihydrochloride.

Yield 33%; mp 172-174 °C. Anal. (C₁₇H₂₀N₄O₂ · 2HCl) C, H, N.
The starting material 2,3-dihydrobenzofuran-2-ylmethanol* was obtained from benzofuran-2-carboxaldehyde by reduction with sodium borohydride and subsequent catalytic hydrogenation.
*Previously described in *Bioorg. Med. Chem. Lett.* **1999,** *9*, 401-406.

### EXAMPLE 124

### 2-(1-Piperazinyl)-3-(tetrahydro-2-furanylmethoxy)pyrazine, Hydrochloride.

The title compound was prepared starting from tetrahydrofurfuryl alcohol. Yield 65%; mp 130-136 °C. Anal. (C₁₃H₂₀N₄O₂ · HCl · H₂O) C, H, N.

### EXAMPLE 125

### 2-(1-Piperazinyl)-3-[3-(2-pyridinyl)propoxy]pyrazine, Maleate.

The title compound was prepared starting from 2-pyridinepropanol. Yield 51%; mp 147-148 °C. Anal. (C₁₆H₂₁N₅O · 1.05 C₄H₄O₄) C, H, N.

### EXAMPLE 126

### 2-(1-Piperazinyl)-3-[3-(3-pyridinyl)propoxy]pyrazine, Maleate.

The title compound was prepared starting from 3-pyridinepropanol.
Yield 43%; mp 128-130 °C. Anal. (C₁₆H₂₁N₅O · C₄H₄O₄) C, H, N.

### EXAMPLE 127

### 2-(1-Piperazinyl)-3-[3-(4-pyridinyl)propoxy]pyrazine, Maleate.

The title compound was prepared starting from 4-pyridinepropanol.
Yield 41%; mp 90-91 °C. Anal. (C₁₆H₂₁N₅O · C₄H₄O₄ · 0.7 H₂O) C, H, N.

### EXAMPLE 128

### 2-[3-(6-Methyl-2-pyridinyl)propoxy]-3-(1-piperazinyl)pyrazine, Maleate.

The title compound was prepared starting from 6-methyl-2-pyridinepropanol.
Yield 30%; mp 134-136 °C. Anal. (C₁₇H₂₃N₅O · C₄H₄O₄) C, H, N.

### EXAMPLE 129

### 2-[(E)-3-Phenyl-2-propenyloxy]-3-(1-piperazinyl)pyrazine.

The title compound was prepared starting from cinnamyl alcohol.
Oil; yield 39%. Anal. (C₁₇H₂₀N₄O · 0.2 H₂O) C, H, N.
*The starting material 3,4-dihydro-2H-1,4-benzoxazin-2-ylmethanol was prepared as described in *J. Heterocycl. Chem.* **1996***, 33,* 191-196.

### EXAMPLE 130

### 2-(3,4-Dihydro-2H-1,4-benzoxazin-2-ylmethoxy)-3-(1-piperazinyl)pyrazine, Dihydrochloride.*

Yield 22%; mp 171-175 °C. Anal. (C₁₇H₂₁N₅O₂ · 2HCl) C, H, N.
* The starting material 3,4-dihydro-4 methyl-2*H*-1,4-benzoxazin-2-ylmethanol has previously been described in *J. Heterocycl. Chem.* **1996***, 33,* 191-196.

### EXAMPLE 131

### 2-(4-Methyl-3,4-dihydro-2H-1,4-benzoxazin-2-ylmethoxy)-3-(1-piperazinyl)-pyrazine, Hydrochloride.*

Yield 49%; mp 119°C (dec). Anal. (C₁₈H₂₃N₅O₂ · 1.33 HCl) C, H, N.
*The starting material 2,3-dihydro-1,4-benzoxathiin-2-yl)-methanol was prepared as described in *J. Pharm. Sci.* **1972,** *61,* 228-231.

### EXAMPLE 132

### 2-(2,3- Dihydro-1,4-benzoxathiin-2-ylmethoxy)-3-(1-piperazinyl)pyrazine, Fumarate.*

Yield 41 %; mp 178-79 °C. Anal. (C₁₇H₂₀N₄O₂S · C₄H₄O₄) C, H, N.
*The starting material 3,4-dihydro-2*H*-chromen-2-ylmethanol was prepared as described in *Eur. J. Med. Chem.* **1985,** *20,* 117-120.

### EXAMPLE 133

### 2-(3,4-Dihydro-2H-chromen-2-ylmethoxy)-3-(1-piperazinyl)pyrazine, Hydrochloride.*

Yield 45%; mp 170-175 °C. Anal. (C₁₈H₂₂N₄O₂ · HCl) C, H, N.

### EXAMPLE 134

### 2-(5-Isoquinolinyloxy)ethyl 3-(1-piperazinyl)-2-pyrazinyl ether, Maleate.

### Step 1: 2-(5-Isoquinolinyloxy)ethanol.

A mixture of 5-hydroxyisoquinoline (2.05 g, 14.1 mmol), ethylene carbonate (1.43 g, 16.2 mmol), K₂CO₃ (0.97 g, 7.1 mmol) in DMF (6 mL) was heated at 155 °C for 1.5 h under stirring in a sealed tube. After cooling, the mixture was diluted with CHCl₃ and filtered through a pad of Celite. The brownish filtrate was concentrated *in vacuo,* and the residue was purified by chromatography on silica gel using EtOAc as eluent to give 1.8 g (67%) of the title product as a beige oil which solidified on standing: mp 64-67 °C; HRMS *m*/*z* calcd for C₁₁H₁₁NO₂ (M)⁺ 189.0790, found 189.0789. Anal. (C₁₁H₁₁NO₂) C, H, N.

### Step 2: 2-(5-Isoquinolinyloxy)ethyl 3-(1-piperazinyl)-2-pyrazinyl ether, Maleate.

Yield 55% as the free base. The free base was converted to the maleate salt: mp 178-180.5 °C; HRMS *m*/*z* calcd for C₁₉H₂₁N₅O₂ (M)⁺ 351.1695, found 351.1694. Anal. (C₁₉H₂₁N₅O₂ · C₄H₄O₄) C, H, N.

### EXAMPLE 135

### 2-(5-Quinolinyloxy)ethyl 3-(1-piperazinyl)-2-pyrazinyl ether, Maleate.

### Step 1: 2-(5-Quinolinyloxy)ethanol

The title compound was prepared according to the procedure described in Example 134, Step 1, starting from 5-hydroxyquinoline (0.93 g, 6.40 mmol). Yield 1.19 g (91 %); mp 109-111 °C; HRMS *m*/*z* calcd for C₁₁H₁₁NO₂ (M)⁺ 189.0790, found 189.0786. Anal. (C₁₁H₁₁NO₂) C, H, N.

### Step 2: 2-(5-Quinolinyloxy)ethyl 3-(1-piperazinyl)-2-pyrazinyl ether, Maleate.

Yield 61% as the free base. The free base was converted to the maleate salt: mp 132-135 °C; HRMS *m*/*z* calcd for C₁₉H₂₁N₅O₂ (M)⁺ 351.1695, found 351.1681. Anal. (C₁₉H₂₁N₅O₂ · 1.8C₄H₄O₄) C, H, N.

### EXAMPLE 136

### 2-(6-Quinolinyloxy)ethyl 3-(1-piperazinyl)-2-pyrazinyl ether, Maleate.

### Step 1: 2-(6-Quinolinyloxy)ethanol.

The title compound was prepared according to the procedure described in Example 91, Step 1, starting from 6-hydroxyquinoline. Yield 78%; mp 68-70 °C. Anal. (C₁₁H₁₁NO₂) C, H, N.

### Step 2: 2-(6-Quinolinyloxy)ethyl 3-(1-piperazinyl)-2-pyrazinyl ether, Maleate.

Yield 33%; mp 167-169 °C. Anal. (C₁₉H₂₁N₅O₂ · C₄H₄O₄) C, H, N.

### EXAMPLE 137

### 2-(7-Quinolinyloxy)ethyl 3-(1-piperazinyl)-2-pyrazinyl ether, Maleate.

### Step 1: 2-(7-Quinolinyloxy)ethanol.

The title compound was prepared according to the procedure described in Example 91, Step 1, starting from 7-hydroxyquinoline. Yield 76%; mp 93-95 °C. Anal. (C₁₁H₁₁NO₂) C, H, N.

### Step 2: 2-(7-Quinolinyloxy)ethyl 3-(1-piperazinyl)-2-pyrazinyl ether, Maleate.

Yield 39%; mp 156-158 °C. Anal. (C₁₉H₂₁N₅O₂ · C₄H₄O₄) C, H, N.
*Previously described in *Pharm. Chem. J.* **1994,** *28*, 934-936.

### EXAMPLE 138

### 2-(8-Quinolinyloxy)ethyl 3-(1-piperazinyl)-2-pyrazinyl ether.

### Step 1: 2-(8-Quinolinyloxy)ethanol.*

The title compound was prepared according to the procedure described in Example 91, Step 1, starting from 8-hydroxyquinoline. Yield 48%; mp 82-84 °C. Anal. (C₁₁H₁₁NO₂) C, H, N.

### Step 2: 2-(8-Quinolinyloxy)ethyl 3-(1-piperazinyl)-2-pyrazinyl ether.

Yield 62%; mp 98-100 °C. Anal. (C₁₉H₂₁N₅O₂ · 0.05 EtOAc) C, H, N.

### EXAMPLE 139

### 5-Chloro-8-(2-{[3-(1-piperazinyl)-2-pyrazinyl]oxy}ethoxy)-2-quinolinamine, Fumarate. Step 1: 2-[(5-Chloro-8-quinolinyl)oxy]-1-ethanol.

The title compound was prepared according to the procedure described in Example 91, Step 1, starting from 5-chloro-8-hydroxyquinoline (3.59 g, 20 mmol). Yield 48 %. Pos-EI-MS shows M⁺ and 5 ions supporting the stated structure; HRMS *m*/*z* calcd for C₁₁H₁₀ClNO₂ (M)⁺ 223.0400, found 223.0392. Anal. (C₁₁H₁₀ClNO₂) C, H, N.

### Step 2: 2-(5-Chloro-8-quinolinyloxy)ethyl 3-(1-piperazinyl)-2-pyrazinyl ether, Fumarate.

The crude product was purified by column chromatography on silica using CHCl₃/MeOH/NH₄OH (90:10:0.3) as eluent. After evaporation of solvents from the combined pure fractions, the resulting free base was converted to its fumaric acid salt. Recrystallization from water afforded 1.22 g (50%) of the title product as white crystals: mp 178 °C (dec). Anal. (C₁₉H₂₀ClN₅O₂ · C₄H₄O₄) C, H, N.

### EXAMPLE 140

### 2-(Benzofuran-7-yloxy)ethyl 3-(1-piperazinyl)-2-pyrazinyl ether, Maleate.

### Step 1: 2-(Benzofuran-7-yloxy)ethanol.

The title compound was prepared according to the procedure described in Example 91, Step 1, starting from 7-hydroxybenzofuran* and was isolated as a semi-solid; yield 79%.
*Prepared as described in *J*. *Med Chem.* **1987,** *30*, 62-67.

### Step 2: 2-(Benzofuran-7-yloxy)ethyl 3-(1-piperazinyl)-2-pyrazinyl ether, Maleate.

Yield 42% as the free base. A portion of the free base was converted to the maleate salt: mp 177-179 °C; MS *m*/*z* 341 (M + H)⁺. Anal. (C₁₈H₂₀N₄O₃ · C₄H₄O₄) C, H, N.

### EXAMPLE 141

### 7-(2-{[3-(1-Piperazinyl)-2-pyrazinyl] oxy}ethoxy)-1H-indole.

### Step 1: 2-(1H-Indol-7-yloxy)-1-ethanol.

A mixture of 7-hydroxyindole (1.00 g, 7.51 mmol) and ethylene oxide (0.33 g, 7.50 mmol) in dioxane (3 mL) was placed in a reaction tube. NaH (60% in oil; 0.100 g, 2.45 mmol) was added and the reaction mixture was stirred at 100 °C for 5 days. The reaction mixture was poured into water, extracted with EtOAc, dried (MgSO₄) and concentrated. The residue was purified by chromatography on silica gel using toluene/EtOAc (1:1) as eluent to give 0.45 g (34%) of the title product. Pos-EI-MS shows M⁺ and 5 ions supporting the stated structure. HRMS *m*/*z* calcd for C₁₀H₁₁NO₂ (M)⁺ 177.0790, found 177.0799.

### Step 2: 7-(2-{[3-(1-Piperazinyl)-2-pyrazinyl]oxy}ethoxy)-1H-indole

The product obtained in Step 1 above (0.200 g, 1.13 mmol) and dioxane (4 mL) was placed in a reaction tube and NaH (60% in oil; 0.060 g, 1.50 mmol) was added. When the gas evolution had ceased, 2-chloro-3-(1-piperazinyl)pyrazine (0.228 g, 1.15 mmol; from Example 90, Step 1) was added and the tube sealed. The mixture was stirred at 100 °C for 3 h. After cooling, the reaction mixture was poured into water and extracted with EtOAc. The organic layer was extracted with 0.5 M aqueous HCl. The pH of the aqueous phase was adjusted to pH 11 with NaOH and extracted with EtOAc. The organic layer was dried (MgSO₄) and concentrated to give 0.31 g (81%) of the title product. Pos-EI-MS shows M⁺ and 9 ions supporting the stated structure. HRMS *m*/*z* calcd for C₁₈H₂₁N₅O₂ (M)⁺ 339.1695, found 339.1696.

### EXAMPLE 142

### 6-(2-{[3-(1-Piperazinyl)-2-pyrazinyl]oxy}ethoxy)-1H-indole.

### Step 1: 2-(1H-Indol-6-yloxy)-1-ethanol.

A mixture of 6-hydroxyindole (2.66 g, 20 mmol) and K₂CO₃ (3.04 g, 22 mmol) was stirred in DMF (10 mL) at 80 °C for 10 min. A solution of ethylene carbonate (1.94 g, 22 mmol) in DMF (4 mL) was added and the mixture was heated at 125 °C for 3 h. The mixture was concentrated, diluted with water, and extracted with toluene. The organic phase was washed with 1 M aqueous Na₂CO₃ and brine. The organic layer was dried (Na₂SO₄) and concentrated. The residue was purified by flash chromatography on silica using EtOAc/toluene (1:4 → 36:65) as eluent. Yield 1.02 g (29%); mp 74-77 °C; HRMS *m*/z calcd for C₁₀H₁₁NO₂ (M)⁺ 177.0790, found 177.0784. Anal. (C₁₀H₁₁NO₂) C, H, N.

### Step 2: 6-(2-{[3-(1-Piperazinyl)-2-pyrazinyl]oxy}ethoxy)-1H-indole.

Yield 59%; mp 111-113 °C; MS-EI *m*/*z* 339 (M)⁺. Anal. (C₁₈H₂₁N₅O₂) C, H, N.

### EXAMPLE 143

### 2-Methyl-5-(2-{[3-(1-piperazinyl)-2-pyrazinyl]oxy}ethoxy)-1,3-benzothiazole, Hydrochloride.

### Step 1 : 2-[(2-Methyl-1,3-benzothiazol-5-yl)oxy]ethanol.

The title compound was prepared according to the procedure described in Example 91, Step 1, starting from 2-methyl-1,3-benzothiazol-5-ol. Yield 61%; mp 67-68 °C. HRMS *m*/*z* calcd for C₁₀H₁₁NO₂S (M)⁺ 209.0510, found 209.0502. Anal. (C₁₀H₁₁NO₂S) C, H, N.

### Step 2: 2-Methyl-5-(2-{[3-(1-piperazinyl)-2-pyrazinyl]oxy}ethoxy)-1,3-benzothiazole, Hydrochloride.

Yield 58%; mp 188-190 °C. HRMS *m*/*z* calcd for C₁₈H₂₁N₅O₂S (M)⁺ 371.1416, found 371.1421. Anal. (C₁₈H₂₁N₅O_{2S} · HCl · 0.3 H₂O) C, H, N.

### EXAMPLE 144

### 2-[2-(2-Methoxy-5-methylphenoxy)ethoxy]-3-(1-piperazinyl)pyrazine, Maleate. Step 1: 2-(2-Methoxy-5-methylphenoxy)ethanol.

The title compound was prepared according to the procedure described in Example 91, Step 1, starting from 2-methoxy-5-methylphenol. Yield 56%; mp 42-43 °C. Anal. (C₁₀H₁₄O₃) C, H.

### Step 2: 2-[2-(2-Methoxy-5-methylphenoxy)ethoxy]-3-(1-piperazinyl)pyrazine, Maleate.

Yield 56%; mp 148-149 °C. Anal. (C₁₈H₂₄N₄O₃ · C₄H₄O₄). C, H, N.

### EXAMPLE 145

### 7-(2-{[3-(1-Piperazinyl)-2-pyrazinyl]oxy}ethoxy)-1-naphthylamine, Maleate.

### Step 1: 2-[(8-Amino-2-naphthyl)oxy]ethanol, Hydrochloride.

The title compound was prepared according to the procedure described in Example 91, Step 1, starting from 8-amino-2-naphthol. Yield 40%; mp 185 °C (dec). Anal. (C₁₂H₁₃NO₂ · HCl) C, H, N.

### Step 2: 7-(2-{[3-(1-Piperazinyl)-2-pyrazinyl]oxy}ethoxy)-1-naphthylamine, Maleate.

Yield 21%; mp 120 °C (dec). Anal. (C₂₀H₂₃N₅O₂ · 1.7C₄H₄O₄) C, H, N.

### EXAMPLE 146

### 2-(7-Nitro-2,3-dihydro-1,4-benzodioxin-2-ylmethoxy)-3-(1-piperazinyl)pyrazine.

### Step 1: 7-Nitro-2-hydroxymethyl-2,3-dihydro-1,4-benzodioxine.

Trifluoroacetic anhydride (20 mL, 149 mmol) was added dropwise to solution of 2-hydroxymethyl-2,3-dihydro-1,4-benzodioxine (6.64 g, 40 mmol) and NH₄NO₃ (84.8 g, 60 mmol) in CH₂Cl₂ (300 mL). The reaction mixture was stirred at room temperature for 4 h and then concentrated under reduced pressure. The residue was purified by chromatography on silica gel using EtOAc/isohexane (1:3 to 2:3) as eluent to give a small fraction (0.3 g, 4%) of the pure title compound and 6.5 g of a mixture of the C6- and C7 regioisomeric nitro derivatives. The regioisomeric mixture was taken on to Example 147 Step 1, without separation.

### Step 2: 2-(7-Nitro-2,3-dihydro-1,4-benzodioxin-2-ylmethoxy)-3-(1-piperazinyl)pyrazinc.

The title compound was prepared starting from the pure product obtained in Step 1 above. Oil; yield 43%; mp 133-135 °C. HRMS *m*/*z* calcd for C₁₇H₁₉N₅O₅ (M)⁺ 373.1386, found 373.1367.

### EXAMPLE 147

### 2-(7-Acetamido-2,3-dihydro-1,4-benzodioxin-2-ylmethoxy)-3-(1-piperazinyl)pyrazine.

### Step 1: 7-Amino-2-hydroxymethyl-2,3-dihydro-1,4-benzodioxine.

A mixture of 6- and 7-nitro-2-hydroxymethyl-2,3-dihydro-1,4-benzodioxine (6.3 g, 30 mmol; from Example 146, Step 1), ammonium formate (2.8 g, 45 mmol), and 10% Pd/C (0.4 g) in MeOH (200 mL) was stirred at room temperature for 3 h. The reaction mixture was filtered through a pad of Celite. The pad was washed with several portions of MeOH and the filtrate was concentrated *in vacuo.* The mixture of 6- and 7-amino substituted-2,3-dihydro-1,4-benzodioxin derivatives was separated by chromatography on silica gel using EtOAc/isohexane (1:3 to 2:3) as eluent to give 2.8 g (58%) of the title product as an oil which was used directly in the next step.

### Step 2: 7-Acetamido-2-hydroxymethyl-2,3-dihydro-1,4-benzodioxine.

Acetic anhydride (0.24 g, 2.4 mmol) was added to a stirred mixture of the title compound from Step 1 above (0.30 g, 1.66 mmol) and Et₃N (0.70 g, 6.9 mmol) in CH₂Cl₂ (30 mL). The reaction was stirred at room temperature for 15 h. More acetic anhydride (0.10 g, 1.0 mmol) was added and the mixture was stirred for a further 5 h at room temperature. The mixture was concentrated and the residue purified by chromatography on silica gel using EtOAc/isohexane (1:3 to 2:3) as eluent to give 170 mg (46%) of the title product as an oil.

### Step 3: 2-(7-Acetamido-2,3-dihydro-1,4-benzodioxin-2-ylmethoxy)-3-(1-piperazinyl)pyrazine.

Oil; yield 45%. HRMS *m*/*z* calcd for C₁₉H₂₃N₅O₄ (M)⁺ 385.1750, found 385.1741.
*The starting material (*S*)-2-hydroxymethyl-2,3-dihydro-1,4-benzodioxine was prepared as described in *Tetrahedron Lett.* **1988,** *29*, 3671-3674.

### EXAMPLE 148

### 2-[(2S)-2,3-Dihydro-1,4-benzodioxin-2-ylmethoxy]-3-(1-piperazinyl)pyrazine, Hydrochloride.*

Mp 160 °C (dec). Anal. (C₁₇H₂₀N₄O₃ · HCl) C, H, N.
*The starting material (*R*)-2-hydroxymethyl-2,3-dihydro-1,4-benzodioxine was prepared as described in *Tetrahedron Lett.* **1988,** *29*, 3671-3674.

### EXAMPLE 149

### 2-[(2R)-2,3-Dihydro-1,4-benzodioxin-2-ylmethoxy]-3-(1-piperazinyl)pyrazine, Hydrochloride.*

Mp 165 °C (dec). Anal. (C₁₇H₂₀N₄O₃ · HCl) C, H, N.
*Previously described in EP 286242(A2).

### EXAMPLE 150

### 2-(3-Pyridyloxy)ethyl 3-(1-piperazinyl)-2-pyrazinyl ether, Hydrochloride.

### Step 1: 2-(3-Pyridyloxy)ethanol.*

A mixture of 3-hydroxypyridine (2.00 g, 21 mmol), 2-chloroethanol (1.69 g, 21 mmol) and K₂CO₃ (8.7 g, 63 mmol) in DMF (10 mL) was stirred at 130 °C for 3 h. After cooling, the black mixture was filtered through a short bed of alumina using acetone as eluent to afford a brown oil which was partitioned between water (30 mL) and CH₂Cl₂ (40 mL). The aqueous phase was extracted with CH₂Cl₂ (3 x 40 mL), and the combined organic extracts were dried over MgSO₄ and filtered. The filtrate was concentrated to give 0.55 g (19%) of the title compound as a light brown oil. Anal. (C₇H₉NO₂ · 0.2 H₂O) C, H, N.

### Step 2: 2-(3-Pyridyloxy)ethyl 3-(1-piperazinyl)-2-pyrazinyl ether, Hydrochloride.

Yield 41%; mp 170-175 °C. Anal. (C₁₅H₁₉N₅O₂ · 2 HCl · 2 H₂O) C, H, N.
*Previously described in *J*. *Chem. Soc., Perkin Trans 2*. **1987**, 1867-1870.

### EXAMPLE 151

### 2-(4-Pyridyloxy)ethyl 3-(1-piperazinyl)-2-pyrazinyl ether, Hydrochloride.

### Step 1: 2-(4-Pyridyloxy)ethanol.*

The title compound was prepared according to the procedure described in Example 150, Step 1. Yield 19%; mp 119-121 °C. Anal. (C₇H₉NO₂) C, H, N.

### Step 2: 2-(4-Pyridyloxy)ethyl 3-(1-piperazinyl)-2-pyrazinyl ether, Hydrochloride.

Yield 58%; mp 170 °C (dec). Anal. (C₁₅H₁₉N₅O₂ · 2 HCl · 2 H₂O) C, H, N.
*The free base of the title compound has been reported in *J. Org. Chem.* **1977,** 42, 1500-1508.

### EXAMPLE 152

### 2-(2-Pyridyloxy)ethyl 3-(1-piperazinyl)-2-pyrazinyl ether, Maleate.

### Step 1: 2-(2-Pyridyloxy)ethanol, Hydrochloride.*

A mixture of ethylene carbonate (2.78 g, 31.5 mmol), 2-hydroxypyridine (3.00 g, 31.5 mmol) and potassium carbonate (4.36 g, 31.5 mmol) in DMF (50 mL) was heated at 150 °C for I h. After cooling, the reaction mixture was concentrated *in vacuo.* The residue was dissolved in water (5 mL) and applied to a column with hydromatrix material. The column was eluted with EtOAc. The elute was concentrated and the remaining oil was purified by silica gel chromatography using isohexane/EtOAc (6:4) as eluent. This furnished the free base of the title compound as an oil which was converted to the hydrochloride salt: yield 1.00 g (18%); mp 97-98 °C. Anal. (C₇H₉NO₂ · HCl) C, H, N.

### Step 2: 2-(2-Pyridyloxy)ethyl 3-(1-piperazinyl)-2-pyrazinyl ether, Maleate.

Yield 27%; mp 130-132 °C. Anal. (C₁₅H₁₉N₅O₂ · C₄H₄O₄) C, H, N.

### EXAMPLE 153

### 2-(6-Methyl-3-pyridyloxy)ethyl 3-(1-piperazinyl)-2-pyrazinyl ether, Hydrochloride. Step 1: 2-(6-Methyl-3-pyridyloxy)ethanol.

The title compound was prepared according to the procedure described in Example 91, Step 1, starting from 3-hydroxy-6-methylpyridine. Yield 25%; mp 49-51 °C. Anal. (C₈H₁₁NO₂) C, H, N.

### Step 2: 2-(6-Methyl-3-pyridyloxy)ethyl 3-(1-piperazinyl)-2-pyrazinyl ether, Hydrochloride.

Yield 50%; mp 157°C (dec). Anal. (C₁₆H₂₁N₅O₂ · 2.3 HCl · 0.2 Et₂O) C, H, N.

### EXAMPLE 154

### 2-(2-Methyl-3-pyridyloxy)ethyl 3-(1-piperazinyl)-2-pyrazinyl ether, Hydrochloride. Step 1: 2-(2-Methyl-3-pyridyloxy)ethanol.

The title compound was prepared according to the procedure described in Example 150, Step 1, starting from 3-hydroxy-2-methylpyridine. Yield 36%; mp 75-80 °C. Anal. (C₈H₁₁NO₂) C, H, N.

### Step 2: 2-(2-Methyl-3-pyridyloxy)ethyl 3-(1-piperazinyl)-2-pyrazinyl ether, Hydrochloride.

Yield 36%; mp 50 °C (dec). Anal. (C₁₆H₂₁N₅O₂ · 3.1 HCl · 0.2 Et₂O) C, H, N.

### EXAMPLE 155

### 2-(5-Chloro-3-pyridyloxy)ethyl 3-(1-piperazinyl)-2-pyrazinyl ether, Hydrochloride, Step 1: 2-(5-Chloro-3-pyridyloxy)ethanol.

The title compound was prepared according to the procedure described in Example 150, Step 1, starting from 5-chloro-3-pyridinol. Yield 25%; mp 38-40 °C. HRMS *m*/*z* calcd for C₇H₈ClNO₂ (M)⁺ 173.0244, found 173.0244.

### Step 2: 2-(5-Chloro-3-pyridyloxy)ethyl 3-(1-piperazinyl)-2-pyrazinyl ether, Hydrochloride.

Yield 65%; mp 104 °C (dec). Anal. (C₁₅H₁₈ClN₅O₂ · 1.9 HCl · 0.3 Et₂O) C, H, N.

### EXAMPLE 156

### 2-[2-(1-Benzothien-3-yloxy)ethoxy]-3-(1-piperazinyl)pyrazine, Maleate.

### Step 1: 2-(1-Benzothien-3-yloxy)ethanol.

A mixture of 3-bromobenzothiophene (3.00 g, 14.1 mmol), KO-*t*-Bu (4.74 g, 42.2 mmol), copper (II) oxide (0.56 g, 7.0 mmol) and potassium iodide (2.34 g, 14.1 mmol) in ethylene glycol (10 mL) was stirred at 140 °C for 24 h. The reaction was quenched with water (100 mL) and filtered through a pad of Celite. The water phase was extracted with EtOAc (3 x 50 mL). The organic phase was treated with MgSO₄ and silica, filtered through Celite and concentrated. The remaining oil was purified by column chromatography on silica using isohexane/EtOAc (6:4) as eluent. The product crystallized on standing to give 1.63 g (60%) of the title product: mp 55-56°C. Anal. (C₁₀H₁₀O₂S) C, H.

### Step 2: 2-[2-(1-Benzothien-3-yloxy)ethoxy]-3-(1-piperazinyl)pyrazine, Maleate.

Solid; yield 0.23 g (32%); mp 147-149 °C. Anal. (C₁₈H₂₀N₄O₂S · C₄H₄O₄) C, H, N.
*The starting material 2-(3-thienyloxy)ethanol was prepared as described in *Synth. Met.* **1988**, *26*, 153-168.

### EXAMPLE 157

### 2-(3-Thienyloxy)ethyl 3-(1-piperazinyl)-2-pyrazinyl ether, Dihydrochloride.*

Yield 39%; mp 75-85 °C. Anal. (C₁₄H₁₈N₄O₂S · 2.1 HCl) C, H, N.

### EXAMPLE 158

### 2-(2,3-Dihydro-2,2-dimethyl-7-benzofuranyloxy)ethyl 3-(1-piperazinyl)-2-pyrazinyl ether, Maleate.

A mixture of 2,3-dihydro-2,2-dimethyl-7-benzofuranol (0.82 g, 5.0 mmol), ethylene oxide (0.22 g, 5.0 mmol), triethylamine (3 drops) and dioxane (4 mL) was heated at 100 °C in a sealed tube for 3 d. The solution was cooled in an ice-bath and KO-*t*-Bu (0.56 g, 5 mmol) followed by 2-chloro-3-(1-piperazinyl)pyrazine (0.79 g, 4.0 mmol; from Example 90, Step 1) were added. The mixture was stirred at room temperature for 30 min and at 100 °C for 4 h, allowed to cool, diluted with CH₂Cl₂ and filtered. The filtrate was concentrated and purified by chromatography on silica gel (gradient: PhMe to PhMe/MeOH/Et₃N, 8:1:1) to give 1.46 g of a beige viscous oil. Maleic acid (0.46 g, 4.0 mmol) and dry MeOH (10 mL) was added. The mixture was heated until a clear solution formed. Upon cooling, the maleate salt of the title compound crystallized as a pale beige solid: yield 1.40 g (72%); mp 156-157 °C. Anal. (C₂₀H₂₆N₄O₃ · C₄H₄O₄) C, H, N.

### EXAMPLE 159

### 2-(1,3-Benzoxazol-4-yloxy)ethyl 3-(1-piperazinyl)-2-pyrazinyl ether, Maleate.

### Step 1: 2-(1,3-Benzoxazol-4-yloxy)ethanol.

K₂CO₃ (254 mg, 1.84 mmol) was added to a stirred solution of 4-hydroxybenzoxazole* (248 mg, 1.84 mmol) in DMF (5 mL) at room temperature. The mixture was heated at 90 °C for 15 min and ethylene carbonate (176 mg, 2.00 mmol) was added. The mixture was stirred at 150 °C for 2 h and allowed to attain room temperature. The mixture was diluted with water (2 mL) and MeOH (2 mL), and concentrated. The residue was partitioned between water and CH₂Cl₂. The aqueous layer was separated and extracted with CH₂Cl₂ (2×10 mL). The organic phases were pooled and washed with water and brine, and dried over MgSO₄. The filtrate was concentrated to give the product as a brown oil that slowly solidified: yield 278 mg (84%); mp 47-49 °C. Anal. (C₉H₉NO₃) C, H, N.
*Prepared as described in *J. Med. Chem.* **1987,** *30*, 62-67.

### Step 2: 2-(1,3-Benzoxazol-4-yloxy)ethyl 3-(1-piperazinyl)-2-pyrazinyl ether, Maleate.

KO-*t*-Bu (146 mg, 1.30 mmol) was added in one portion to a stirred solution of 2-(1,3-benzoxazol-4-yloxy)ethanol (223 mg, 1.24 mmol) in dioxane (5 mL) at room temperature. After 15 min, 2-chloro-3-(1-piperazinyl)pyrazine (247 mg, 1.24 mmol; from Example 90, Step 1) was added in one portion followed by dioxane (3 mL). The mixture was stirred at room temperature for 10 min and at 100 °C for 3 h, allowed to cool, diluted with CH₂Cl₂ and filtered through Celite. The filtrate was concentrated and purified by chromatography on silica gel (gradient: PhMe to PhMe/MeOH/Et₃N, 8:1:1) to give the product (70 mg, 17%) as a beige viscous oil. The maleate salt was prepared as described in Example 158: mp 191-193 °C. Anal. (C₁₇H₁₉N₅O₃ · C₄H₄O₄) C, H, N.

### EXAMPLE 160

### 4-(2-{[3-(1-Piperazinyl)-2-pyrazinyl]oxy}ethoxy)-2-quinolinamine, Trihydrochloride.

### Step 1: 2-[(2-Amino-4-quinolinyl)oxy]-1-ethanol.

To a mixture of 2-amino-4-hydroxyquinoline (0.505 g, 3.15 mmol) and K₂CO₃ (0.87 g, 6.3 mmol) in DMF (10 mL) was added of 2-bromoethanol (0.470 g, 3.78 mmol). The mixture was stirred at 150 °C for 7 h. The reaction mixture was concentrated and the residue purified by column chromatography on silica using MeOH/CHCl₃ (1:9) as eluent. The resulting brown oil (0.30 g, 30%), which was used directly in the next step, contained 75% of the desired product and 25% of unreacted starting material. MS *m*/*z* 205 (M+H)⁺.

### Step 2: 4-(2-{[3-(1-Piperazinyl)-2-pyrazinyl]oxy}ethoxy)-2-quinolinamine, Trihydrochloride.

The product obtained in Step 1 (0.30 g, 1.5 mmol) was dissolved in DMF (4 mL) and treated with NaH (55% dispersion in mineral oil; 0.12 g, 3.0 mmol). After beeing stirred for 5 min at room temperature, 2-chloro-3-(1-piperazinyl)pyrazine (0.212 g, 1.07 mmol; from Example 90, Step 1) was added. The reaction mixture was stirred at 70 °C for 4 h and at room temp for 14 h. Purification by column chromatography on silica gel using 20% MeOH in CHCl₃ as eluent furnished 0.102 g (25%) of the free base of the title compound which was converted to a hydrochloric acid salt: mp 156 °C (dec). HRMS *m*/*z* calcd for C₁₉H₂₂N₆O₂ (M)⁺ 366.1804, found 366.1795.

### EXAMPLE 161

### 2-(3,4-Dihydro-2H-pyrido[3,2-b]-1,4-oxazin-2-ylmethoxy)-3-(1-piperazinyl)pyrazine, Maleate.

### Step 1: 3,4-Dihydro-3-oxo-2H-pyrido[3,2-b]-1,4-oxazin-2-carboxylic acid ethyl ester.

Diethyl chloromalonate (9.73 g, 50 mmol) was added to a stirred mixture of 3-hydroxy-2-aminopyridine (5.51 g, 50 mmol), triethylamine (6.97 mL, 50 mmol) and EtOH (100 mL) at room temperature. The mixture was heated at reflux for 17 h and allowed to attain room temperature. The precipitate formed was filtered off, washed with EtOH and dried to give the product as a white solid: yield 3.85 g (35%); mp 160-162 °C. Anal. (C₁₀H₁₀N₂O₄) C, H, N.

### Step 2: 3,4-Dihydro-2H-pyrido[3,2-b]-1,4-oxazin-2-ylmethanol.

A solution of 3,4-dihydro-3-oxo-2*H*-pyrido[3,2-*b*]-1,4-oxazin-2-carboxylic acid ethyl ester (1.10 g, 5.0 mmol) in THF (50 mL) was added over 5 min to a stirred mixture of LiAlH₄ (0.38 g, 10 mmol) and THF (20 mL) at 40 °C. The mixture was stirred at 45 °C for 30 min and at reflux for 4 h. The mixture was allowed to attain room temperature and excess LiAlH₄ decomposed with 50% aqueous NaOH. The mixture was filtered through Celite, and the filtrate was concentrated. The residue (0.42 g) was extracted with CH₂Cl₂ and the extract was concentrated and purified by chromatography on silica gel (gradient: PhMe to PhMe/MeOH/Et₃N, 8:1:1) to give the product as a white solid: yield 0.11 g (13%); mp 123-124 °C. Anal. (C₈H₁₀N₂O₂) C, H, N.

### Step 3: 2-(3,4-Dihydro-2H-pyrido[3,2-b]-1,4-oxazin-2-ylmethoxy)-3-(1-piperazinyl)pyrazine, Maleate.

The title compound was prepared in an analogous manner to Example 159, Step 2, starting from 3,4-dihydro-2*H*-pyrido[3,2-*b*]-1,4-oxazin-2-ylmethanol and was isolated as a brownish solid. Yield 47%; mp 154-158 °C. Anal. (C₁₆H₂₀N₆O₂ · 1.2 C₄H₄O₄) C, H, N.
*The corresponding hydrochloride salt has been reported in *J. Med. Chem.* **1981,** *24,* 93-101.

### EXAMPLE 162

### 2-[2-(3-Pyridinyloxy)ethoxy]-3-(1-piperazinyl)quinoxaline, Maleate.

### Step 1: 2-Chloro-3-(1-piperazinyl)quinoxaline, Maleate.*

A mixture of 2,3-dichloroquinoxaline (3.05 g, 15.3 mmol), piperazine (2.64 g, 30.6 mmol) and K₂CO₃ (2.12 g, 15.3 mmol) in acetonitrile (20 mL) was stirred at 55 °C for 1 h. The reaction mixture was diluted with CHCl₃, filtered, and concentrated to give a yellowish solid which was purified by chromatography on silica gel using CHCl₃/MeOH (9:1) as eluent. The resulting solid was redissolved in CHCl₃ and applied to a short (4 cm) plug of alumina. Elution with CHCl₃ afforded 3.08 g (81%) of the free base of the title compound, which was isolated as a light yellow solid. A portion of the free base was converted to the maleate and recrystallized from MeOH/ether: mp 165-166 °C; HRMS *m*/*z* calcd for C₁₂H₁₃ClN₄ (M)⁺ 248.0829, found 248.0830. Anal. (C₁₂H₁₃ClN₄ · C₄H₄O₄) C, H, N.

### Step 2: 2-[2-(3-Pyridinyloxy)ethoxy]-3-(1-piperazinyl)quinoxaline, Maleate.

KO-*t*-Bu (0.57 g, 5.1 mmol) was added to a stirred solution of 2-(3-pyridyloxy)ethanol (0.97 g, 7.0 mmol; from Example 150, Step 1) in dioxane (20 mL) and the mixture was stirred for 3 min at room temperature. The free base from Step 1 above (0.97 g, 3.89 mmol) was then added and the resulting mixture was stirred at 80 °C for 4 h. The reaction mixture was diluted with CH₂Cl₂, filtered, and concentrated to give a yellowish oil which was purified by chromatography on silica gel using CHCl₃/MeOH (9: 1) as eluent. The resulting oil was redissolved in CHCl₃ and applied to a short (4 cm) plug of alumina. Elution with CHCl₃ afforded 1.20 g (88%) of the free base of the title compound as a light beige oil. The free base was converted to the maleate salt and recrystallized from MeOH/ether: mp 137-139 °C; HRMS *m*/*z* calcd for C₁₉H₂₁N₅O₂ (M)⁺ 351.1695, found 351.1701. Anal. (C₁₉H₂₁N₅O₂ · 1.3C₄H₄O₄) C, H, N.

### EXAMPLE 163

### 2-[2-(3-Pyridinyloxy)ethoxy]-3-(1-piperazinyl)-6,7-difluoroquinoxaline, Maleate Step 1: 2-Chloro-3-(I-piperazinyl)-6,7-difluoroquinoxaline.

Piperazine (0.86 g, 10 mmol) was added to a stirred suspension of 6,7-difluoro-2,3-dichloroquinoxaline* (1.18 g, 5 mmol) in EtOH (50 mL) at room temperature. The mixture was heated at 75 °C for 17 h, allowed to cool and filtered to give the product as a pale yellow solid: yield 0.40 g (28%); mp 294-297 °C (dec). MS *m*/*z* 285/287 (M + H)⁺.
*Previously described in *J. Med. Chem.* **1990,** *33*, 2240-2254.

### Step 2: 2-[2-(3-Pyridinyloxy)ethoxy]-3-(1-piperazinyl)-6,7-difluoroquinoxaline, Maleate.

The title compound was prepared ) according to the procedure described in Example 162, Step 2, starting from 2-chloro-3-(1-piperazinyl)-6,7-difluoroquinoxaline (230 mg, 0.8 mmol) and 2-(3-pyridinyloxy)ethanol (140 mg, 1 mmol; from Example 150, Step 1). Yield 95 mg (31%). The maleate salt was obtained as described in Example 158: mp 110-5 °C. Anal. (C₁₉H₁₉F₂N₅O₂ · 1.5 C₄H₄O₄) C, H, N.

### EXAMPLE 164

### 2-[2-(3-Pyridinyloxy)ethoxy]-3-(1-piperazinyl)-6,7-dichloroquinoxaline, Maleate. Step 1: 3-(1-Piperazinyl)-2,6,7-trichloroquinoxaline.

Piperazine (0.69 g, 8.06 mmol) was added to a stirred suspension of 2,3,6,7-tetrachloroquinoxaline (1.08 g, 4.03 mmol) in EtOH (125 mL) at room temperature. The mixture was stirred at room temperature for 8 h and filtered to give the product as a pale yellow solid: yield 0.88 g (69%); mp >300 °C. MS *m*/*z* 316/318 (M)⁺.

### Step 2: 2-[2-(3-Pyridinyloxy)ethoxy]-3-(1-piperazinyl)-6,7-dichloroquinoxaline, Maleate.

The title compound was prepared according to the procedure described in Example 162, Step 2, starting from 3-(1-piperazinyl)-2,6,7-trichloroquinoxaline (270 mg, 1.00 mmol) and 2-(3-pyridinyloxy)ethanol (170 mg, 1.20 mmol; from Example 150, Step 1). Yield 135 mg (32%). The maleate salt was obtained as described in Example 158: mp 187-189 °C. Anal. (C₁₉H₁₉Cl₂N₅O₂ · C₄H₄O₄) C, H, N.

### EXAMPLE 165

### 2-[2-(3-Pyridinyloxy)ethoxy]-3-(1-piperazinyl)thieno[3,4-b]pyrazine, Maleate.

### Step 1: 2,3-Dichlorothieno[3,4-b]pyrazine.

1,4-Dihydro-thieno[3,4-*b*]pyrazine-2,3-dione* (1.26 g, 7.5 mmol) and POCl₃ (6.9 mL, 75 mmol) was stirred at reflux for 21 h. The dark mixture was poured onto ice and extracted with CH₂Cl₂ (4x30 mL). The organic extracts were pooled and concentrated to give 0.11 g (7%) of the title product which was used in the next step without purification.
*Previously described in *Bull. Soc. Chim. Fr.* **1983,** 159-163.

### Step 2: 2-Chloro-3-(1-piperazinyl)-thieno[3,4-b]pyrazine.

Piperazine (92 mg, 1.07 mmol) was added to a stirred suspension of 2,3-dichlorothieno[3,4-*b*]pyrazine (110 mg, 0.54 mmol) in EtOH (5 mL) at room temperature. The mixture was stirred at reflux for 16 h and filtered. The solid was purified by chromatography on silica gel (gradient: PhMe to PhMe/MeOH/Et₃N, 8:1:1) to give 40 mg (29%) of the product as an orange solid. The material was used without further purification in the next step.

### Step 3: 2-[2-(3-Pyridinyloxy)ethoxy]-3-(1-piperazinyl)thieno[3,4-b]pyrazine, Maleate.

The title compound was prepared according to the procedure described in Example 162, Step 2, starting from 2-chloro-3-(1-piperazinyl)-thieno[3,4-*b*]pyrazine (40 mg, 0.16 mmol; from Step 2 above) and 2-(3-pyridinyloxy)ethanol (44 mg, 0.31 mmol; from Example 150, Step 1. Yield 38 mg (66%). The maleate was obtained as described in Example 158: mp 152-156 °C. Anal. (C₁₇H₁₉N₅O₂S · 1.8 C₄H₄O₄) C, H, N.

### EXAMPLE 166

### 2-[2-(5-Pyrimidinyloxy)ethoxy]-3-(1-piperazinyl)quinoxaline.

### Step 1: 2-(5-Pyrimidinyloxy)ethanol.

KO-*t*-Bu (7.70 g, 68.6 mmol) was added to ethylene glycol (10.45 g, 168.4 mmol) under stirring. The mixture was gently heated in order to consume all KO-*t*-Bu. 5-Bromopyrimidine (8.45 g, 53.1 mmol) was added to the light brownish mixture. After 8 h of stirring at 130 °C, CH₂Cl₂ (50 mL) was added to the brownish reaction mixture and two layers were formed. The CH₂Cl₂ layer was saved and the other layer was extracted with additional portions of CH₂Cl₂. The combined CH₂Cl₂ layers were concentrated *in vacuo* and the oily residue purified by column chromatography on silica gel using CHCl₃/MeOH (9:1) as eluent to give 0.45 g (6%) of the title product as an oil sufficiently pure for the next step. HRMS *m*/*z* calcd for C₆H₈N₂O₂ (M)⁺ 140.0586, found 140.0589.

### Step 2: 2-[2-(5-Pyrimidinyloxy)ethoxy]-3-(1-piperazinyl)quinoxaline.

The title compound was prepared according to the procedure described in Example 162, Step 2, starting from 2-chlom-3-(1-piperazinyl)quinoxaline (0.66 g, 2.65 mmol; from Example 162, Step 1) and 2-(5-pyrimidinyloxy)ethanol (0.45 g, 3.20 mmol). Yield 0.55 g (59%); mp 115-117 °C; HRMS *m*/*z* calcd for C₁₈H₂₀N₆O₂ (M)⁺ 352.1648, found 352.1654. Anal. (C₁₈H₂₀N₆O₂) C, H, N.

### EXAMPLE 167

### 2-(3,4-Dihydro-2H-1,4-benzoxazin-2-ylmethoxy)-3-(1-piperazinyl)quinoxaline, 0.5 Fumarate.

The title product was prepared according to the procedure described in Example 162, Step 2, starting from 2-chloro-3-(1-piperazinyl)quinoxaline (described in Example 162, Step 1) and 3,4-dihydro-2*H*-1,4-benzoxazin-2-ylmethanol.* Yield 41%; mp 125-134 °C (dec). Anal. (C₂₁H₂₃N₄O₄ · 0.5C₄H₄O₄ · 2H₂O) C, H, N.
*See Example 130.

### EXAMPLE 168

### 2-(3,4-Dihydro-2H-chromen-2-ylmethoxy)-3-(1-piperazinyl)quinoxaline, Hydrochloride.

The title product was prepared according to the procedure described in Example 162, Step 2, starting from 2-chloro-3-(1-piperazinyl)quinoxaline (described in Example 162, Step 1) and 3,4-dihydro-2H chromen-2-ylmethanol.* The product was obtained as a yellow amorphous substance: yield 23%; mp 202-204 °C; HRMS *m*/*z* calcd for C₂₂H₂₄N₄O₂ (M)⁺ 376.1899, found 376.1899. Anal. (C₂₂H₂₄N₄O₂ HCl 0.5 H₂O) C, H; N: calcd, 13.3; found 13.9.
*See Example 133.

### EXAMPLE 169

### 2,3-Dihydro-1,4-benzodioxin-2-ylmethyl 3-(4-methyl-1-piperazinyl)-2-pyrazinyl ether, Dihydrochloride.

### Step 1: 1-(3-Chloro-2-pyrazinyl)-4-methylpiperazine.

A mixture of 2,3-dichloropyrazine (10.1 g, 68 mmol) and N-methylpiperazine (10.15 g, 100 mmol) in acetonitrile (250 mL) was stirred at room temperature for 60 h. The solvent was removed under reduced pressure and the residue was taken up in CHCl₃/brine. The dried (MgSO₄ organic layer was concentrated and the remaining oil was purified by chromatography on silica gel using CHCl₃/MeOH (95:5) as eluent to give 10.7 g (75%) of the title compound as an oil that crystallized upon standing: mp 34-36 °C. Anal. (C₉H₁₃ClN₄) C, H, N.

### Step 2: 2,3-Dihydro-1,4-benzodioxin-2-ylmethyl 3-(4-methy)-1-piperazinyl)-2-pyrazinyl ether, Dihydrochloride.

The title compound was prepared according to the procedure described in Example 90, Step 2, starting from 1-(3-chloro-2-pyrazinyl)-4-methylpiperazine (described in Example 169, Step 1) and 2-hydroxymethyl-2,3-dihydro-1,4-benzodioxine. Yield 87%; mp 160-167 °C. Anal. (C₁₈H₂₂N₄O₃ · 2 HCl) H, N; C: calcd, 52.06; found, 52.6.

### EXAMPLE 170

### 2-(Phenoxy)ethyl-3-(4-methyl-1-piperazinyl)-2-pyrazinyl ether, Hydrochloride.

The title compound was prepared according to the procedure described in Example 90, Step 2, starting from 1-(3-chloro-2-pyrazinyl)-4-methylpiperazine (described in Example 169, Step 1) and 2-phenoxyethanol. Yield 79%; mp 172-174 °C. Anal. (C₁₇H₂₂N₄O₂ · HCl) C, H, N.

### EXAMPLE 171

### 2-(2-Phenoxy)ethyl 3-(2-methyl-1-piperazinyl)-2-pyrazinyl ether, Maleate.

### Step 1: 3-Chloro-2-(4-benzyl-2-methyl-1-piperazinyl)pyrazine.

A mixture of 2,3-dichloropyrazine (0.332 g, 2.23 mmol), 1-benzyl-3-methylpiperazine* (0.423 g, 2.23 mmol), and K₂CO₃ (0.339 g, 2.45 mmol) in acetonitrile (2.5 mL) was stirred at 115 °C for 17 h in a sealed tube. The reaction mixture was diluted with ether, filtered, and concentrated. The brownish oily residue was purified by chromatography on silica gel using *n*-hexane/EtOAc (7:3) as eluent to give 0.19 g (28%) of the title product as a viscous oil that was sufficiently pure for the next step.
*Previously described in *J*. *Med*. *Chem.* **1996**, *39*, 2962-2970.

### Step 2: 2-(1-Phenoxy)ethyl 3-(4-benzyl-2-methyl-1-piperazinyl)-2-pyrazinyl ether.

KO-*t*-Bu (0.087 g, 0.77 mmol) was added in one portion to a stirred solution of 2-phenoxyethanol (0.164 g, 1.19 mmol) in dioxane (5 mL) at room temperature. After being stirred for 5 min at 90 °C, a solution of the product obtained in Step 1 above (0.18 g, 0.59 mmol) in dioxane (3 mL) was added. The mixture was stirred at room temperature for 10 min and at 85 °C for 2 h. The reaction mixture was diluted with ether, filtered, and concentrated. The remaining beige oil was purified by chromatography on silica gel using n-hexane/EtOAc (7:3) as eluent to give 0.22 g (93%) of the title product as an oil. HRMS *m*/*z* calcd for C₂₄H₂₈N₄O₂ (M)⁺ 404.2212, found 404.2232.

### Step 3: 2-(2-Phenoxy)ethyl 3-(2-methyl-1-piperazinyl)-2-pyrazinyl ether, Maleate.

Ammonium formate (0.18 g, 2.85 mmmol) was added to a solution of the product from Step 2 above (0.22 g, 0.54 mmol) in ethanol (5 mL). The mixture was purged with nitrogen and 5% Pd/C (0.15 g) was added. The flask was sealed and the mixture was stirred at 90 °C for 2.5 h. The reaction mixture was filtered through Celite and concentrated. The residue was purified by chromatography on silica gel using CHCl₃/MeOH (10:1) to give 0.12 g (72%) of the free base of the title compound as an oil. The free base was converted to the maleate and recrystallized from MeOH/ether: mp 102-103 °C; HRMS *mlz* calcd for C₁₇H₂₂N₄O₂ (M)⁺ 314.1743, found 314.1754. Anal. (C₁₇H₂₂N₄O₂ · C₄H₄O₄) C, H, N.

### EXAMPLE 172

### (2R)-Methyl-1-[3-(2-phenoxyethoxy)-2-pyrazinyl]piperazine, Hydrochloride.

### Step 1: 4-tert-Butoxycarbonyl-(2R)-methylpiperazine.

Glacial acetic acid (6.3 g, 105 mmol), followed by di-*tert*-butyldicarbonate (23.11g, 106 mmol), were added to a stirred solution of (2R)-methylpiperazine (10.3 g, 103 mmol) in MeOH (200 mL) at 0 °C, and the resulting mixture was stirred for 1 h. The reaction mixture was allowed to warm to room temperature and stirred for further 15 h. An excess of triethylamine (20 mL, 140 mmol) was added and the reaction mixture was concentrated under reduced pressure. The residue was suspended in CHCl₃ and filtered through a fritted glass filter. The filtrate was concentrated, and the residue was purified by chromatography on silica gel using CHCl₃/MeOH (85:15) as eluent to give 21 g (65%) of the title product as an oil that crystallized upon standing. The product was used in the next step without further characterization.

### Step 2: 3-Chloro-2-[4-tert-butoxycarbonyl-(2R)-methyl-1-piperazinyl]pyrazine.

A mixture of 2,3-dichloropyrazine (22.0 g, 148 mmol), 4-*tert*-butoxycarbonyl-(2*R*)-methylpiperazine (21 g, 105 mmol) and K₂CO₃ (30.4 g, 220 mmol) in DMF was heated at 95 °C for 15 h. The black reaction mixture was filtered through a bed of silica gel, and the filtrate was concentrated. The residue was purified by chromatography on silica gel using petroleum ether/EtOAc (9:1) as eluent to afford 3.2 g (10% over two steps) of the title compund as a colourless oil. HRMS *m*/*z* calcd for C₁₄H₂₁ClN₄O₂ (M)⁺ 312.1353, found 312.1358.

### Step 3: (2R)-Methyl-1-[3-(2-phenoxyethoxy)-2-pyrazinyl]piperazine, Hydrochloride.

KO-*t*-Bu (0.52 g, 4.66 mmol) was added to a solution of 2-phenoxyethanol (0.42 g, 3.0 mmol) in dioxane (15 mL) and the mixture was stirred at room temperature for 15 minutes. 4-*tert*-Butoxycarbonyl-(2*R*)-methylpiperazine (0.73 g, 2.33 mmol; from Step 1 above) was added to the suspension and the reaction was stirred at 85 °C for 15 h. The reaction mixture was filtered through Celite and the filtrate was concentrated under reduced pressure. The crude product was purified by chromatography on silica gel using CHCl₃/MeOH/NH₄OH (97:3:0.2) as eluent. The resulting oil was dissolved in CH₂Cl₂/TFA (1:1) and the mixture was stirred at room temperature for 15 h. The mixture was concentrated under reduced pressure and the remaining oil was partitioned between 1 M NaOH/CHCl₃.
The organic phase was dried (MgSO₄) and the solvent evaporated. The resulting oil was purified by chromatography on silica gel using CHCl₃/MeOH/NH₄OH (95:5:0.2) as eluent to provide the free base of the title compound. The free base was precipitated as its hydrochloride salt with HCl/ether to give 0.36 g (44%) of the title compound as white crystals: mp 164-166 °C; HRMS *m*/*z* calcd for C₁₇H₂₂N₄O₂ (M)⁺ 314.1743, found 314.1731. Anal. (C₁₇H₂₂N₄O₂ · HCl) C, H, N.

Examples 173-177 were prepared according to the procedure of Example 172, step 3.

### EXAMPLE 173

### (2R)-Methyl-1-{3-[2-(3-pyridinyloxy)ethoxy]-2-pyrazinyl}piperazine, Hydrochloride.

The title compound was prepared starting from 2-(3-pyridyloxy)ethanol (from Example 150, Step 1) and 3-chloro-2-[4-*tert*-butoxycarbonyl-(2*R*)-methyl-1-piperazinyl]pyrazine (from Example 172, Step 2). The pure free base was precipitated as its hydrochloride salt with HCl/ether to give the title compound as white crystals: yield 31%; mp 180-183 °C; HRMS *m*/*z* calcd for C₁₆H₂₁N₅O₂ (M)⁺ 315.1695, found 315.1689. Anal. (C₁₆H₂₁N₅O₂ · 1.33HCl) C, H, N.

### EXAMPLE 174

### (2S)-Methyl-1-{3-[2-(3-pyridinyloxy)ethoxy]-2-pyrazinyl}piperazine, Hydrochloride.

The title compound was prepared starting from 2-(3-pyridyloxy)ethanol (from Example 150, Step 1) and 3-chloro-2-[4-*tert*-butoxycarbonyl-(2*S*)-methyl-1-piperazinyl]pyrazine (prepared according to the procedure of Example 172, Step 2, with the exception that (2*S*)-methylpiperazine was substituted for (2R)-methylpiperazine). MS *m*/*z* 316 (M+H)⁺. Yield 58%; mp 170-172 °C. Anal. (C₁₆H₂₁N₅O₂ 1.45 HCl) C, H, N.

### EXAMPLE 175

### (2S)-Methyl-1-[3-(2-phenoxyethoxy)-2-pyrazinyl]piperazine, Hydrochloride.

The title compound was prepared starting from 2-phenoxy-1-ethanol and 3-chloro-2-[4-*tert*-butoxycarbonyl-(2*S*)-methyl-1-piperazinyl]pyrazine (prepared according to the procedure of Example 172, Step 2, with the exception that (2*S*)-methylpiperazine was substituted for (2*R*)-methylpiperazine). MS *m*/*z* 315 (M+H)⁺. Yield 68%; mp 162-163 °C. Anal. (C₁₇H₂₂N₄O₂ · HCl) C, H, N.

### EXAMPLE 176

### (2R)-2-Methyl-1-(3-{2-[(6-methyl-3-pyridinyl)oxy]ethoxy}-2-pyrazinyl)piperazine, Fumarate.

The title product was prepared from 2-(6-methyl-3-pyridyloxy)ethanol (from Example 153, Step 1) and 3-chloro-2-[4-*tert*-butoxycarbonyl-(2*R*)-methyl-1-piperazinyl]pyrazine (from Example 172, Step 2). The crude product was purified by column chromatography on silica using CHCl₃/MeOH/NH₄OH (95:5:0.2) as eluent. After evaporation of the solvent from the combined pure fractions, the resulting free base was precipitated as its fumaric acid salt. Recrystallization from MeOH/ether gave 46% of the title product as an amorphous solid: mp 115°C (dec). Anal. (C₁₇H₂₃N₅O₂ · C4H404) C, H, N.

### EXAMPLE 177

### (2R)-Methyl-1-{3-[2-(2-amino-8-quinolinyloxy)ethoxy]-2-pyrazinyl}piperazine, Fumarate.

### Step 1: 2-(2-Amino-8-quinolinyloxy)-ethanol.

A mixture of 2-amino-8-quinolinol (3.20 g, 20.0 mmol), ethylene carbonate (1.76 g, 20.0 mmol) and KO-*t*-Bu (2.24 g, 20.0 mmol) in DMF (20 mL) was stirred at 90 °C for 15 h. The mixture was poured into brine, extracted with EtOAc, dried (MgSO₄), and concentrated under reduced pressure. The residue was purified by chromatography on silica gel using toluene/EtOAc/MeOH (50:50:2) as eluent to give 1.13 g (28%) of the title product as a solid. HRMS *m*/*z* calcd for C₁₁H₁₂N₂O₂ (M)⁺ 204.0899. found 204.0906.

### Step 2: (2R)-Methyl-1-{3-[2-(2-amino-8-quinolinyloxy)ethoxy])-2-pyrazinyl}piperazine, Fumarate.

The title compound was prepared starting from 2-(2-amino-8-quinolinyloxy)ethanol and 3-chloro-2-[4-*tert*-butoxycarbonyl-(2*R*)-methyl-1-piperazinyl]pyrazine (from Example 172, Step 2). The pure free base was precipitated as its fumaric acid salt to give the title compound as yellow, slightly hygroscopic, crystals: yield 5%; mp 160-163 °C (dec). HRMS *m*/*z* calcd for C₂₀H₂₄N₆O₂ (M)⁺ 380.1961, found 380.1958.

### EXAMPLE 178

### 2-Ethyl-1-{3-[2-(3-pyridinyloxy)ethoxy]-2-pyrazinyl}piperazine, Fumarate.

### Step 1: 1-Benzyl-3-ethylpiperazine.

Benzyl bromide (38.7 g, 0.22 mol) was added in portions to an ice cold (~ 0 °C) solution of 2-ethylpiperazine (25 g, 0.22 mol) in DMF (150 mL) with such a rate that the temperature did not exceed 20 °C. The mixture was stirred for 1 h, the solvent was evaporated and the residue was partitioned between CHCl₃/0.5 M HCl. The aqueous phase was made alkaline (11M NaOH) and extracted three times with CHCl₃. The combined organic phases were dried (MgSO₄) and concentrated. The resulting oil was purified by column chromatography on silica using CHCl₃, followed by CHCl₃/MeOH/NH₄OH (95:5:0.3) as eluents to give 31.6 g (70%) of the title compound as a yellowish oil. Anal. (C₁₃H₂₀N₂) H, N; C: calc, 76.42 found 75.85; H, N.

### Step 2: 4-Benzyl-1-(3-chloro-2-pyrazinyl)-2-ethylpiperazine.

A mixture of 2,3-dichloropyrazine (3.9 g, 27 mmol), 1-benzyl-3-ethylpiperazine (4.6 g, 22.5 mmol; from Step 1) and K₂CO₃ (6.2 g, 45 mmol) in DMF (10 mL) was stirred at 100 °C for 4 days. The solvent from the filtered reaction mixture was evaporated off and the residue was purified by column chromatography on silica using heptane/EtOAc (90:10) as eluent to yield 5.0 g (71%) of the title compound as a light yellow oil. HRMS *m*/*z* calcd for C₁₇H₂₁ClN₄ (M)⁺ 316.1455, found 316.1448.

### Step 3: 1-(3-Chloro-2-pyrazinyl)-2-ethylpiperazine, Hydrochloride.

1-Chloroethyl chloroformate (2.11 g, 15.2 mmol) was added with a syringe during I h to an ice cold (- 0 °C) solution of 4-benzyl-1-(3-chloro-2-pyrazinyl)-2-ethylpiperazine (3.22 g, 10.1 mmol, from Step 2) in dry CH₂Cl₂ (30 mL). The reaction was allowed to slowly reach room temperature and stirred for 48 h. The solvent was evaporated from the reaction mixture and MeOH was added to the resulting oil. The mixture was heated to reflux for 2 h, the solvent was evaporated and the resulting oil was partitioned between CHCl₃/H₂O. The aqueous phase was made alkaline and extracted with CHCl₃ (x 3). The combined organic phases were dried (MgSO₄), and the solvent was evaporated. The resulting oil was purified by column chromatography on silica using CHCl₃/MeOH/NH₄OH (95:5:0.2) as eluent to give a quantitative yield (2.35 g) of the free base of the title compound as a slightly yellow oil. An analytical sample was prepared as its hydrochloric acid salt (from HCl/ether): mp 195-197 °C. Anal. (C₁₀H₁₅CIN₄ · HCl) C, H, N.

### Step 4. 2-Ethyl-1-{3-[2-(3-pyridinyloxy)ethoxy]-2-pyrazinyl}piperazine, Fumarate.

The title compound was prepared starting from 2-(3-pyridyloxy)ethanol (from Example 150, Step 1) and the product obtained in Step 3 according to the procedure described in Example 162, Step 2. The crude product was purified by column chromatography on silica using CHCl₃/MeOH/NH₄OH (90:10:0.3) as eluent. After evaporation of the solvent from the combined pure combined fractions, the resulting free base was converted to its fumarate salt and crystallized from MeOH to afford 0.21 g (8%) of the title compound as light yellow crystals: mp 125-127 °C; HRMS *m*/*z* calcd for C₁₇H₂₃N₅O₂ (M)⁺ 329.1852, found 329.1845. Anal. (C₁₇H₂₃N₅O₂ · C₄H₄O₄) C, H, N.
*Previously descibed in *J. Med. Chem.* **1964,** *7*, 241-242 and *Org. Prep. Proc. Int.* **1976,** *8*, 19-23.

### EXAMPLE 179

### cis-2,6-Dimethyl-1-{3-[2-(3-pyridinyloxy)ethoxy]-2-pyrazinyl}piperazine, Fumarate.

### Step: 1-Benzyl-cis-3,5-dimethylpiperazine.*

To an ice cold (∼ 0 °C) suspension of cis-2,6-dimethylpiperazine (4.0 g, 35 mmol) in DMF (100 mL) was benzyl bromide (6.0 g, 35.0 mmol) added in portions under a period of 45 min and the reaction mixture was stirred at room temperature for 48 h. The solvent was evaporated from the suspension and the residue was dissolved in aqueous HCl and washed twice with CHCl₃. The aqueous phase was made alkaline and extracted twice with CHCl₃. The combined organic phases were washed with H₂O and brine, dried (MgSO₄), and concentrated to give 4.9 g (68%) of the title product as a colorless oil.

### Step 2: 4-Benzyl-1-(3-chloro-2-pyrazinyl)-cis-2,6-dimethylpiperazine.

A mixture of 2,3-dichloropyrazine (4.9 g, 32.9 mmol), 1-benzyl-*cis*-3,5-dimethylpiperazine (5.9 g, 29.0 mmol), tributylamine (5.9 g, 32 mmol) and diphenyl ether (70 mL) was heated in a sealed tube at 220 °C for 3 days. The reaction mixture was cooled to ambient temperature and EtOAc (200 mL) and toluene (100 mL) were added. The organic phase was extracted with 5 M aqueous HCl (x 3) and the combined aqueous phases were extracted with CHCl₃ (x 3). The combined organic layers, which contained the product, were washed with aqueous NaOH (5 M), dried (MgSO₄), and the solvent was evaporated. The crude product (0.96 g), about 80% pure based on GC, was used in the next step without further purification.

### Step 3. 1-(3-Chloro-2-pyrazinyl)-cis-2,6-dimethylpiperazine, Hydrochloride.

The title compound was prepared by treating the product obtained in Step 2 with 1-chloroethyl chloroformate according to the procedure described in Example 178, Step 3. The crude product was purified by column chromatography on silica using CHCl₃/MeOH/NH₄OH (95:5:0.2, followed by 90:10:0.5) as eluent to give 0.50 g (8% over two steps) of the free base of the title compound as a colorless oil. An analytical sample was precipitated as its HCl salt with HCl/ether: mp 199-200 °C. Anal. (C₁₀H₁₅ClN₄ · HCl) C, H, N.

### Step 4: cis-2,6-Dimethyl-1-{3-[2-(3-pyridinyloxy)ethoxy]-2-pyrazinyl}piperazine, Fumarate.

The title compound was prepared starting from 2-(3-pyridyloxy)ethanol (from Example 150, Step 1) and the product obtained in Step 3 according to the procedure of Example 162, Step 2. The crude product was purified by column chromatography on silica using CHCl₃/MeOH/NH₄OH (95:5:0.2) as eluent. After evaporation of the solvent from the combined pure fractions, the resulting oil was crystallized as its fumaric acid salt from MeOH/ether to afford 0.29 g (36%) of the title compound as white crystals: mp 157 °C (dec). Anal. (C₁₇H₂₃N₅O₂ · C₄H₄O₄) C, H, N.

### EXAMPLE 180

### N-[8-(2-{[3-(1-Piperazinyl)-2-pyrazinyl]oxy}ethoxy)-2-quinolinyl]acetamide.

### Step 1: tert-Butyl 4-(3-{2-[(2-amino-8-quinolinyl)oxylethoxy}-2-pyrazinyl)-1-piperazinecarboxylate.

The product of Example 177, Step 1 (503 mg, 2.46 mmol) and 2-chloro-3-(4-*tert*-butoxycarbonyl-1-piperazinyl)pyrazine (659 mg, 2.21 mmol, from Example 52, Step 1) were dissolved in dry dioxane (20 mL). KOt-Bu (305 mg, 2.72 mmol) was added and the reaction mixture was heated. The reaction mixture was heated at 80 °C under inert atmosphere for 3.5 h (monitoring by MS). The reaction mixture was concentrated *in vacuo* and the residue was dissolved in EtOAc. The organic phase was washed with H₂O and the aqueous phase was back-extracted with CH₂Cl₂. The combined organic phases were washed with brine and dried (MgSO₄). Evaporation of the solvents gave a yellow oil (1.37 g), that was purified by column chromatography using EtOAc/Et₃N (95:5) as eluent to give the title compound as a yellow solid: yield 898 mg (87 %); MS *m*/*z* 467 (M⁺H)⁺. Anal. (C₂₄H₃₀N₆O₄ 0.9 H₂O) C, H, N.

### Step 2: tert-Butyl 4-[3-(2-{[2-(acetylamino)-8-quinolinyl]oxy}ethoxy)-2-pyrazinyl]-1-piperazinecarboxylate.

The product obtained in Step 1 (502 mg, 1.08 mmol) was dissolved in pyridine. Ac₂O (0.42 mL, 4.45 mmol) was added, and the reaction mixture was stirred under inert atmosphere at room temperature for 5 h. Concentration *in vacuo* afforded a yellow oil, which was purified by column chromatography using EtOAc/Et₃N (95:5) as eluent to give the title product as a yellow solid: yield 531 mg (97 %); MS *m*/*z* 509 (M+H)⁺.
Anal. (C₂₆H₃₂N₆O₅ · 0.4 H₂O) C, H, N.

### Step 3: N-[8-(2-{[3-(1-Piperazinyl)-2-pyrazinyl]oxy}ethoxy)-2-quinolinyl]acetamide.

TFA (1.5 mL) was added to a solution of the product from Step 2 (112 mg, 0.22 mmol) in CH₂Cl₂ (1.5 mL) at 0 °C and the mixture was stirred under inert atmosphere for 1 h.
The reaction mixture was diluted with CH₂Cl₂ and washed with 2 M aqueous NaOH (x 4) and brine. The organic layer was dried (MgSO₄) and concentrated. The crude solid product (83 mg) was purified by column chromatography on silica using CHCl₃/MeOH (9:1) + 1 % NH₃ as eluent to give the title compund as an oil that solidified after repeated additions of pentane: yield 67 mg (75 %); MS *m*/*z* 409 (M+H)⁺. Anal. (C₂₁H₂₄N₆O₃ · 0.6 H₂O · 0.1 C₅H₁₂) C, H, N.

### EXAMPLE 181

### N-Methyl-8-(2-{[3-(1-piperazinyl)-2-pyrazinyl]oxy}ethoxy)-2-quinolinamine.

### Step 1: 2-(Methylamino)-8-quinolinol.

To a solution of 2-chloro-8-hydroxyquinoline* (2.0 g, 11.1 mmol) in EtOH (40 mL) was added H₂NMe (40 % aqueous solution, 40 mL, 464 mmol). The mixture was heated in a sealed Pyrex tube at 100 °C for 24 h. The solvent was evaporated and the resulting crude product was purified by column chromatography [gradient: CH₂Cl₂/MeOH (99:1) to 1% NH₄OH in CH₂Cl₂/MeOH (80:20)] to give the title compound as a black-green solid. Yield 1.31 g (67 %); MS *m*/*z* 174 (M)⁺. Anal. (C₁₀H₁₀N₂O) C, H, N.
*Prepared as described in *J. Org. Chem.* **1971,** *36,* 3490-3493.

### Step 2: 2-{[2-(Methylamino)-8-quinolinyl]oxy}-1-ethanol.

The title compound was prepared according to the procedure of Example 91, Step 1, starting from the product obtained in Step 1 above. The crude product was purified by column chromatography on silica gel using EtOAc/MeOH (95:5) containing Et₃N (2%). Solid; yield 1.21 g (88 %); MS *m*/*z* 218 (M)⁺. Anal. (C₁₂H₁₄N₂O₂ · 0.6 H₂O) C, H, N.

### Step 3: N-Methyl-8-(2-{[3-(1-piperazinyl)-2-pyrazinyl]oxy}ethoxy)-2-quinolinamine.

The title compound was prepared according to the procedure of Example 90, Step 2, starting from the product obtained in Step 2 above and 2-chloro-3-(1-piperazinyl)pyrazine (from Example 90, Step 1). The crude product was purified by column chromatography (gradient: 1% NH₄OH in EtOAc/MeOH (90:10) to 1% NH₄OH in EtOAc/MeOH (80:20) in first purification step and gradient 0.5% NH₄OH in CHCl₃/MeOH (95:5) to 1% NH₄OH in CHCl₃/MeOH (90:10) in second purification). Yield 31%; mp 54.0-56.5 °C; MS *m*/*z* 381 (M+H)⁺. Anal. (C₂₀H₂₄N₆O₂ · 0.5 H₂O) C, H, N.
*Previously described in *J*. *Med*. *Chem.* **1993***, 36*, 690-698.

### EXAMPLE 182

### 2-[2-(1,3-Benzodioxol-4-yloxy)ethoxy]-3-(2-met6yl-1-piperazinyl)quinoxaline, Hydrochloride.

### Step 1: 4-tert-Butoxycarbonyl-2-methylpiperazine.*

The title compound was prepared according to the procedure described in Example 172, Step 1, except that racemic 2-methylpiperazine was used instead of (2*R*)-methylpiperazine. Yield 82% of the free base. An analytical sample was prepared by conversion to the hydrochloride salt: mp 162 °C (dec). Anal. (C₁₀H₂₀N₂O₂ · HCl) C, H, N.

### Step 2: tert-Butyl 4-(3-chloro-2-quinoxalinyl)-3-methyl-1-piperazinecarboxylate.

A mixture of the title product from Step 1 above (12.3 g, 61.4 mmol), 2,3-dichloroquinoxaline (14.4 g, 72.2 mmol), and K₂CO₃ (13.8 g, 100 mmol) in DMF (100 mL) was stirred at 85 °C for 15 h. The mixture was filtered and the resulting filtrate was concentrated under reduced pressure. The residue was purified by chromatography on silica gel using petroleum ether/EtOAc (94:6) as eluent to give 5.8 g (36%) of a reddish oil that crystallized on standing: mp 93-97 °C. Anal. (C₁₈H₂₃ClN₄O₂) C, H, N.

### Step 3: 2-(1,3-Benzodioxol-4-yloxy)ethanol.

A mixture of 1,3-benzodioxol-4-ol* (0.74 g, 5.36 mmol), ethylene carbonate (0.47 g, 5.3 mmol) and K₂CO₃ (0.67 g, 4.8 mmol) in DMF (30 mL) was heated at 150 °C. After the evolution of carbon dioxide had ceased (1 h), the reaction mixture was filtered and concentrated. The crude product was purified by chromatography on silica gel using petroleum ether/EtOAc (70:30) as eluent to give 0.64 g (65%) of the title product as an oil which spontaneously crystallized to a white solid: mp 56-57 °C. Anal. (C₉H₁₀O₄) C, H.
*See Example 78.

### Step 4: 2-[2-(1,3-Benzodioxol-4-yloxy)ethoxy]-3-(2-methyl-1-piperazinyl)quinoxaline, Hydrochloride.

KO-*t*-Bu (0.41 g, 3.7 mmol) was added to a solution of 2-(1,3-benzodioxol-4-yloxy)ethanol (0.33 g, 1.8 mmol; from Step 3 above) in dioxane (15 mL). After 15 min of stirring at room temperature, the title compound from Step 2 above (0.73 g, 2.0 mmol) was added and the mixture was stirred at 95 °C for 1.5 h. The reaction mixture was filtered through a pad of Celite, concentrated *in vacuo,* and the residue was purified by chromatography on silica gel using petroleum ether/EtOAc (9:1) as eluent. The resulting oil was dissolved in CH₂Cl₂/TFA (1:1,20 mL) and stirred at room temperature for 15 h. The mixture was concentrated and the resulting oil was co-evaporated twice with 2 M HCl. The residue was recrystallized from water to give 0.22 g (26%) of the title compound as white crystals: mp 133-138 °C; HRMS *m*/*z* calcd for C₂₂H₂₄N₄O₄ (M)⁺ 408.1798, found 408.1782. Anal. (C₂₂H₂₄N₄O₄ · HCl · H₂O) C, H, N.

### EXAMPLE 183

### 5,6-Dimethyl-2-(2-phenoxyethoxy)-3-(1-piperazinyl)pyrazine, Dihydrochloride.

### Step 1. 2-Chloro-5,6-dimethyl-3-(1-piperazinyl)pyrazine.

The title compound was prepared according to the procedure described in Example 90, Step 1, starting from 2,3-dichloro-5,6-dimethylpyrazine* (0.60 g, 3.39 mmol) and piperazine (0.88 g, 10.2 mmol). Yield 0.51 g (66%). MS *m*/*z* 226 (M)⁺.

*Prepared as described in *J*. *Am*. *Chem*. *Soc*. **1956**, *78*, 4071-4077.

### Step 2: 5,6-Dimethyl-2-(2-phenoxyethoxy)-3-(1-piperazinyl)pyrazine, Dihydrochloride.

The product from Step 1 above (506 mg, 2.20 mmol) and 2-phenoxyethanol (370 mg, 2.70 mmol) was dissolved in dioxane (10 mL). KO-*t*-Bu (626 mg, 5.60 mmol) was added and the resulting mixture was stirred at 95 °C for 4 h. The solvent was evaporated and the residue was purified by chromatography on silica gel to give the free base as a yellow oil. The free base was converted to the dihydrochloride salt which was obtained as a monohydrate: yield 0.46 g (50%); mp 138-140 °C. Anal. (C₁₈H₂₄N₄O₂ · 2HCl · H₂O) C, H, N.

### EXAMPLE 184

### 1-[2-(2-Phenoxyethoxy)phenyl]piperazine, Hydrochloride.

### Step 1: tert-Butyl 4-(2-hydroxyphenyl)-1-piperazinecarboxylate.

Triethylamine (8.23 mL, 59.5 mmol) was added to a stirred solution of 2-(1-piperazinyl)phenol dihydrobromide (9.87 g, 29.0 mmol) in a mixture of water and dioxane (1:1, 60 mL). The reaction mixture was cooled on an ice-bath (0 °C) and di-*tert*-butyldicarbonate (6.33 g, 29.0 mmol) was added in one portion. The resulting mixture was allowed to warm to room temperature and stirred for 15 h. The mixture was concentrated to a small volume. Water (20 mL) was added and the white crystals formed were filtered off and dried (1 mm Hg, 70 °C) to give a quantitative yield (7.24 g) of the title compound: mp 115-117 °C. Anal. (C₁₅H₂₂N₂O₃) C, H, N.

### Step 2: 1-[2-(2-Phenoxyethoxy)phenyl]piperazine, Hydrochloride.

The product from Step 1 above (0.55 g, 2.0 mmol) was added to a stirred solution of NaO-*t*-Bu (0.55 g, 2.0 mmol) in DME (5 mL). After 5 min, β-bromophenetole (0.56 g, 2.8 mmol) was added, and the reaction was stirred at 55 °C for 15 h. 2 M HCl was added, and the reaction mixture was stirred for another 3 h. The reaction mixture was concentrated *in vacuo* and the residue was partitioned between 2 M NaOH/CHCl₃. The aqueous phase was extracted with CHCl₃ (x 3). The combined organic layers were dried (MgSO₄), concentrated, and the residue was purified by chromatography on silica gel using CHCl₃/MeOH/NH₄OH (90:10:0.4) as eluent to give the free base of the title compound. The free base was treated with HCl/ether, and the resulting hydrochloride salt was dried (80 °C, 1 mmHg) to afford 0.254 g (35%) of the title compound as a light brown semi-solid material that solidified upon standing. Anal. (C₁₈H₂₂N₂O₂ · HCl · H₂O) H, N; C: calc, 61.27; found, 61.9.

### EXAMPLE 185

### 1-[3-(2-Phenoxyethoxy)-2-pyridinyl]piperazine, Dihydrochloride.

### Step 1: 2-Chloro-3-(2-phenoxyethoxy)pyridine.

A mixture of 2-chloro-3-hydroxypyridine (3.0 g, 23.1 mmol), β-bromophenetole (4.66 g, 23.2 mmol), and K₂CO₃ (8.0 g, 57.9 mmol) in DMF (50 mL) was heated at 100 °C for 1.5 h. The solvent was removed under reduced pressure and the residue was partitioned between EtOAc and H₂O. The organic phase was dried (MgSO₄), filtered, and the solvent evaporated. The solid residue was triturated with a small amount of ether and dried (65 °C, 1 mmHg) to afford 4.23 g (74%) of the title product as white crystals: mp 77-78 °C. Anal. (C₁₃H₁₂ClNO₂) C, H, N.

### Step 2: 1-[3-(2-Phenoxyethoxy)-2-pyridinyl]piperazine, Dihydrochloride.

A mixture of the product from Step 1 above (0.84 g, 3.1 mmol) and piperazine hexahydrate (5.3 g, 27.3 mmol) was heated in a sealed tube at 165 °C for 1 h. After cooling, the mixture was diluted with water (100 mL) and extracted twice with EtOAc. The combined and dried (MgSO₄) organic phases were concentrated *in vacuo* and the residue was purified by chromatography on silica gel using CHCl₃/MeOH/NH₄OH (90:10:0.4) as eluent to give the free base of the title compound. The free base was treated with HCl/ether, and the resulting dihydrochloride salt was dried (80 °C, 1 mmHg) to afford 0.598 g (52%) of the title compound as a light yellow solid: mp 91-120 °C. Anal. (C₁₇H₂₁N₃O₂ · 2HCl) C, H, N.

### EXAMPLE 186

### 2-(2,3-Dihydrobenzofuran-7-yloxy)ethyl 3-(1-piperazinyl)-2-pyrazinyl ether, Maleate.

The title compound obtained in Example 140, Step 2, (390 mg, 0.85 mmol) was dissolved in MeOH (50 mL) and hydrogenated over 10% Pd/C (50 mg) at atmospheric pressure for 7 h. The reaction mixture was filtered through Celite and concentrated. The residue was purified by chromatography on silica gel using EtOAc/MeOH/Et₃N (9:1:0.25) as eluent to give 57 mg (20%) of the free base of the title compound. The free base was converted to the maleate and recrystallized from MeOH/ether: mp 149-154 °C; MS *m*/*z* 343 (M + H)⁺. Anal. (C₁₈H₂₂N₄O₃ · 1.1C₄H₄O₄ · 0.6H₂O) C, H, N.

### EXAMPLE 187

### 5-Chloro-2-(1-piperazinyl)-3-[2-(3-pyridinyloxy)ethoxy]pyrazine, Fumarate.

### Step 1: 3,5-Dichloro-2-(1-piperazinyl)pyrazine.

To a suspension of 2-chloro-3-(1-piperazinyl)pyrazine (11.7 g, 58.7 mmol; from Example 90, Step 1) in CHCl₃ (50 mL) was *N*-chlorosuccinimid (14.4 g, 108 mmol) added and the mixture was heated at reflux for 30 minutes. The reaction mixture was cooled to ambient temperature and extracted twice with water. The combined aqueous phases were made alkaline (11M NaOH), saturated with NaCl, cooled, and extracted with EtOAc (x 3). Concentration of the combined, dried (MgSO₄), organic phases afforded 6.12 g (45%) of the title compound as a brown oil which crystallized upon standing: mp 89-97 °C. Anal. (C₈H₁₀Cl₂N₄ · 1/3 H₂O) C, H, N.

### Step 2: 5-Chloro-2-(1-piperazinyl)-3-[2-(3-pyridinyloxy)ethoxy]pyrazine, Fumarate

The title compound was prepared starting from 2-(3-pyridyloxy)ethanol (from Example 150, Step 1) and the product from Step 1 according to the procedure described in Example 162, Step 2. The crude product was purified by column chromatography on silica using CHCl₃/MeOH/NH₄OH (95:5:0.2, followed by 90:10:0.3) as eluent. After evaporation of the solvent from the combined pure fractions, the resulting oil was crystallized as its fumaric acid salt from MeOH/ether to afford 3.06 g (39%) of the title compound as light yellow crystals: mp 162°C (dec). Anal. (C₁₅H₁₈CIN₅O₂ · C₄H₄O₄) C, H, N .

### EXAMPLE 188

### 5-Bromo-2-(1-piperazinyl)-3-[2-(3-pyridinyloxy)ethoxy]pyrazine, Acetate.

### Step 1: 5-Bromo-3-chloro-2-(1-piperazinyl)pyrazine.

To a mixture of 2-chloro-3-(1-piperazinyl)pyrazine (5.94 g, 30 mmol, from Example 90, Step 1) and Na₂CO₃ (6.0 g, 57 mmol) in AcOH (25 mL) was added a solution of Br₂ (6.00 g, 38.0 mmol) in AcOH (25 mL) dropwise at room temperature. The reaction mixture was stirred overnight. Water (50 mL) was added and the solution was stirred for 30 min and then concentrated to a small volume. Water (20 mL) was added and the solution was basified to pH 9 by adding solid Na₂CO₃. The mixture was extracted with CH₂Cl₂ containing 10% MeOH. The combined extract was evaporated to dryness and the residue was recrystallized from MeOH to give 7.43 g (89%) of the title compound: mp 114-115 °C. HRMS *m*/*z* calcd for C₈H₁₀BrClN₄ (M)⁺ 275.9777, found 275.9765.

### Step 2: 5-Bromo-2-(1-piperazinyl)-3-[2-(3-pyridinyloxy)ethoxy]pyrazine, Acetate.

To a solution of the product obtained in Step 1 above (3.73 g, 13.5 mmol) and 2-(3-pyridyloxy)ethanol (2.06 g, 14.8 mmol, from Example 150, Step 1) in DMSO (10 mL) was added NaH (50% in mineral oil; 0.93 g, 20 mmol) at room temperature. The mixture was stirred at room temperature overnight. EtOAc (100 mL) was added and the mixture was washed with water, dried over Na₂CO₃ and filtered. AcOH (1.0 mL) was added to the filtrate. Crystals were formed upon cooling. The crystals were collected, washed with ethyl ether, and dried in vacuum to give 2.45 g (41 %) of the title product: mp 108-109 °C. Anal. (C₁₅H₁₈BrN₅O₂ · CH₃COOH) C, H, N.

### EXAMPLE 189

### 5-Methyl-2-(1-piperazinyl)-3-[2-(3-pyridinyloxy)ethoxy]pyrazine.

### Step 1: tert-Butyl 4-{5-bromo-3-[2-(3-pyridinyloxy)ethoxy]-2-pyrazinyl}-1-piperazinecarboxylate.

To a solution of the free base of the product obtained in Example 188, Step 2, (0.70 g, 1.85 mmol) in EtOAc (10 mL) and Et₃N (2 mL) was added (*t*-BuO₂C)₂O (0.46 g, 2.0 mmol). The mixture was stirred at room temperature overnight. The reaction mixture was washed with 2 M aqueous NaOH and brine, dried over Na₂CO₃ and concentrated. The residue was purified by column chromatography on silica gel, using ethyl ether/hexane (1:3 to 3:1) as eluent to give 0.64 g (72%) of the title product as an oil. MS *m*/*z* 481 (M)⁺.

### Step 2: tert-Butyl 4-{5-Methyl-3-[2-(3-pyridinyloxy)ethoxy]-2-pyrazinyl}-1-piperazinecarboxylate.

To a mixture of the product obtained in Step 1 (0.38 g, 0.8 mmol) and NiCl₂(dppp) (0.054 g, 0.10 mmol) in dry THF (3 mL) was added dropwise 2N dimethylzinc in toluene (0.5 mL, 1 mmol) at room temperature under stirring. [The reaction was monitored by TLC on SiO₂ using ethyl ether/*n*-hexane (1:1)]. After 4 h, an additional portion (0.3 mL, 0.6 mmol) of the dimethylzinc solution was added. The reaction was stirred for another 2 h and quenched with water (3 mL). The mixture was extracted with ethyl acetate. The organic extract was washed with brine, dried over Na₂CO₃ and concentrated to give 0.29 g of a yellowish oil. Columm chromatography on silica gel, using ethyl ether/*n*-hexane (1:1 to 4:1) as eluent, gave 0.19 g (57%) of the title product as an oil. MS *m*/*z* 415 (M)⁺. HRMS *m*/*z* calcd for C₂₁H₂₉N₅O₄ (M)⁺ 415.2220, found 415.2200.

### Step 3: 5-Methyl-2-(1-piperazinyl)-3-[2-(3-pyridinyloxy)ethoxy]pyrazine.

To a solution of the product obtained in Step 2 (0.507 g, 1.22 mmol) in CH₂Cl₂ (3.0 mL) was added TFA (1.5 mL) at 5 °C. The solution was stirred for 2 h and evaporated to dryness. The residue was dissolved in water (5 mL) and the solution was basified with 50% aqueous NaOH to pH > 13 and extracted with EtOAc. The organic extract was dried over Na₂CO₃.
Evaporation of the solvent gave 0.51 g of the free base. Attempts to prepare a salt of the free base from oxalic acid, fumaric acid or acetic acid failed. The free base was re-generated [treatment with 10% NH₃ aqueous solution, extraction with EtOAc and drying (Na₂CO₃)] to give 0.15 g (40%) of the title compound as an oil. MS *m*/*z* 315 (M)⁺. HRMS *m*/*z* calcd for C₁₆H₂₁N₅O₂ (M)⁺ 315.1695, found 315.1701.

### EXAMPLE 190

### 2-{2-[(3-Methoxy-2-pyrazinyl)oxy)ethoxy}-3-(1-piperazinyl)pyrazine, Maleate. Step 1: tert-Butyl 4-(3-{2-[(3-Chloro-2-pyrazinyl)oxy]ethoxy}-2-pyrazinyl)-1-piperazinecarboxylate.

NaH (50% in mineral oil; 0.153 g, 3.50 mmol) was added to a stirred solution of 2-[3-(4-*tert*-butoxycarbonyl-1-piperazinyl)-2-pyrazinyloxy]ethanol (1.00 g, 3.08 mmol; prepared in Example 52, Step 2) and 2,3-dichloropyrazine (1.0 g, 6.71 mmol) in dioxane (10 mL). The reaction was stirred in a sealed tube at 100 °C overnight. The reaction mixture was poured into water and extracted with diethyl ether. The ether phase was dried (MgSO₄), concentrated and purified by column chromatography on silica using toluene/EtOAc (7:3) as eluent to give 0.823 g (61%) of the title product: Pos-EI-MS shows M⁺ and 14 ions supporting the stated structure. HRMS *m*/*z* calcd for C₁₉H₂₅ClN₆O₄ (M)⁺ 436.1626, found 436.1606.

### Step 2: tert-Butyl 4-(3-{2-[(3-methoxy-2-pyrazinyl)oxy]ethoxy}-2-pyrazinyl)-1-piperazinecarboxylate.

NaH (50% in mineral oil; 0.010 g, 2.1 mmol) was added to a stirred solution of the product from Step 1 (0.38 g, 0.87 mmol) in dioxane (8 mL) and MeOH (2 mL) in a reaction tube. As the gas evolution had ceased, the tube was sealed and the mixture was stirred at 100 °C for 15 min. The reaction was poured into water and extracted with toluene, dried (MgSO₄) and concentrated. The residue was purified by column chromatography on silica using toluene/EtOAc (7:3) as eluent to give 0.178 g (47%) of the title product. Pos-EI-MS shows M⁺ and 11 ions supporting the stated structure.
HRMS *m*/*z* calcd for C₂₀H₂₈N₆O₅ (M)⁺ 432.2121, found 432.2126.

### Step 3: 2-{2-[(3-Methoxy-2-pyrazinyl)oxy]ethoxy}-3-(1-piperazinyl)pyrazine, Maleate.

The product from Step 2 (0.175 g, 0.40 mmol) was treated with CH₂Cl₂/TFA/H₂O (50:45:5; 10 mL) for 30 min at room temperature, poured into 0.1 M aqueous HCl, and washed with toluene (x 2). The aqueous phase was alkalinized to pH 11 with NaOH, and extracted with diethyl ether (x 3). The organic layers were dried (MgSO₄) and concentrated to give 0.120 g (0.36 mmol) of the free base of the title product. Maleic acid (0.042 g, 0.36 mmol) in dioxane (0.5 mL) was added to a solution of the free base in dioxane (3 mL) and left to crystallize to give 0.147 g (82%) of the title product. Pos-EI-MS shows M⁺ and 11 ions supporting the stated structure. HRMS *m*/*z* calcd for C₁₅H₂₀N₆O₃ (M)⁺ 332.1597, found 332.1607.

### EXAMPLE 191

### 2-{2-[(2-Methoxy-3-pyridinyl)oxy]ethoxy}-3-(1-piperazinyl)pyrazine, Fumarate. Step 1: 2-Bromo-3-(2-hydroxyethoxy)pyridine.

The title compound was prepared from 2-bromo-3-hydroxypyridine according to the procedure described in Example 91, Step 1. Oil; yield 2.33 g (76 %).

### Step 2: 3-(2-Hydroxyethoxy)-2-methoxypyridine.

A mixture of the product obtained in Step 1 (2.33 g, 10.7 mmol) and NaOMe (0.634 mg, 11.8 mmol) in MeOH (50 mL) was refluxed overnight. The reaction mixture was filtered and concentrated. The residue was purified by column chromatography on silica, using CH₂Cl₂/MeOH/heptane (4:1:5) as eluent, to give 0.81 g (45%) of the title compound as a colorless oil.

### Step 3: 2-{2-[(2-Methoxy-3-pyridinyl)oxy]ethoxy}-3-(1-piperazinyl)pyrazine, Fumarate.

2-Chloro-3-(1-piperazinyl)pyrazine (0.454 g, 2.13 mmol; from Example 90, Step 1) was added to a mixture of the product obtained in Step 2 (0.380 g, 2.25 mmol) and NaO*t*-Bu (0.432 g, 4.50 mmol) in dioxane (25 mL). The reaction mixture was stirred at 90 °C for 2 h and then quenched by adding MeOH (0.6 mL, 13.3 mmol). Silica gel was added and the mixture was filtered and concentrated *in vacuo.* The residue was purified by column chromatography using CHCl₃/MeOH/heptane (4:1:5) containing 0.2 % aqueous NH₃ as eluent to give 0.257 g (40 %) of the free base of the title compound as a colorless oil. The free base was dissolved in MeOH (3 mL) and converted into its fumarate salt by adding fumaric acid (0.104 g, 0.87 mmol) in MeOH (3 mL) followed by addition of ether to obtain 0.214 g (56 %) of the title compound as white needles: mp 181-183 °C; Anal. (C₂₀H₂₅N₅O₇) C, H, N.

### EXAMPLE 192

### (2R)-1-(3-{2-[(2-Methoxy-3-pyridinyl)oxy]ethoxy}-2-pyrazinyl)-2-methylpiperazine, Fumarate.

### Step 1: (R)-3-Methyl-1-tritylpiperazine.

(2*R*)-Methylpiperazine-*L*-(+)-tartrate* (300 g, 1.2 mol) was added to a hot stirred solution of KOH (240 g, 4.3 mol) in H₂O (240 mL). Two phases were formed and the mixture was cooled to room temperature and extracted with CH₂Cl₂ (3x400 mL). The extracts were dried over K₂CO₃ and filtered. Trityl chloride (260 g, 0.93 mol) was slowly added to a well-stirred solution of the generated (*R*)-2-metyhylpiperazine* in CH₂Cl₂ under cooling (exotermic). After 45 min, the solution was poured into a solution of K₂CO₃ (140 g, 1.00 mol) in water (500 mL). The resulting organic phase was separated and dried over K₂CO₃, and the solvent was evaporated. This gave 370 g of the title product as an oil, which was used without purification.
*Previously reported in *J. Med. Chem.* **1990,** *33*, 1645-1656.

### Step 2: (2R)-1-(3-Chloro-2-pyrazinyl)-2-methylpiperazine.

A mixture of the product obtained in Step 1 above, 2,3-dichloropyrazine (154 g, 1.00 mol) and K₂CO₃ (160 g, 1.20 mol) in DMF (1 L) was heated at 110 °C for 20 h with vigorous stirring. (TLC monitoring system: CHCl₃: EtOH (20: 1); silica). The mixture was cooled and poured slowly into water (6 L) with stirring. The solid was collected, washed with water, dried, dissolved in CHCl₃ (1 L), diluted with n-heptane (1 L), and filtered through SiO₂. Solvents were evaporated off. The resulting oil was dried under vacuum (3 mm/50 °C, 30 min) to remove the DMF. The oil was dissolved in hot ethanol (1 L), and HCl (10% aqueous solution, 300 mL) was added slowly. Trityl carbinol began to crystallize out after 10 min. After 30 min, the formed trityl carbinol was filtered off and the ethanol was evaporated. The aqueous solution was extracted by ether (2 x 200 mL) and made basic by addition of K₂CO₃ to pH 12. The alkaline layer was extracted with CHCl₃ (3 x 200 mL). The combined organic layers were dried (K₂CO₃) and concentrated to give 140 g (66%) of the title compound.

### Step 3: (2R)-1-(3-{2-[(2-Methoxy-3-pyridinyl)oxy]ethoxy}-2-pyrazinyl)-2-methylpiperazine, Fumarate.

The product obtained in Step 2 (0.494 g, 2.18 mmol) was added to a solution of 3-(2-hydroxyethoxy)-2-methoxypyridine (0.410 g, 2.42 mmol; from Example 191, Step 2) and NaO*t*-Bu (0.349 g, 3.63 mmol) in dioxane (25 mL) at 90 °C. The reaction mixture was stirred at 90 °C for 2 h and then quenched by adding MeOH (0.6 mL, 13.3 mmol). Silica gel was added and the mixture was filtered and concentrated in *vacuo.* The residue was purified by column chromatography on silica using CHCl₃/MeOH/*n*-heptane (4:1:5) containing 0.2 % aqueous NH₃ as eluent to give 0.382 g (45 %) of the free base of the title compound as a colorless oil. Part of the free base (0.150 g, 0.48 mmol) was converted to its fumarate salt to give 0.061 g (28%) of the title compound: mp 141-143 °C. Anal. (C₂₁H₂₇N₅O₇ · 0.25 H₂O) C, H, N.

### EXAMPLE 193

### 2-{2-[(2-Chloro-3-pyridinyl)oxy]ethoxy}-3-(1-piperazinyl)pyrazine, Fumarate. Step 1: 2-[(2-Chloro-3-pyridinyl)oxy]ethanol.

The title compound was prepared from 2-chloro-3-hydroxypyridine according to the procedure described in Example 91, Step 1. Oil; yield (77%; purity about 80% according to ¹H NMR). This material was used without further purification in the next synthetic step. HRMS *m*/*z* calcd for C₇H₈ClNO₂ (M)⁺ 173.0240. Found: 173.0244.

### Step 2: 2-{2-[(2-Chloro-3-pyridinyl)oxy]ethoxy}-3-(1-piperazinyl)pyrazine, Fumarate.

The title compound was prepared according to the procedure of Example 90, Step 2, except that the reaction was carried out at room temperature, starting from the product obtained in Step 1 above and 2-chloro-3-(1-piperazinyl)pyrazine (from Example 90, Step 1). The crude product was purified by column chromatography on silica, using CHCl₃/MeOH/NH₄OH (95:5:0.25, followed by 90:10:0.3) as eluent. The free base of the title compound was converted to the fumarate salt. Yield 41 %; mp 200 °C. Anal. (C₁₅H₁₈ClN₅O · C₄H₄O₄) C, H, N.

### EXAMPLE 194

### (2R)-1-(3-{2-[(2-Chloro-3-pyridinyl)oxy]ethoxy}-2-pyrazinyl)-2-methylpiperazine, Fumarate.

The title compound was prepared according to the procedure of Example 192, Step 3, except that the reaction was carried out at room temperature and replacing 3-(2-hydroxyethoxy)-2-methoxypyridine with 2-[(2-chloro-3-pyridinyl)oxy]ethanol (obtained in Example 193, Step 1). The crude product was purified by column chromatography on silica, using CHCl₃/MeOH/NH₄OH (97:3:0.2, followed by 95:5:0.25) as eluent. The free base of the title compound was converted to the fumarate salt: yield 25%; mp 147 °C. Anal. (C₁₆H₂₀ClN₅O₂ · C₄H₄O₄) C, H, N.

### EXAMPLE 195

### 2-{2-[(2-Bromo-3-pyridinyl)oxy]ethoxy}-3-(1-piperazinyl)pyrazine, Fumarate.

The title compound was prepared according to the procedure of Example 90, Step 2, except that the reaction was carried out at room temperature, starting from 2-bromo-3-(2-hydroxyethoxy)pyridine (obtained in Example 191, Step 1) and 2-chloro-3-(1-piperazinyl)pyrazine (from Example 90, Step 1). Yield 39%; mp 202 °C. Anal. (C₁₅H₁₈BrN₅O₂) · C₄H₄O₄) C, H, N.

### EXAMPLE 196

### (2R)-1-(3-{2-[(2-Bromo-3-pyridinyl)oxy]ethoxy}-2-pyrazinyl)-2-methylpiperazine, Fumarate.

The title compound was prepared according to the procedure of Example 192, Step 3, except that the reaction was carried out at room temperature, starting from 2-bromo-3-(2-hydroxyethoxy)pyridine (obtained in Example 191, Step 1) and (2*R*)-1-(3-chloro-2-pyrazinyl)-2-methylpiperazine (from Example 192, Step 2). Yield 23%; mp 154 °C.
Anal. (C(₆H₂₀BrN₅O₂) · C₄H₄O₄) C, H, N.

### EXAMPLE 197

### 2-(2-{[2-(Methylsulfanyl)-3-pyridinyl]oxy}ethoxy)-3-(1-piperazinyl)pyrazine, Fumarate.

### Step 1: 2-{[2-(Methylsulfanyl)-3-pyridinyl]oxy}ethanol.

To a suspension of sodium thiomethoxide (3.01 g, 42.9 mmol) in dry DMF (30 mL) was 2-[(2-chloro-3-pyridinyl)oxy]ethanol (7.82 g, 45.1 mmol, from Example 193, Step 1) added and the reaction mixture was stirred at ambient temperature for 1 h.. The solvent was removed under reduced pressure and the residue was partitioned between brine/1 M aqueous NaOH and CHCl₃. The aqueous phase was extracted with an additional portion of CHCl₃ and the combined organic layers were dried (MgSO₄) and concentrated. The resulting red oil was purified by column chromatography on silica using toluene/Et₃N/MeOH (96:2:2) as eluent to give 3.0 g (36%) of the title compound as brownish crystals: mp 80 °C. Anal. (C₇H₁₁NO₂S) C, H, N.

### Step 2: 2-(2-{[2-(Methylsulfanyl)-3-pyridinyl]oxy}ethoxy)-3-(1-piperazinyl)pyrazine, Fumarate.

The title compound was prepared according to the procedure of Example 90, Step 2, starting from from 2-chloro-3-(1-piperazinyl)pyrazine (from Example 90, Step 1) and the product obtained in Step 1 above. The free base of the title compound was converted to its fumarate salt: yield 47%; mp 201 °C. Anal. (C₁₆H₂₁N₅O₂S · C₄H₄O₄) C, H, N.

### EXAMPLE 198

### (2R)-2-Methyl-1-[3-(2-{[2-(methylsulfanyl)-3-pyridinyl]oxy}ethoxy)-2-pyrazinyl]piperazine, Fumarate.

The title compound was prepared according to the procedure of Example 192, Step 3, starting from (2*R*)-1-(3-chloro-2-pyrazinyl)-2-methylpiperazine (from Example 192, Step 2) and the product of Example 197, Step 1. The free base of the title compound was converted to the fumarate salt: yield 39%; mp 179 °C. Anal. (C₁₇H₂₃N₅O₂S · C₄H₄O₄) C, H, N.

### EXAMPLE 199

### 2-{2-[(2-Ethoxy-3-pyridinyl)oxy]ethoxy}-3-(1-piperazinyl)pyrazine, Fumarate. Step 1: 2-Bromo-3-(2-{[tert-butyl(dimethyl)silyl]oxy}ethoxy)pyridine.

A solution of dimethyl-*t*-butylsilyl chloride (11.1 g, 74 mmol) in DMF (30 mL) was added to a suspension of 2-bromo-3-(2-hydroxyethoxy)pyridine (15.3 g, 70 mmol; obtained in Example 191, Step 1) and imidazole (10.0 g, 147 mmol) in DMF (30 mL). The reaction mixture was stirred at ambient temperature for 2 h, diluted with 0.5 M aqueous NaOH (0.7 L), and extracted twice with toluene. The combined organic phases were washed once with water, dried (MgSO₄), and concentrated. The resulting oil was treated with a mixture of petroleum ether and ethyl acetate (95:5; 50 mL) and an analytical sample of white crystals was collected. The remaining crystals were mixed with the filtrate, the solvent was evaporated and the resulting crude product was used directly in the next step. Solid; yield 18.3 g (78%); mp 81 °C. Anal. (C₁₃H₂₂BrNO₂Si) C, H, N.

### Step 2: 2-[(2-Ethoxy-3-pyridinyl)oxy]ethanol.

The product obtained in Step 1 above (13.2 g, 39.7 mmol) was added to sodium ethoxide in EtOH [prepared from Na (10.5 g, 457 mmol) and EtOH (200 mL)]. The resulting mixture was heated at 70 °C overnight. After cooling to ambient temperature, the reaction mixture was diluted with ice water (0.8 L) and extracted with EtOAc (x 4). The combined organic phases were washed twice with brine, dried (MgSO₄) and concentrated. The resulting dark red oil was purified by column chromatography on silica using toluene/Et₃N (95:5), followed by (90:10), as eluent to give 3.0 g (41 %) of the title product as a red oil. HRMS *m*/*z* calcd for C₉H₁₃NO₃ (M)⁺ 183.0896. Found: 183.0895.

### Step 3: 2-{2-[(2-Ethoxy-3-pyridinyl)oxy]ethoxy}-3-(1-piperazinyl)pyrazine, Fumarate.

The title compound was prepared according to the procedure of Example 90, Step 2, starting from 2-chloro-3-(1-piperazinyl)pyrazine (from Example 90, Step 1) and the product obtained in Step 2 above. The free base of the title compound was converted to its fumarate salt: yield 30%; mp 186 °C. Anal. (C₁₇H₂₃N₅O₃ · C₄H₄O₄) C, H, N.

### EXAMPLE 200

### (2R)-1-(3-{2-[(2-Ethoxy-3-pyridinyl)oxy]ethoxy}-2-pyrazinyl)-2-methylpiperazine, Fumarate.

The title compound was prepared according to the procedure of Example 192, Step 3, starting from (2*R*)-1-(3-chloro-2-pyrazinyl)-2-methylpiperazine (from Example 192, Step 2) and 2-[(2-ethoxy-3-pyridinyl)oxy]ethanol (from Example 199, Step 2). The free base of the title compound was converted to its fumarate salt: yield 17%; mp 179 °C.
Anal. (C₁₈H₂₅N₅O₃ · C₄H₄O₄) C, H, N.

### EXAMPLE 201

### 5-Ethoxy-3-pyridinyl 2-({3-[(2R)-2-methylpiperazinyl]-2-pyrazinyl}oxy)ethyl ether, Fumarate.

### Step 1: 2-[(5-Ethoxy-3-pyridinyl)oxy]-1-ethanol.

The title compound was prepared according to the procedure of Example 91, Step 1, starting from 5-ethoxy-3-pyridinol (1.0 g, 7.2 mmol). Yield: 1.23 g (93%). MS *m*/*z* 184 (M + H)⁺.

### Step 2: 5-Ethoxy-3-pyridinyl 2-({3-[(2R)-2-methylpiperazinyl]-2-pyrazinyl}oxy)ethyl ether, Fumarate.

To a stirred solution of the product obtained in Step 1 (660 mg, 3.60 mmol) in dioxane (6 mL) was added K-*t*-BuO (438 mg, 3.90 mmol). After 15 min, (2*R*)-1-(3-chloro-2-pyrazinyl)-2-methylpiperazine (638 mg, 3.0 mmol; from Example 192, Step 2) was added and the solution was stirred at 85 °C for 2 h. After cooling, CHCl₃ was added and the mixture filtered and concentrated in *vacuo.* The residue was purified by chromatography on silica gel using CH₂Cl₂/methanol (9:1) as eluent to give the free base of the title compound (0.60 g, 56%). The free base was converted to the hydrochloride salt: yield 0.53 g (37%); mp. 101-104 °C; MS *m*/*z* 360 (M + H)⁺. HRMS *m*/*z* calcd for C₁₈H₂₅N₅O₃ (M)⁺ 359.1957, found 359.1943.

### EXAMPLE 202

### 3-[(2R)-2-Methylpiperazinyl]-2-pyrazinyl 2-(5-pyrimidinyloxy)ethyl ether, Fumarate.

The title product was prepared according to the procedure of Example 201, Step 2, starting from 2-(5-pyrimidinyloxy)-1-ethanol (505 mg, 3.60 mmol; from Example 166, Step 1) and (2*R*)-1-(3-chloro-2-pyrazinyl)-2-methylpiperazine (638 mg, 3.00 mmol; from Example 192, Step 2). The residue was purified by column chromatography on silica gel using CH₂Cl₂/methanol (9:1) as eluent to give 456 mg (48%) of the free base of the title compound. The free base was converted to the hydrochloride: mp. 145-147 °C; MS *m*/*z* 317 (M + H)⁺. Anal. (C₁₅H₂₀N₆O₂ · 1.3 C₄H₄O₄) C, H, N.

### EXAMPLE 203

### Step 1: 2-[(6-Chloro-3-pyridinyl)oxy]-1-ethanol.

The title compound was prepared according to the procedure of Example 91, Step 1, starting from 2-chloro-5-hydroxypyridine. Solid; yield 78%. MS *m*/*z* 173 (M)⁺. Anal. (C₇H₈CINO₂) C, H, N.

### Step 2: 2-{2-[(6-Chloro-3-pyridinyl)oxy]ethoxy}-3-(1-piperazinyl)pyrazine, Maleate.

The title compound was prepared according to the procedure of Example 90, Step 2, starting from the product obtained in Step 1 above. The crude product was purified by column chromatography (gradient: EtOAc/MeOH (90:10) + 1% NH₃ to EtOAc/MeOH (80:20) + 1% NH₃) on silica to give 22% of the free base of the title compound as a colorless oil. Part of this material was converted to its maleate salt: mp 145.9-146.3 °C; MS *m*/*z* 336 (M+H)⁺. Anal. (C₁₅H₁₈ClN₅O2 · C₄H₄O₄) C, H, N.

### EXAMPLE 204

### 2-{2-[(6-Methoxy-3-pyridinyl)oxy]ethoxy}-3-(1-piperazinyl)pyrazine, Maleate.

### Step 1: 2-[(6-Methoxy-3-pyridinyl)oxy]-1-ethanol.

A mixture of the product of Example 203, Step 1 (513 mg, 2.96 mmol) and freshly prepared NaOMe (3.06 g, 56.6 mmol) in dry dioxane (25 mL) was heated in a scaled Pyrex tube at 130 °C for 24 h. The solvent was evaporated under reduced pressure and the residue was dissolved in CH₂Cl₂. The organic phase was washed with brine, dried (MgSO₄), and concentrated to give the crude product as an oil (430 mg). Purification by column chromatography, using isohexane/EtOAc (1:1) as an eluent, gave the title product as a white solid. Yield 258 mg (51 %); MS *m*/*z* 169 (M)⁺. Anal. (C₈H₁₁NO₃) C, H, N.

### Step 2: 2-{2-[(6-Methoxy-3-pyridinyl)oxy]ethoxy}-3-(1-piperazinyl)pyrazine, Maleate.

The title compound was prepared according to the procedure of Example 90, Step 2, starting from the product obtained in Step 1 above. Yield of free base of the title product 90%. Part of this material was converted to its maleate salt: mp 151.8-152.6 °C; MS *m*/*z* 332 (M+H)⁺. Anal. (C₁₆H₂₁N₅O₃ · C₄H₄O₄) C, H, N.

### EXAMPLE 205

### 4-Methoxy-N,N-dimethyl-3-[2-({3-[(2R)-2-methylpiperazinyl]-2-pyrazinyl}oxy)ethoxy]aniline, Fumarate.

### Step 1: 5-(Dimethylamino)-2-methoxyphenol.

To a stirred solution of 5-amino-2-methoxyphenol (5.0 g, 36 mmol) in ethanol (250 mL), was added 33% formaldehyde (4 x 1.5 mL at times 0, 60 min, 140 min and 185 min) and sodium cyanoborohydride (4 x 0.8 g, at times 30 min, 120 min, 165 min and 210 min). After each addition of the hydride, pH was adjusted to -6 using acetic acid. The mixture was stirred for an additional 2 h and then concentrated in *vacuo.* The residue was partitioned between brine/diethyl ether and the organic phase was dried and concentrated. The residue was purified by column chromatography on silica gel using hexane/EtOAc (7:3) as eluent to give 1.17 g (20%) of the title compound. MS *m*/*z* 168 (M + H)⁺.

### Step 2: 2-[5-(Dimethylamino)-2-methoxyphenoxy]-1-ethanol.

The title compound was prepared according to the procedure of Example 91, Step 1, starting from 5-(dimethylamino)-2-methoxyphenol (1.17 g, 7.0 mmol; from Step 1 above): Yield: 1.44 g (97%).

### Step 3: 4-Methoxy-N,N-dimethyl-3-[2-({3-[(2R)-2-methylpiperazinyl]-2-pyrazinyl}oxy)ethoxy]aniline, Fumarate.

The title product was prepared according to the procedure of Example 192, Step 3, starting from the product obtained in Step 2 above (700 mg, 3.30 mmol) and (2*R*)-1-(3-chloro-2-pyrazinyl)-2-methylpiperazine (585 mg, 2.75 mmol; from Example 192, Step 2). The crude product was purified by column chromatography on silica gel using CH₂Cl₂/CH₃OH (9:1) as eluent to give the free base of the title compound. The free base was converted to the fumaric acid salt. Yield: 135 mg (10% over all); mp. 147-149 °C; MS *m*/*z* 388 (M + H)⁺. Anal. (C₂₀H₂₉N₅O₃ · C₄H₄O₄) C, H, N.
*Previously reported in *J. Med. Chem.* **1963,** *6*, 63-69. However, no experimental details were given.

### EXAMPLE 206

### (2R)-Methyl-1-[3-(2,5-dimethoxyphenoxyethoxy)-2-pyrazinyl]piperazine, Maleate. Maleate.

### Step 1: 2-(2,5-Dimethoxyphenoxy)-1-ethanol*.

The title compound was prepared according to the procedure of Example 91, Step 1, starting from 2,5-dimethoxyphenol.** Yield 85%; mp 50-52 °C. Anal (C₁₀H₁₄O₄) C, H.
**Prepared as described in *Synth.Commun.* **1995,** *25*, 2121-2134.

### Step 2: (2R)-Methyl-1-[3-(2,5-dimethoxyphenoxyethoxy)-2-pyrazinyl]piperazine, Maleate.

The title product was prepared according to the procedure of Example 192, Step 3, starting from the product obtained in Step 1 above and (2*R*)-1-(3-chloro-2-pyrazinyl)-2-methylpiperazine (from Example 192, Step 2). Yield 26%; mp 105-109 °C. Anal (C₁₉H₂₆N₄O₄ · C₄H₄O₄) C, H, N.

### EXAMPLE 207

### 3-(1-Piperazinyl)-2-pyrazinyl 1,2,3,4-tetrahydro-2-naphthalenylmethyl ether, Fumarate.

The title product was prepared according to the procedure of Example 90, Step 2, starting from (1,2,3,4-tetrahydro-naphthalen-2-yl)methanol (0.79 g, 4.88 mmol) and 2-chloro-3-(1-piperazinyl)pyrazine (0.99 g, 4.6 mmol; from Example 90, Step 1). Oil; yield 57% of the free base. The free base was converted to the fumarate: mp 186-188 °C; Anal. (C₁₉H₂₄N₄O · C₄H₄O₄ · 0.25H₂O) C, H, N. *Previously described in *Aust. J. Chem.* **1992**, *45,* 289-308.

### EXAMPLE 208

### 3-[(2R)-2-methylpiperazinyl]-2-pyrazinyl 1,2,3,4-tetrahydro-2-naphthalenylmethyl ether, Fumarate.

The title product was prepared according to the procedure of Example 192, Step 3, starting from (1,2,3,4-tetrahydro-naphthalen-2-yl)methanol (0.98 g, 6.04 mmol) and (2*R*)-1-(3-chloro-2-pyrazinyl)-2-methylpiperazine (1.3 g, 5.7 mmol; from Example 192, Step 2). The product was isolated as a mixture of two diastereomers. Yield of the free base was 63%. The free base was converted to the fumarate salt: mp 169-172 °C. Anal (C₂₀H₂₆N₄O · C₄H₄O₄) C, H, N.

### EXAMPLE 209

### 1-{4-[2-(3-Pyridinyloxy)ethoxy]-1,2,5-thiadiazol-3-yl}piperazine, Maleate.

### Step 1: 3-Chloro-4-(1-piperazinyl)-1,2,5-thiadiazole, Hydrochloride.

A mixture of 3,4-dichloro-1,2,5-thiadiazole (4.00 g, 25.8 mmol) and piperazine hexahydrate (25.0 g, 130 mmol) in DMF (25 mL) was heated at 70 °C for 20 minutes. After cooling to ambient temperature, the reaction mixture was basified (11M NaOH) and brine was added.
The resulting mixture was extracted with CHCl₃ (x 3). The combined organic phases were washed twice with brine/water (1:1), dried (MgSO₄) and the solvent was evaporated. The resulting oil was purified by column chromatography on silica gel using CHCl₃ as eluent to give 2.77 g (52%) of the title compound as a colourless oil which was converted to its hydrochloride salt: mp 231 °C (dec). Anal. (C₆H₉ClN₄S · HCl), C calc 29.89, found 30.4, H, N.

### Step 2: 1-{4-[2-(3-Pyridinyloxy)ethoxy]-1,2,5-thiadiazol-3-yl}piperazine, Maleate.

The product from Step 1 above (0.70 g, 3.42 mmol), 2-(3-pyridinyloxy)-1-ethanol (0.57 g, 4.10 mmol; from Example 150, Step 1) and sodium hydride (60 % in mineral oil, 0.19 g, 4.79 mmol) was stirred at 70 °C in dioxane for 14 h. The reaction mixture was filtered through a pad of silica gel and concentrated. The remaining oil was purified by by C-18 HPLC using CH₃CN/H₂O (gradient: CH₃CN 0% to 100%). This furnished the free base of the title compound as an oil which was converted to the maleate salt: yield 0.16 g (11%); mp 116-118 °C. Anal. (C₁₃H₁₇N₅O₂S · C₄H₄O₄) C, H, N.

### EXAMPLE 210

### 2-[2-(2,3-Dihydro-1,4-benzodioxin-6-yloxy)ethoxy]-3-(1-piperazinyl)pyrazine, Dihydrochloride.

### Step 1: 2-(2,3-Dihydro-1,4-benzodioxin-6-yloxy)ethanol.

The title compound was prepared according to the procedure described in Example 91, Step 1 starting from 6-hydroxy-1,4-benzodioxane.* Oil; yield 55%. MS *m*/*z* 196 (M)⁺. HRMS *m*/*z* calcd for C₁₀H₁₂O₄ (M)⁺ 196.0736, found 196.0735.
*Prepared as described in *Eur. J. Med. Chem.* **1989,** *24*, 619-22.

### Step 2: tert-butyl 4-{3-[2-(2,3-Dihydro-1,4-benzodioxin-6-yloxy)ethoxy]-2-pyrazinyl}-1-piperazinecarboxylate.

To a solution of the product obtained in Step 1 (0.22 g, 1.12 mmol) and 2-chloro-3-(1-piperazinyl)pyrazine (0.25 g, 1.26 mmol; from Example 90, Step 1) in dioxane (5 mL) was added NaH (50% in mineral oil, 0.05 g, 1.1 mmol). The solution was heated at 90°C overnight. After cooling, EtOAc (10 mL) was added and the solution was washed with water, dried over Na₂CO₃ and concentrated. The oily residue (0.51 g) was dissolved in a mixture of EtOAc (2 mL) and Et₃N (1 mL) and (*t*-BuO₂C)₂O (0.32 g, 1.5 mmol) was added.
The resulting mixture was stirred at room temperature overnight. The reaction mixture was concentrated and the residue was purified by column chromatography on silica gel using MeOH/EtOAc (2: 1) as eluent to give 0.43 g (93%) of the title product as an oil. MS *m*/*z* 458 (M)⁺. HRMS *m*/*z* calcd for C₂₃H₃₀N₄O₆ (M)⁺ 458.2165, found 458.2178.

### Step 3: 2-[2-(2,3-Dihydro-1,4-benzodioxin-6-yloxy)ethoxy]-3-(1-piperazinyl)pyrazine, Dihydrochloride.

To a solution of the product obtained in Step 2 (0.43 g, 0.94 mmol) in CH₂Cl₂ (2 mL) was added CF₃COOH (1.5 mL) at room temperature. The solution was stirred at room temperature for 5 h and evaporated to dryness. The residue was dissolved in EtOAc. The solution was washed with 25% aqueous NaOH and dried over K₂CO₃. A solution of HCl in Et₂O was dropped into the solution until no more precipitate formed. The precipitate was collected, washed with ethyl ether, and dried in vacuum to give 251 mg (62%) of the title product: mp 158-160 °C. MS m/z 358 (M)⁺. HRMS *m*/*z* calcd for C₁₈H₂₂N₄O₄ (M)⁺ 358.1641, found 358.1647.

### EXAMPLE 211

### 2-[3-(2-Methoxyphenyl)propoxy]-3-(1-piperazinyl)pyrazine, Maleate.

A mixture of 2-chloro-3-(1-piperazinyl)pyrazine (0.40 g, 2.0 mmol; from Example 90 Step 1), 3-(2-methoxyphenyl)-propan-1-ol (0.50 g, 3.0 mmol), and KO-*t*-Bu (1 M in *t*-BuOH; 3 mL, 3 mmol) in dry dioxane (30 mL) was stirred at reflux for 20 h. The reaction was quenched by addition of water (2 mL). The solvent was evaporated and the crude mixture was passed through a column of hydromatrix material with CH₂Cl₂ as eluent. The elute was concentrated and the remaining oil was purified by silica gel chromatography using EtOAc/MeOH/Et₃N (8:1:1) as eluent. This furnished the free base of the title compound as an oil which was converted to the maleate salt: yield 0.20 g (22%); mp 132-133 °C. Anal. (C₁₈H₂₄N₄O₂ · C₄H₄O₄) C, H, N.

### EXAMPLE 212

### 2-{[(2E)-3-(2-Methoxyphenyl)-2-propenyl]oxy}-3-(1-piperazinyl)pyrazine, Maleate.

The title compound was prepared according to the procedure of Example 90, Step 2, starting from 2-chloro-3-(1-piperazinyl)pyrazine (0.50 g, 2.5 mmol, from Example 90, Step 1) and 2-methoxy-cinnamyl alcohol (0.54 g, 3.3 mmol).
Yield 0.45 g (41%); mp 136 °C (dec). Anal. (C₁₈H₂₂N₄O₂) · C₄H₄O₄) C, H, N.

### EXAMPLE 213

### 3-(1-Piperazinyl)-2-pyrazinyl 6-quinolinylmethyl ether, Fumarate.

The title compound was prepared according to the procedure of Example 90, Step 2, starting from 2-chloro-3-(1-piperazinyl)pyrazine (1.2 g, 5.6 mmol, from Example 90, Step 1) and 6-quinolinemethanol* (0.89 g, 5.6 mmol). Yield 0.80 g (94%); mp 207-209 °C. Anal. (C₁₈H₁₉N₅O · C₄H₄O₄ · 0.25H₂O) C, H, N.
*Previously described in *J. Org. Chem.* **1953,** *18*, 55-58.

### EXAMPLE 214

### N,N'-Dimethyl-N-[3-(2-phenoxyethoxy)-2-pyrazinyl]-1,2-ethanediamine.

A mixture of 2-chloro-3-(2-phenoxyethoxy)pyrazine (50 mg, 0.20 mmol; from Example 1, Step 1) and *N*,*N*-dimethylethylenediamine (0.2 mL, 1.9 mmol) was heated in a MicroWell 10 microwave reactor for 6 min at 100W. The reaction mixture was partitioned between water and EtOAc and applied to a hydromatrix column. The column was eluted with EtOAc and the elute was concentrated. The product was purified by Amberchrom CG-161m LC using CH₃CN/H₂O (gradient: CH₃CN 0% to 100%). Yield 2 mg (4%). MS *m*/*z* 303 (M + H)⁺.

### EXAMPLE 215

### N,N-Dimethyl-2-{[3-(2-phenoxyethoxy)-2-pyrazinyl]sulfanyl}ethanamine.

A mixture of 2-chloro-3-(2-phenoxyethoxy)pyrazine (100 mg, 0.40 mmol, from Example 1, Step 1), dimethylaminoethanethiol hydrochloride (56 mg, 0.40 mmol) and sodium hydride (60% in mineral oil; 32 mg, 0.80 mmol) was stirred at 60 °C for 12 h. The reaction mixture was concentrated and partitioned between water and EtOAc and applied on a hydromatrix column. The column was eluted with EtOAc and the elute was concentrated. The product was purified by Amberchrom CG-161m LC using CH₃CN/H₂O (gradient: CH₃CN 0% to 100%). Yield 24 mg (24%). MS *m*/*z* 320 (M+H)⁺.

### EXAMPLE 216

### (2R)-Methyl-1-(3-{2-[(1-oxido-3-pyridinyl)oxy]ethoxy}-2-pyrazinyl)piperazine.

### Step 1: tert-Butyl (3R)-3-methyl-4-{3-[2-(3-pyridinyloxy)ethoxy]-2-pyrazinyl}-1-piperazinecarboxylate.

Di-*tert*-butyl dicarbonate (1.01 g, 4.64 mmol) in dioxane (50 mL) was added to a solution of the fumararate salt of (2R)-methyl-1-{3-[2-(3-pyridinyloxy)ethoxy]-2-pyrazinyl}piperazine* (2.0 g, 4.64 mmol) in a mixture of dioxane/10% aqueous NaHCO₃ (1:1; 100 mL). The reaction mixture was stirred overnight at room temperature and extracted with EtOAc (3 x100 mL). The organic phase was concentrated and the residue purified by column chromatography on silica using isohexane/EtOAc (3:1 to 1: 1) as eluent. This furnished 1.48 g (77%) of the title product as an oil. MS *m*/*z* 416 (M+H)⁺. HRMS *m*/*z* calcd for C₂₁H₂₉N₅O₄ (M)⁺ 415.2220 found 415.2224.
*The corresponding hydrochloride salt is described in Example 173.

### Step 2: tert-Butyl (3R)-methyl-4-(3-{2-[(1-oxido-3-pyridinyl)oxy]ethoxy}-2-pyrazinyl)-1-piperazinecarboxylate.

MCPBA (2.91 g, 16.9 mmol) was added to the solution of the product obtained in Step 1 above (1.4 g, 3.37 mmol) in CH₂Cl₂ (50 mL). The reaction mixture was stirred for 1 h at room temperature. This gave a mixture of two unknown di-oxidized derivatives (MS). Aqueous sodium metabisulfite (10%, 50 mL) was added and the reaction mixture was stirred at room temperature until the di-oxidized products had converted to a single mono-oxidized derivative. Extraction with EtOAc (3 x 50 mL), concentration, and purification of the residue by column chromatography on silica, using isohexane/EtOAc (3:1 to 1:1) as eluent, furnished 0.96 g (66%) of the title product as an oil. MS *m*/*z* 432 (M+H)⁺. HRMS *m*/*z* calcd for C₂₁H₂₉N₅O₅ (M)⁺ 431.2169, found 431.2171.

### Step 3: (2R)-2-Methyl-1-(3-{2-[(1-oxido-3-pyridinyl)oxy]ethoxy}-2-pyrazinyl)piperazine.

The product obtained in Step 2 (0.86 g, 2.0 mmol) was treated with CH₂Cl₂/TFA (75:25; 10 mL) for 1 h at room temperature. After concentration, the residue was purified by column chromatography on silica using EtOAc/MeOH/Et₃N (80:15:5) as eluent to give 0.32 g (48%) of the title compound as an oil. MS *m*/*z* 332 (M+H)⁺.
HRMS *m*/*z* calcd for C₁₆H₂₁N₅O₃ (M)⁺ 331.1644, found 331.1655.

### EXAMPLE 217

### 2-[(2-Phenoxyethyl)sulfanyl]-3-(1-piperazinyl)pyrazine, Trifluoroacetate.

### Step 1: tert-Butyl 4-{3-[(2-hydroxyethyl)sulfanyl]-2-pyrazinyl}-1-piperazinecarboxylate.

Sodium hydroxide (0.92 g, 23 mmol) was dissolved in water (9 mL) and dioxane (15 mL). 2-Mercaptoethanol (0.54 mL, 7.7 mmol) was added while stirring and 2-chloro-3-(4-*tert*-butoxycarbonyl-1-piperazinyl)pyrazine (2.3 g, 7.7 mmol, from Example 52, step 1) in dioxane (10 mL) was added. The reaction mixture was heated at 100 °C for 22 h. After cooling to room temperature, it was extracted with EtOAc. The organic layer was dried (K₂CO₃) and the solvent was removed under reduced pressure. The residue was purified by chromatography on silica gel using hexane/ EtOAc (1:1) as eluent to give 1.71 g (65%) of the title compound as an orange oil. MS *m*/*z* 341 (M+H)⁺ .

### Step 2: tert-Butyl 4-{3-[(2-phenoxyethyl)sulfanyl]-2-pyrazinyl}-1-piperazinecarboxylate.

The product obtained in Step 1 (0.135 g, 0.39 mmol), triphenylphosphine (0.134 g, 0.51 mmol) and phenol (48 mg, 0.51 mmol) were dissolved in dry THF (4 mL). Diethyl azodicarboxylate (80 µL, 0.51 mmol) was then added dropwise. The reaction mixture was stirred at room temperature overnight. The solvent was then removed under reduced pressure. The residue was purified by chromatography on silica gel using hexane/EtOAc (1:1) follwed by a second column using (CH₂Cl₂ → CH₃OH). This gave 29 mg (18%) of the title compound as a yellow oil.

### Step 3: 2-[(2-Phenoxyethyl)sulfanyl]-3-(1-piperazinyl)pyrazine, Trifluoroacetate.

The product obtained in Step 2 (30 mg, 0.072 mmol) was dissolved in CH₂Cl₂ (0.75 mL) and cooled to ~ 0 °C. Trifluoroacetic acid (0.25 mL) was then added and the reaction mixture was stirred for 40 min at °C. The solvent was evaporated under vacuum. It gave a beige solid 26 mg of the crude product. Purification by reverse phase preparative HPLC on a SymmetryPrep C₁₈ column (150 x 19 mm, 7 µm), eluting with a gradient of 5% CH₃CN in 95% water to 95% CH₃CN in 5% water (0.2% TFA buffer), of a 13 mg sample gave 6 mg (39%) of the title product as a white solid. MS *m*/*z* 317 (M+H)⁺; HRMS *m*/*z* calcd for C₁₆H₂₀N₄OS (M)⁺ 316.1358, found 316.1355. Anal. (C₁₆H₂₀N₄OS · C₂HF₃O₂ · 0.5H₂O) C, H, N.

### EXAMPLE 218

### 7-(2-{[4-(1-Piperazinyl)-1,2,5-thiadiazol-3-yl]oxy}ethoxy)-2H-chromen-2-one.

### Step 1: tert-Butyl 4-(4-chloro-1,2,5-thiadiazol-3-yl)-1-piperazinecarboxylate.

A mixture of *N*-Boc-piperazine (2.97 g, 15.9 mmol), K₂CO₃ (2.20 g, 15.9 mmol) and 3,4-dichloro-1,2,5-thiadiazole (1.5 mL, 15.9 mmol) in CH₃CN (30 mL) was stirred at 100 °C for 19 h and at room temperature for 4 days. The reaction mixture was then diluted with EtOAc and water. The organic phase was dried (K₂CO₃) and the solvent was removed under reduced pressure. The residue was purified by chromatography on two successive silica gel columns eluting with hexane/EtOAc (1:1) and (7:3), respectively. This gave 1.54 g (31%) of the title product as a yellow solid.

### Step 2: tert-Butyl 4-[4-(2-hydroxyethoxy)-1,2,5-thiadiazol-3-yl]-1-piperazinecarboxylate.

K-*t*-BuO (1.24 g, 11 mmol) was added to a mixture of the product obtained in Step 1 (1.98 g, 6.5 mmol) and ethylene glycol (2.54 ml, 45.5 mmol) in pyridine (15 ml). The reaction mixture was stirred at 100 °C for 6 h and at room temperature overnight. The solution was then poured into ice-water and diluted with EtOAc. The organic phase was dried (MgSO₄) and the solvent removed under reduced pressure. The residue was purified by chromatography on silica gel eluting hexane/EtOAc (1:1) to give 1.71 g (65%) of title product as an orange oil. MS *m*/*z* 331 (M+H)⁺.

### Step 3: tert-Butyl 4-(4-{2-[(2-oxo-2H-chromen-7-yl)oxy]ethoxy}-1,2,5-thiadiazol-3-yl)-1-piperazinecarboxylate.

Diethyl azodicarboxylate (DEAD; 61 µl, 0.39 mmol) was added dropwise to a mixture of the product obtained in Step 2 (100 mg, 0.30 mmol), triphenylphosphine (102 mg, 0.39 mmol) and 7-hydroxycoumarin (63 mg, 0.39 mmol) in dry THF (5 mL). The reaction mixture was stirred at room temperature for 5 days. After solvent removal, the residue was purified by chromatography on silica gel eluting with hexane/EtOAc (1:1) to give the title product as a white solid (176 mg) which had low purity (67% by HPLC). This material was used directly in the following step without further purification.

### Step 4: 7-(2-{[4-(1-Piperazinyl)-1,2,5-thiadiazol-3-yl]oxy}ethoxy)-2H-chromen-2-one.

The product obtained in Step 3 (176 mg) was diluted in CH₂Cl₂ (1.5 mL) and cooled to ~0 °C. Trifluoroacetic acid (0.5 mL) was added and the reaction mixture was stirred for 1 h at 5 °C. After solvent removal *in vacuo*, the residue was purified by chromatography on silica gel using hexane/EtOAc (1:1) followed by CH₂Cl₂/MeOH/Et₃N (90:5:5) as eluents This furnished 45 mg (40%) of the title product as a white solid. MS *m*/*z* 375 (M+H)⁺.

### EXAMPLE 219

### 7-(2-{[4-(1-Piperazinyl)-1,2,5-thiadiazol-3-yl]oxy}ethoxy)isoquinoline

### Step 1: tert-Butyl 4-{4-[2-(7-isoquinolinyloxy)ethoxy]-1,2,5-thiadiazol-3-yl}-1-piperazinecarboxylate.

The title compound was prepared according to the procedure of Example 218, Step 3 starting from the product of Example 218, Step 2 (100 mg, 0.30 mmol), triphenylphosphine (102 mg, 0.39 mmol), 7-hydroxyisoquinoline (57 mg, 0.39 mmol) and diethyl azodicarboxylate (61 µl, 0.39 mmol) except that the reaction mixture was stirred at room temperature for 5 days. It gave an orange oil (0.34 g) of low purity (63% by HPLC). It was used directly in the following step without further purification.

### Step 2: 7-(2-{[4-(1-Piperazinyl)-1,2,5-thiadiazol-3-yl]oxy}ethoxy)isoquinoline.

The product obtained in Step 1 (340 mg) was dissolved in CH₂Cl₂ (1.5 mL) and cooled to ~0 °C. Trifluoroacetic acid (0.5 mL) was added and the reaction mixture was stirred at 5 °C for 1 h. After solvent removal *in vacuo*, the residue was purified by chromatography on silica gel using hexane/EtOAc (1:1) followed by CH₂Cl₂/CH₃OH/Et₃N (90:5:5) as eluents. This furnished 59 mg (55%) of the title product as a yellow oil. MS *m*/*z* (M+H)⁺ 358.

### EXAMPLE 220

### 2-{2-[3-(2-Fluoroethoxy)phenoxy]ethoxy}-3-(1-piperazinyl)pyrazine, Trifluoroacetate.

### Step 1: tert-Butyl 4-{3-[2-(3-hydroxyphenoxy)ethoxy]-2-pyrazinyl}-1-piperazinecarboxylate.

To a stirred solution of 2-(3-hydroxyphenoxy)ethanol (7.71 g, 0.05 mol) in dry DMF (100 mL) at ∼0 °C (ice bath) was added NaH (3.90 g, 0.163 mol) in portions under N₂. When the H₂-evolution had ceased, a solution of 2-chloro-3-(4-*tert*-butoxycarbonyl-1-piperazinyl)pyrazine (0.05 mol, 14.9 g; from Example 52, Step 1) in dry DMF (70 mL) was added in one portion. The mixture was stirred at 65 °C for 1.75 h. After cooling, the reaction was quenched by addition of H₂O in DMF. The mixture was filtered and concentrated *in vacuo.* The residue was dissolved in EtOAc, washed with 1 M KHSO₄ and water, dried (Na₂SO₄ + Norit), and concentrated *in vacuo* to give the title compound as a beige sticky oil. Yield 94%. This product was used directly in the next step without further purification.

### Step 2: 2-{2-[3-(2-Fluoroethoxy)phenoxy]ethoxy}-3-(1-piperazinyl)pyrazine, Trifluoroacetate.

The product obtained in Step 1 above (83 mg, 0.20 mmol) was dissolved in CH₂Cl₂ (8.0 mL) and treated first with polymer supported triphenylphosphine (3.0 mmol/g) (133 mg, 0.40 mmol), 2-fluoroethanol (26 mg, 0.40 mmol) and then diethyl azodicarboxylate (DEAD; 70 mg, 0.40 mmol). The reaction mixture was shaken at room temperature, for 18 h, then filtered to remove the solid support. The filtrate was concentrated under reduced pressure then diluted with MeOH (1.5 mL) and purified by preparative C-18 HPLC using CH₃CN/H₂O/TFA (gradient: CH₃CN 60% to 90%, TFA 0.1%) to give the Boc-protected intermediate. This material was not isolated but treated directly with a solution of 25% TFA in CH₂Cl₂ for 30 min then concentrated under reduced pressure to afford 24 mg (25%) of the title compound. MS *m*/*z* 363 (M+H)⁺.

### EXAMPLE 221

### 2-(2-{3-[2-(4-Methyl-1,3-thiazol-5-yl)ethoxy]phenoxy}ethoxy)-3-(1-piperazinyl)pyraxine, Trifluoroacetate.

The title compound was prepared according to the procedure of Example 220, Step 2, starting from 4-methyl-5-thiazolylethanol (57 mg, 0.40 mmol) and the product obtained in Example 220, Step 1, (83 mg, 0.20 mmol) to give 57 mg (51 %) of the title compound. MS *m*/*z* 442 (M+H)⁺.

### EXAMPLE 222

### 2-{2-[3-(Cyclopropylmethoxy)phenoxy]ethoxy}-3-(1-piperazinyl)pyrazine, Trifluoroacetate.

The title compound was prepared according to the procedure of Example 220, Step 2, starting from cyclopropylmethanol (29 mg, 0.40 mmol) and the product obtained in Example 220, Step 1, (83 mg, 0.20 mmol) to give 30 mg (31%) of the title compound. MS *m*/*z* 371 (M+H)⁺.

### EXAMPLE 223

### 2-{2-[3-(3-Butenyloxy)phenoxy]ethoxy}-3-(1-piperazinyl)pyrazine, Trifluoroacetate.

The title compound was prepared according to the procedure of Example 220, Step 2, starting from 3-buten-1-ol (29 mg, 0.40 mmol) and the product obtained in Example 220, Step 1 (83 mg, 0.20 mmol) to give 57 mg (59%) of the title compound. MS *m*/*z* 371 (M+H)⁺.

### EXAMPLE 224

### N,N-Dimethyl-N-{2-[3-(2-{[3-(1-piperazinyl)-2-pyrazinyl]oxy}ethoxy)phenoxy]-ethyl}amine, Trifluoroacetate.

The title compound was prepared according to the procedure of Example 220, Step 2, starting from 2-dimethylaminoethanol (36 mg, 0.40 mmol) and the product obtained in Example 220, Step 1 (83 mg, 0.20 mmol) to give 37 mg (37%) of the title product. MS *(ES+) m*/*z* 388 (M+H)⁺.

### EXAMPLE 225

### 2-(1-Piperazinyl)-3-{2-[3-(tetrahydro-2H-pyran-4-yloxy)phenoxy]ethoxy}pyrazine, Trifluoroacetate.

A solution of the product obtained in Example 220, Step 1 (208 mg, 0.50 mmol) in CH₂Cl₂ (8.0 mL) was treated first with polymer supported triphenylphosphine (3.0 mmol/g) (200 mg, 0.60 mmol), tetrahydro-4*H*-pyran-4-ol (51 mg, 0.50 mmol) and then diethyl azodicarboxylate (87 mg, 0.50 mmol). The reaction mixture was shaken at room temperature for 18 h, then filtered to remove the solid support. The filtrate was concentrated under reduced pressure and the residue was purified by chromatography on silica gel. Elution with CH₂Cl₂/Et₂O (10:1) gave the crude *N*-Boc-protected intermediate which was further purified by preparative C-18 HPLC using CH₃CN/H₂O/TFA (gradient: CH₃CN 60% to 90%, TFA 0.1%). This material was not isolated but treated directly with a solution of 25 % TFA in CH₂Cl₂ for 30 min then concentrated under reduced pressure to afford the title compound 19.0 mg (7%), MS *m*/*z* 401 (M+H)⁺.

### EXAMPLE 226

### 2-{2-[3-(2-Methoxyethoxy)phenoxy]ethoxy}-3-(1-piperazinyl)pyrazine.

### Step 1: tert-Butyl 4-(3-{2-[3-(2-methoxyethoxy)phenoxy]ethoxy}-2-pyrazinyl)-1-piperazinecarboxylate.

NaH (80% dispersion in mineral oil; 66 mg, 2.75 mmol) was added to a stirred solution of the product obtained in Example 220, Step 1 (716 mg, 1.84 mmol) in dry DMF (10 mL). When the H₂-evolution had ceased, 2-bromoethyl methyl ether (256 mg, 1.84 mmol) was added, and the mixture was stirred at 60 °C for 2.5 h. The reaction was quenched by addition of a mixture of HOAc (0.065 mL) and DMF (0.5 mL) followed by a mixture of water (0.065 mL) and DMF (0.50 mL). The resulting mixture was filtered, concentrated *in vacuo,* and the residue was dissolved in toluene. The solution was washed with 1 M aqueous KHSO₄, dried (Na₂SO₄) and concentrated *in vacuo* to give 0.60 g (69%) of the title compound as an oil which was used in the next step without further purification.

### Step 2: 2-{2-[3-(2-Methoxyethoxy)phenoxy]ethoxy}-3-(1-piperazinyl)pyrazine.

The product obtained in Step 1 above was dissolved in a mixture of EtOAc (18 mL) and ether (9 mL) and 5 M HCl/ether (9 mL) was added. The mixture was stirred at room temperature for 3 h. The precipitatated hydrochloride salt was filtered off and washed thoroughly with ether. The hydrochloride was dissolved in EtOAc/MeOH/Et₃N (100:40:5) and purified by column chromatography on silica using NH₃-satd.
EtOAc/CH₃OH (100:15) as eluent. The combined pure fractions were concentrated *in vacuo* and the resulting oil was dried (~0.8 torr, room temperature) to give 0.36 g (69%) of the title product. HRMS *m*/*z* calcd for C₁₉H₂₆N₄O₄ (M)⁺ 374.1954, found 374.1947.

### EXAMPLE 227

### 1-{2-[3-(2-{[3-(1-Piperazinyl)-2-pyrazinyl]oxy}ethoxy)phenoxy]ethyl}-2-pyrrolidinone.

Diethyl azodicarboxylate (DEAD; 575 mg, 3.30 mmol), dissolved in dry THF (5 mL), was added to a stirred mixture of the compound obtained in Example 220, Step 1 (983 mg, 2.36 mmol), 1-(2-hydroxyethyl)-2-pyrrolidinone (427 mg, 3.30 mmol) and Ph₃P (867 mg, 3.30 mmol) in dry THF (50 mL). The reaction mixture was stirred at room temperature overnight and the solvent was evaporated. The residue was treated with EtOAc/hexane (1:1) and solids were filtered off. The filtrate was washed with 0.5 M aqueous Na₂CO₃, dried (Na₂SO₄), and concentrated *in vacuo.* The residue was purified by column chromatography on silica to give 1.1 g (88%) of the N-Boc protected intermediate. This material was directly N-Boc deprotected as described in Example 220, Step 2, to give 0.50 g (50% over two steps) of the title product as an oil. HRMS *m*/*z* calcd for C₂₂H₂₉N₅O₄ (M)⁺ 427.2220, found 427.2227.

### EXAMPLE 228

### 2-(1-Piperazinyl)-3-{2-[3-(tetrahydro-3-furanyloxy)phenoxy]ethoxy}pyrazine.

The title compound was prepared according to the procedure of Example 227 starting from the product of Example 220, Step 1, and 3-hydroxytetrahydrofuran. Yield 29% over two steps. HRMS *m*/*z* calcd for C₂₀H₂₆N₄O₄ (M)⁺ 386.1954, found 386.1963.

### EXAMPLE 229

### 2-(1-Piperazinyl)-3-{2-[3-(tetrahydro-3-furanylmethoxy)phenoxy]ethoxy}pyrazine.

The title compound was prepared according to the procedure of Example 227 starting from the product of Example 220, Step 1, and tetrahydro-3-furanmethanol. Yield 48% over two steps. HRMS *m*/*z* calcd for C₂₁H₂₈N₄O₄ (M)⁺ 400.2111, found 400.2098.

### EXAMPLE 230

### 2-{[3-(2-{[3-(1-Piperazinyl)-2-pyrazinyl]oxy}ethoxy)phenoxy]methyl}benzonitrile, Trifluoroacetate.

A suspension of the product obtained in Example 220, Step 1 (62 mg, 0.15 mmol), 2-cyanobenzyl bromide (20 mg, 0.10 mmol) and polymer supported triazabicyclodecene (PTBD) (400 mg) in MeCN (8 mL) was shaken at room temperature for 48 h.
The mixture was filtered and the filtrate concentrated under reduced pressure to give the crude *N*-Boc protected intermediate which still contained some unreacted phenol (by HPLC). This material was dissolved in MeCN (8 mL) then retreated with PTBD (300 mg) and shaken at room temperature overnight. The mixture was filtered and the filtrate was concentrated under reduced pressure. The residue was then treated directly with a solution of 25% TFA in CH₂Cl₂ for 30 min at room temperature. The reaction mixture was concentrated under reduced pressure to afford 32 mg (59%) of the title compound. MS *m*/*z* 432 (M+H)⁺.

### EXAMPLE 231

### 2-{2-[3-(Benzyloxy)phenoxy]ethoxy}-3-(1-piperazinyl)pyrazine, Trifluoroacetate.

The title compound was prepared according to the procedure of Example 230 starting from the product obtained in Example 220, Step 1 (62 mg, 0.15 mmol), and benzyl bromide (17 mg, 0.10 mmol) to give 14 mg (27%) of the title compound.MS *m*/*z* 407 (M+H)⁺.

### EXAMPLE 232

### 2-(1-Piperazinyl)-3-(2-{3-[2-(2-pyridinyl)ethoxy]phenoxy}ethoxy)pyrazine, Trifluoroacetate.

A solution of the product obtained in Example 220, Step 1 (208 mg, 0.50 mmol) in CH₂Cl₂ (8 mL) was treated first with polymer supported triphenylphosphine (3.0 mmol/g) (200 mg; 0.60 mmol), 2-(2-hydroxyethyl)pyridine (62 mg, 0.50 mmol) and then diethyl azodicarboxylate (87 mg, 0.50 mmol). The reaction mixture was shaken at room temperature for 3 h, then filtered to remove the solid support. The filtrate was concentrated under reduced pressure and the residue was purified by chromatography on silica gel. Elution with diethyl ether gave the crude *N*-Boc-protected intermediate which was treated directly with a solution of 25% TFA in CH₂Cl₂ for 30 min at room temperature. The reaction mixture was concentrated under reduced pressure to afford 54 mg (20%) of the title compound. MS *m*/*z* 422 (M+H)⁺.

### EXAMPLE 233

### 2-[(4-Methoxybenzyl)oxy]-3-(1-piperazinyl)pyrazine.

The title compound was prepared according to the procedure of Example 90, Step 2, starting from 2-chloro-3-(1-piperazinyl)pyrazine (0.80 g, 4.0 mmol; from Example 90, Step 1) and 4-methoxybenzyl alcohol (0.88 g, 6.4 mmol). Oil; yield 72%. MS *m*/*z* 301 (M+H)⁺.

### EXAMPLE 234

### 2-(1-Methyl-2-phenylethoxy)-3-(1-piperazinyl)pyrazine, Trifluoroacetate.

The title compound was prepared according to the procedure of Example 90, Step 2, starting from 2-chloro-3-(1-piperazinyl)pyrazine (0.15 g, 0.75 mmol; from Example 90, Step 1) and 1-phenyl-2-propanol (163 mg, 1.2 mmol).
The crude product was purified by reverse phase preparative HPLC on a SymmetryPrep C₁₈ column (150 x 19 mm, 7 µm), eluting with a gradient of 5% CH₃CN in 95% water to 95% CH₃CN in 5% water (0.2% TFA buffer) to give gave 55 mg (18%) of the title product as a white solid. MS *m*/*z* 299 (M+H)⁺.

### EXAMPLE 235

### 2-[2-(1-Naphthyl)ethoxy]-3-(1-piperazinyl)pyrazine, Trifluoroacetate.

The title compound was prepared according to the procedure of Example 90, Step 2, starting from 2-chloro-3-(1-piperazinyl)pyrazine (0.50 g, 2.5 mmol; from Example 90, Step 1) and 1 naphthaleneethanol (0.69 g, 4.0 mmol). The crude product was purified by reverse phase preparative HPLC on a SymmetryPrep C₁₈ column (150 x 19 mm, 7 µm), eluting with a gradient of 5% CH₃CN in 95% water to 95% CH₃CN in 5% water (0.2% TFA buffer) to give gave 290 mg (26%) of the title product as a white solid. MS *m*/*z* 335 (M+H)⁺. Anal. (C₂₀H₂₂N₄O · C₂HF₃O₂) C, H, N.

### PREPARATION OF PHARMACEUTICAL COMPOSITIONS

EXAMPLE: Preparation of tablets

| | Ingredients | mg/tablet |
|---|---|---|
| 1. | Active compound | 10.0 |
| 2. | Cellulose, microcrystalline | 57.0 |
| 3. | Calcium hydrogen phosphate | 15.0 |
| 4. | Sodium starch glycolate | 5.0 |
| 5. | Silicon dioxide, colloidal | 0.25 |
| 6. | Magnesium stearate | 0.75 |

The active ingredient 1 is mixed with ingredients 2, 3, 4 and 5 for about 10 minutes. The magnesium stearate is then added, and the resultant mixture is mixed for about 5 minutes and compressed into tablet form with or without film-coating.

### Pharmacological tests

The ability of a compound of the invention to bind or act at specific 5-HT receptor subtypes can be determined using *in vitro* and *in vivo* assays known in the art. The biological activity of compounds prepared in the Examples was tested using different tests.

### Affinity assay

The 5-HT_{2c} receptor affinity of compounds in the Examples was determined in competition experiments, where the ability of each compound in serial dilution to displace ³H-labelled 5-HT, bound to membranes prepared from a transfected HEK293 cell line stably expressing the human 5-HT_{2c} receptor protein, was monitored by Scintillation Proximity Assay technology. Non-specific binding was defined using 5 µM mianserin. Results obtained for exemplary compounds of the invention are illustrated in Table 1 below. Typically, the 5HT_{2C} receptor affinity values (Kᵢ, nM) were in the range of 1 nM to 1500 nM.

**Table 1**

| Compound | Ki (nM) |
|---|---|
| Example 23 | 377 |
| Example 57 | 24 |
| Example 82 | 116 |
| Example 125 | 171 |
| Example 122 | 45 |
| Example 150 | 50 |
| Example 151 | 185 |
| Example 167 | 34 |
| Example 173 | 16 |
| Example 183 | 10 |
| Example 194 | 5 |
| Example 231 | 12 |

### Efficacy assay

The agonist efficacy at the 5-HT_{2c} receptor of the compounds in the Examples was determined by the ability of each compound to mobilise intracellular calcium in transfected HEK293 cells, stably expressing the human 5-HT_{2c} receptor protein, using the calcium-chelating fluorescent dye FLUO-3 (Sigma, St. Louis, MO, U.S.A.).

Typically, the maximum responses of 5-HT_{2c} agonists were in the range of 20-100% relative to the maximum response of 5-HT (serotonin) at a concentration of 1 µM.

### Toxicity tests

Acute toxicity studies in mice following oral administration of a number of compounds in the Examples showed that mortality typically occurred at doses between about 200 mg/kg body weight to about 450 mg/kg body weight.

## Claims

1. **A compound of the general formula (Ib):** 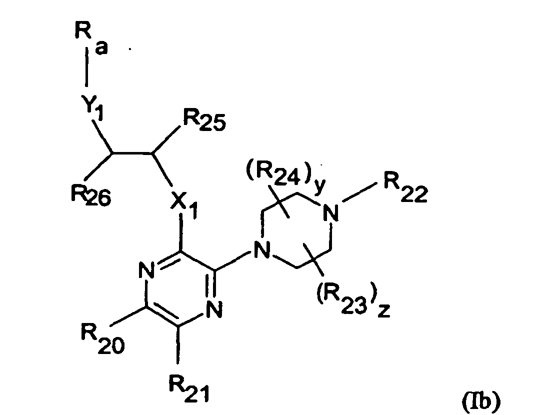 wherein:
X₁ is independently -O-, -S- or -N(R₂₇)-;
Y₁ is independently -O-, -S- or -N(R₂₇)-;
Rₐ is aryl or heteroaryl, each of which may be unsubstituted or substituted in one or more positions independently of each other by C₁₋₆-alkyl, C₁₋₆-alkoxy, fluoro-C₁₋₆-alkoxy, 2,2,2-trifluoroethoxy, C₃₋₅-alkynyloxy, C₃₋₅-alkenyloxy, dimethylamino-C₁₋₆-alkoxy, methylamino-C₁₋₆-alkoxy, C₁₋₆-alkylthio, fluoromethyl, difluoromethyl, trifluoromethyl, fluoromethylthio, difluoromethoxy, difluoromethylthio, trifluoromethoxy, trifluoromethylthio, halogen, hydroxy, nitro, cyano, trifluoromethylsulphonyloxy, C₁₋₆-alkylsulphonamido, C₂₋₆-alkenyl, C₂₋₆-alkynyl, C₁₋₆-acyl, C₁₋₆-alkylcarbonyl-C₁₋₆-alkyl, C₁₋₆-alkylsulphonyl, C₁₋₆-alkylsulphonyloxy, C₁₋₆-alkoxy-C₁₋₆-alkyl, C₁₋₆-alkoxycarbonyl, C₁₋₆-alkoxycarbonyl-C₁₋₆-alkyl, C₁₋₆-acyloxy-C₁₋₆-alkyl, hydroxy C₁₋₆-alkyl, hydroxy-C₁₋₆-alkoxy, C₁₋₆-alkoxy-C₁₋₆-alkoxy, C₁₋₆-alkoxy-C₁₋₆-alkylthio, hydroxy-C₁₋₆-alkylthio, heteroaryl-C₁₋₆-alkylthio, aryl-C₁₋₆-alkylthio, C₁₋₆-alkoxy-C₁₋₆-alkylamino, N-(C₁₋₆-alkoxy-C₁₋₆-alkyl)-N-methylamino, C₃₋₆-cycloalkyl-C₁₋₆-alkoxy, aryl-C₁₋₆-alkoxy, heterocyclyl-C₁₋₆-alkoxy, C₃₋₈-cycloalkyl, C₃₋₈-cycloalkyloxy, aryl, aryloxy, arylthio, arylsulphonyl, aryl-C₁₋₆-acyl, aryl-C₁₋₆-alkyl, aryl-C₂₋₆-alkenyl, aryl-C₂₋₆-alkynyl, heterocyclyl-C₁₋₆-alkyl, heterocyclyl-C₂₋₆-alkenyl, heterocyclyl-C₂₋₆-alkynyl, heterocyclyl, heterocyclyloxy, heterocyclylthio, heterocyclylsulphonyl, heterocyclylamino, heterocyclyl-C₁₋₆-acyl, -N(R₂)(R₃), -CON(R₇)(R₈),
wherein any cycloalkyl, aryl and heterocyclyl residues as substituents on aryl or heteroaryl or as part of substituents on aryl or heteroaryl in turn may be substituted in one or more positions independently of each other by C₁₋₄-alkyl, C₁₋₄-alkoxy, methanesulphonamido, C₁₋₄-alkylthio, C₁₋₄-alkylsulphonyl, C₁-₄-acyl, heterocyclyl, heterocyclyloxy, heterocyclylthio, aryloxy, arylthio, fluoromethyl, trifluoromethyl, trifluoromethoxy, trifluoromethylthio, halogen, hydroxy, nitro, cyano, N(R₂)(R₃) or, for C₃₋₈-cycloalkyl and partially or fully saturated heterocyclyl, oxo or hydroxy;
R₂₀ and R₂₁ independently of each other are hydrogen, C₁₋₄-alkyl (preferably methyl), C₁₋₄-alkoxy (preferably methoxy), C₁₋₄-alkylthio (preferably methylthio), halogen, amino, methylamino, dimethylamino, acetamido, phenyl, phenoxy or phenylthio, wherein phenyl, phenoxy and phenylthio may be unsubstituted or substituted in one or more positions (preferably mono- or disubstituted) independently of each other by halogen, methyl, methoxy, methylthio, cyano, hydroxy or trifluoromethyl, or R₂₀ and R₂₁ together with the carbon atoms to which they are bound form a 5- or 6-membered aromatic or heteroaromatic ring, which optionally is independently substituted in one or more positions by halogen, methyl, methoxy, methylthio, methylsulphonyl, nitro, cyano, hydroxy, trifluoromethyl, trifluoromethylthio, amino, methylamino, dimethylamino or acetamido;
R₂₂ is hydrogen, hydroxy, C₁₋₄-alkyl, C₃₋₄-alkenyl, C₁₋₄-acyl, C₁₋₄-alkoxycarbonyl, 2-hydroxyethyl, 2-cyanoethyl or tetrahydropyran-2-yl;
R₂₃ and R₂₄ independently of each other are hydrogen, C₁₋₄-alkyl, hydroxymethyl, C₁₋₄-alkoxymethyl or fluoromethyl;
R₂₅ is hydrogen or C₁₋4-alkyl;
R₂₆ is hydrogen, C₁₋₄-alkyl or is linked to a carbon atom in Rₐ adjacent to the atom binding to Y₁ to form a 5- or 6-membered ring which may contain an additional heteroatom,
R₂₇ is hydrogen or C₁₋4-alkyl, preferably methyl or ethyl; and
y and z independently of each other are 1 or 2,
and pharmaceutically acceptable salts, hydrates, geometrical isomers, tautomers, optical isomers, and N-oxides thereof.

2. The compound according to claim 1, wherein R₂₀ and R₂₁ independently of each other are hydrogen, halogen or C₁₋₄-alkyl.

3. The compound according to claim 1, wherein R₂₀ and R₂₁ form a ring together with the ring carbons to which they are bound.

4. The compound according to claim 3, wherein R₂₀ and R₂₁ together with the carbon atoms to which they are bound form benzene, to form a quinoxaline ring, optionally mono- or di-substituted by halogen, or thiophene, to form a thieno[3,4-b]pyrazine ring.

5. The compound according to any one of claims 1 to 4, wherein X₁ and Y₁ both are -O-.

6. The compound according to any one of claims 1 to 4, wherein X₁ is -S- and Y₁ is -O-.

7. The compound according to any one of claims 1 to 4, wherein X₁ is -O- and Y₁ is -S-.

8. The compound according to any one of claims 1 to 4, wherein X₁ and Y₁ both are -S-.

9. The compound according to any one of claims 1 to 8, wherein R₂₂ is hydrogen.

10. The compound according to any one of claims 1 to 9, wherein R₂₃ is methyl, z is 1 and R₂₄ is hydrogen, preferably in (R)-configuration.

11. The compound according to any one of claims 1 to 10, wherein R₂₅ is hydrogen and R₂₆ is methyl.

12. The compound according to any one of claims 1 to 11, wherein R₂₅ is hydrogen.

13. The compound according to claim 1, which is:
2-(2-Phenoxy)ethyl 3-(1-piperazinyl)-2-pyrazinyl ether
2-(2-Fluorophenoxy)ethyl 3-(1-piperazinyl)-2-pyrazinyl ether
2-(3-Cyanophenoxy)ethyl 3-(1-piperazinyl)-2-pyrazinyl ether
2-(3-Methoxyphenoxy)ethyl 3-(1-piperazinyl)-2-pyrazinyl ether
2-[3-(2-hydroxyethoxy)phenoxy]ethyl 3-(1-piperazinyl)-2-pyrazinyl ether
2,3-Dihydro-1,4-benzodioxin-2-ylmethyl 3-(1-piperazinyl)-2-pyrazinyl ether
2-(2-Methoxyphenoxy)ethyl 3-(1-piperazinyl)-2-pyrazinyl ether
2-(2,5-Difluorophenoxy)ethyl 3-(1-piperazinyl)-2-pyrazinyl ether
2-(3,5-Dimethoxyphenoxy)ethyl 3-(1-piperazinyl)-2-pyrazinyl ether
2-(3,4-Dihydro-2*H*-pyrido[3,2-*b*]-1,4-oxazin-2-ylmethoxy)-3-(1-piperazinyl)pyrazine
2-Methyl-1-[3-(2-phenoxyethoxy)-2-pyrazinyl]piperazine, particularly the (*R*)-enantiomer thereof
2-Methyl-1-{3-(2-(3-pyridinyloxy)ethoxy]-2-pyrazinyl}piperazine, particularly the (*R*)-enantiomer thereof
2-(Quinazolinyl-8-yloxy)ethyl 3-(1-piperazinyl)-2-pyrazinyl ether
2-(Isoquinolinyl-5-yloxy)ethyl 3-(1-piperazinyl)-2-pyrazinyl ether
2-[2-(3-Pyridinyloxy)ethoxy]-3-(1-piperazinyl)-6,7-difluoroquinoxaline
2-[2-(3-Pyridinyloxy)ethoxy]-3-(1-piperazinyl)thieno[3,4-*b*]pyrazine
2-(3,4-Dihydro-2*H*-1,4-benzoxazin-2-ylmethoxy)-3-(1-piperazinyl)pyrazine
2-Methyl-1-{3-[2-(2-amino-8-quinolinyloxy)ethoxy]-2-pyrazinyl}piperazine, particularly the (*R*)-enantiomer thereof
1-{3-[2-(2-Methoxy-3-pyridinyloxy)ethoxy]-2-pyrazinyl}piperazine
2-Methyl-1-{3-[2-(2-methoxy-3-pyridinyloxy)ethoxy]-2-pyrazinyl}piperazine, particularly the (*R*)-enantiomer thereof,
2-{2-[(2-Chloro-3-pyridinyl)oxy]ethoxy}-3-(1-piperazinyl)pyrazine,
2-{2-[(2-Ethoxy-3-pyridinyl)oxy]ethoxy}-3-(1-piperazinyl)pyrazine,
1-(3-{2-[(2-Ethoxy-3-pyridinyl)oxy]ethoxy}-2-pyrazinyl)-2-methylpiperazine, particularly the (*R*)-enantiomer thereof,
2-(2-{[2-(Methylsulfanyl)-3-pyridinyl]oxy}ethoxy)-3-(1-piperazinyl)pyrazine,
2-Methyl-1-[3-(2-{[2-(methylsulfanyl)-3-pyridinyl]oxy}ethoxy)-2-pyrazinyl]piperazine, particularly the (*R*)-enantiomer thereof,
2-{2-[(2-Bromo-3-pyridinyl)oxy]ethoxy}-3-(1-piperazinyl)pyrazine,
1-(3-{2-[(2-Bromo-3-pyridinyl)oxy]ethoxy}-2-pyrazinyl)-2-methylpiperazine, particularly the (*R*)-enantiomer thereof,
2-(1-Piperazinyl)-3-(2-{3-[2-(2-pyridinyl)ethoxy]phenoxy}ethoxy)pyrazine,
2-(2-{3-[2-(4-Methyl-1,3-thiazol-5-yl)ethoxy]phenoxy}ethoxy)-3-(1-piperazinyl)pyrazine,
2-(1-Piperazinyl)-3-{2-[3-(tetrahydro-3-furanylmethoxy)phenoxy]ethoxy}pyrazine,
2-(1-Piperazinyl)-3-{2-[3-(tetrahydro-3-furanyloxy)phenoxy]ethoxy}pyrazine,
1-{2-[3-(2-{[3-(1-Piperazinyl)-2-pyrazinyl]oxy}ethoxy)phenoxy]ethyl}-2-pyrrolidinone,
2- {2-[3-(2-Methoxyethoxy)phenoxy]ethoxy}-3-(1-piperazinyl)pyrazine,
2-{[3-(2-{[3-(1-Piperazinyl)-2-pyrazinyl]oxy}ethoxy)phenoxy]methyl}-benzonitrile,
2-(1-Piperazinyl)-3-{2-[3-(tetrahydro-2H-pyran-4-yloxy)phenoxy]ethoxy}-pyrazine,
N,N-Dimethyl-N-{2-[3-(2-{[3-(1-piperazinyl)-2-pyrazinyl]oxy}ethoxy)-phenoxy]-ethyl}amine,
7-(2-{[3-(1-Piperazinyl)-2-pyrazinyl]oxy}ethoxy)-2H-chromen-2-one,
1-(3-{2-[(2-Chloro-3-pyridinyl)oxy]ethoxy}-2-pyrazinyl)-2-methylpiperazine, particularly the (R)-enantiomer thereof,
7-Isoquinolinyl 2-{[3-(1-piperazinyl)]-2-pyrazinyl}oxy)ethyl ether,
2-(2-Chloro-4-methoxyphenoxy)ethyl 3-(1-piperazinyl)-2-pyrazinyl ether,
4-(2-{[3-(-Piperazinyl)-2-pyrazinyl]oxy}ethoxy)-2-quinolinamine,
or a pharmacologically acceptable salt or solvate thereof.

14. A compound according to any one of claims 1 to 13 for use in therapy of a human being or an animal.

15. The compound according to claim 14, wherein the therapy is directed towards prophylaxis or treatment of a serotonin-related disease related to the 5-HT_{2c} receptor.

16. The compound according to claim 15, wherein the serotonin-related disease is selected from eating disorders, especially obesity.

17. The compound according to claim 15, wherein the serotonin-related disease is selected from memory disorders.

18. The compound according to claim 15, wherein the serotonin-related disease is selected from mood disorders, comprising major depression and bipolar depression including both mild and bipolar disorder, and seasonal affective disorder (SAD).

19. The compound according to claim 15, wherein the serotonin-related disease is selected from anxiety disorders, including situational anxiety, generalized anxiety disorder, primary anxiety disorders and secondary anxiety disorders.

20. The compound according to claim 15, wherein the serotonin-related disease is selected from sexual dysfunctions and urinary disorders.

21. The compound according to claim 15, wherein the serotonin-related disease is pain.

22. The compound according to claim 15, wherein the serotonin-related disease is schizophrenia.

23. Use of a compound according to any one of claims 1 to 13 for the manufacture of a medicament for treating or preventing a serotonin-related disease, particularly 5-HT_{2c} receptor-related.

24. The use according to claim 23, wherein the serotonin-relateded disease is selected from eating disorders, especially obesity, memory disorders, schizophrenia, mood disorders, anxiety disorders, pain, sexual dysfunctions, and urinary disorders.

25. A pharmaceutical composition comprising a compound according to any one of claims 1 to 13 as an active ingredient, together with a pharmacologically and pharmaceutically acceptable carrier.

26. A method for modulating 5-HT_{2C} receptor function, comprising contacting the receptor with an effective inhibitory amount of a compound according to any of claims 1 to 13.

27. A process for the preparation of a compound of formula (Ib) according to any one of claims 1-13, which process comprises:
reacting a compound of formula (IIIa): 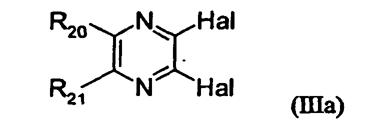
wherein R₂₀ and R₂₁ are as defined for formula (Ib), and Hal is halogen, with a compound of formula (IIIb): 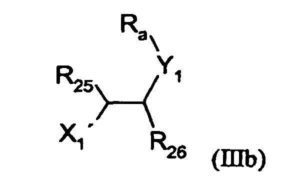 wherein X₁' is selected from -OH, -SH, or -NH(R₂₇), and R₂₅, R₂₆, R₂₇, Y₁, and Rₐ are as defined for formula (Ib), in the presence of a base such as potassium- or sodium *tert*-butoxide or an alkali metal hydride such as sodium hydride or potassium hydride in a suitable solvent such as THF, dioxane or DMF to produce a compound of formula (IIIc): 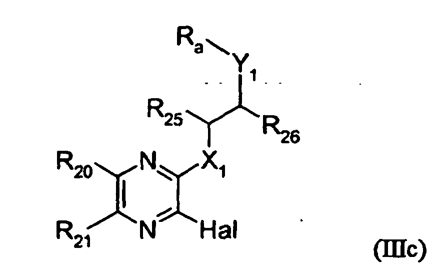 wherein R₂₀, R₂₁, R₂₅, R₂₆, Hal, X₁, Y₁, and Rₐ are as defined above, and subsequently reacting the compound of formula (IIIc) with an appropriate piperazine derivative of formula (IIId): 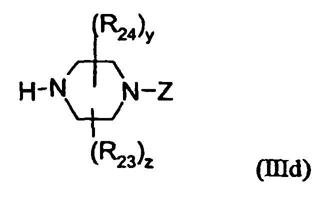 wherein
R₂₃, R₂₄, y and z are as defined for formula (Ib) to produce the compound of formula (Ib); and
Z is as defined for R₂₂ in formula (Ib) or is a suitable protecting group such as *tert*-butoxycarbonyl, trityl or benzyl; or

28. A process for the preparation of a compound of formula (Ib) according to any one of claims 1-13, wherein Y₁ is selected from oxygen or sulphur, which process comprises:
reacting a compound of formula (IIIe): 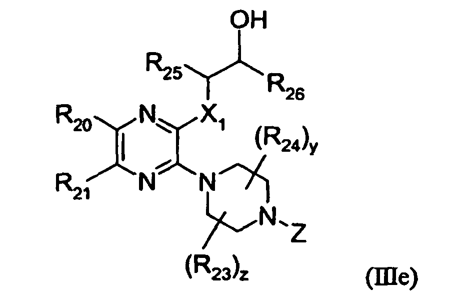
wherein
X₁ is selected from oxygen or sulfur,
R₂₀, R₂₁, R₂₃, R₂₄, y and z are as defined for formula (Ib),
R₂₅ and R₂₆ are each independently hydrogen or methyl, preferably hydrogen; and
Z is as defined for R₂₂ in formula (Ib) or is a suitable protecting group such as *tert* -butoxycarbonyl, trityl or benzyl; with an appropriate phenol or thiophenol corresponding to Rₐ in formula (Ib) under Mitsunobu conditions, e.g., in the presence of diethyl azodicarboxylate (DEAD) or 1,1'-azobis(*N*,*N*-dimethylformamide) (TMAD);
and triphenylphosphine or tri-n-butylphosphine; in a suitable solvent such as tetrahydrofuran (THF) to produce the compound of formula (Ib).

29. A process for the preparation of a compound of formula (Ib) according to any one of claims 1-13 by reacting a compound of formula (IIIa): 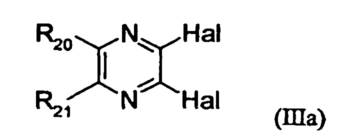 wherein R₂₀ and R₂₁ are as defined for formula (Ib) and Hal is halogen, with a piperazine derivative of formula (IIId): 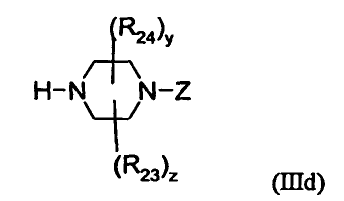 wherein R₂₃, R₂₄, y and z are as defined for formula (Ib), and
Z is as defined for R₂₂ in formula (Ib) or is a suitable protecting group such as *tert*-butoxycarbonyl, trityl or benzyl; to produce a compound of formula (IIIf): 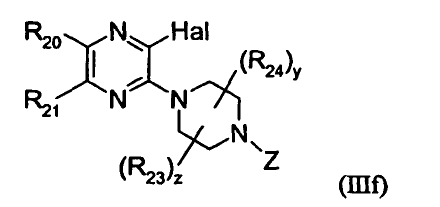 wherein Z, R₂₀, R₂₁, R₂₃, R₂₄, Hal, y and z are as defined above, and subsequently reacting the compound of formula (IIIf) with a compound of formula (IIIb): 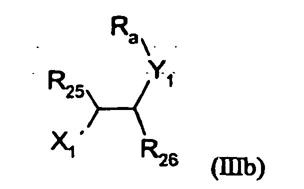 wherein X₁' is selected from -OH, -SH, or -NH(R₂₇) and R₂₅, R₂₆, R₂₇, Y₁, and Rₐ are as defined for formula (Ib), in the presence of a base such as potassium- or sodium *tert*-butoxide or an alkali metal hydride such as sodium hydride or potassium hydride in a suitable solvent such as THF, dioxane or DMF to produce a compound of formula (Ib).

30. A process for the preparation of a compound of formula (Ib) according to any one of claims 1-13 by any one of
(a) converting a resulting compound of formula (Ib) to another compound of formula (Ib);
(b) isolating an isomer of a compound of formula (Ib) from a mixture (e.g., a racemic mixture) thereof; or
(c) converting the free base of a compound of formula (Ib) to a salt thereof.

31. A process according to any one of claims 27 to 29 for the preparation of compounds of formula (Ib) in claim 1 where R₂₂ is H, wherein Z in the corresponding intermediate of formula (IIId), (IIIe) or (IIIf) is a protecting group selected from *tert*-butoxycarbonyl (*t*-BOC), benzyl or trityl.

32. A process according to claim 31 where the said protecting group is cleaved off to produce a compound of formula (Ib).

33. A process according to claim 28, wherein the intermediate of formula (IIIe) is 2-[3-(4-*tert*-butoxycarbonyl-1-piperazinyl)-2-pyrazinyloxy]ethanol.

## Patentansprüche

1. Eine Verbindung mit der allgemeinen Formel (Ib): 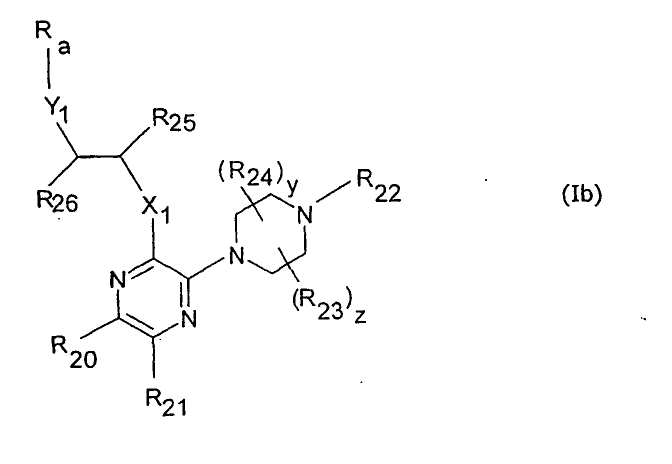 wobei:
X₁ unabhängig -O-, -S- oder -N(R₂₇)- ist;
Y₁ unabhängig -O-, -S- oder -N(R₂₇)- ist;
Rₐ Aryl oder Heteroaryl ist, welches jeweils unsubstituiert oder unabhängig voneinander an einer oder mehreren Positionen substituiert sein kann mit C₁₋₆-Alkyl, C₁₋₆-Alkoxy, Fluor-C₁₋₆-alkoxy, 2,2,2-Trifluorethoxy, C₃₋₅-Alkinyloxy, C₃₋₅-Alkenyloxy, Dimethylamino-C₁₋₆-alkoxy, Methylamino-C₁₋₆-alkoxy, C₁₋₆-Alkylthio, Fluormethyl, Difluormethyl, Trifluormethyl, Fluormethylthio, Difluormethoxy, Difluormethylthio, Trifluormethoxy, Trifluormethylthio, Halogen, Hydroxy, Nitro, Cyano, Trifluormethylsulfonyloxy, C₁₋₆-Alkylsulfonamido, C₂₋₆-Alkenyl, C₂₋₆-Alkinyl, C₁₋₆-Acyl, C₁₋₆-Alkylcarbonyl-C₁₋₆-alkyl, C₁₋₆-Alkylsulfonyl, C₁₋₆-Alkylsulfonyloxy, C₁₋₆-Alkoxy-C₁₋₆-alkyl, C₁₋₆-Alkoxycarbonyl, C₁₋₆-Alkoxycarbonyl-C₁₋₆-alkyl, C₁₋₆-Acyloxy-C₁₋₆-alkyl, Hydroxy-C₁₋₆-alkyl, Hydroxy-C₁₋₆-alkoxy, C₁₋₆-Alkoxy-C₁₋₆-alkoxy, C₁₋₆-Alkoxy-C₁₋₆-alkylthio, Hydroxy-C₁₋₆-alkylthio, Heteroaryl-C₁₋₆-alkylthio, Aryl-C₁₋₆-alkylthio, C₁₋₆-Alkoxy-C₁₋₆-alkylamino, N-(C₁₋₆-Alkoxy-C₁₋₆-alkyl)-N-methylamino, C₃₋₆-Cycloalkyl-C₁₋₆-alkoxy, Aryl-C₁₋₆-alkoxy, Heterocyclyl-C₁₋₆-alkoxy, C₃₋₈-Cycloalkyl, C₃₋₈-Cycloalkyloxy, Aryl, Aryloxy, Arylthio, Arylsulfonyl, Aryl-C₁₋₆-acyl, Aryl-C₁₋₆-alkyl, Aryl-C₂₋₆-alkenyl, Aryl-C₂₋₆-alkinyl, Heterocyclyl-C₁₋₆-alkyl, Heterocyclyl-C₂₋₆-alkenyl, Heterocyclyl-C₂₋₆-alkinyl, Heterocyclyl, Heterocyclyloxy, Heterocyclylthio, Heterocyclylsulfonyl, Heterocyclylamino, Heterocyclyl-C₁₋₆-acyl, -N(R₂)(R₃), -CON(R₇)(R₈),
wobei jeder Cycloalkyl-, Aryl- und Heterocyclylrest als Substituent an Aryl oder Heteroaryl oder als Teil von Substituenten an Aryl und Heteroaryl wiederum an einer oder mehreren Positionen unabhängig voneinander substituiert sein kann mit C₁₋₄-Alkyl, C₁₋₄-Alkoxy, Methansulfonamido, C₁₋₄-Alkylthio, C₁₋₄-Alkylsulfonyl, C₁₋₄-Acyl, Heterocyclyl, Heterocyclyloxy, Heterocyclylthio, Aryloxy, Arylthio, Fluormethyl, Trifluormethyl, Trifluormethoxy, Trifluormethylthio, Halogen, Hydroxy, Nitro, Cyano, N(R₂)(R₃) oder für C₃₋₈-Cycloalkyl und teilweise oder vollständig gesättigtes Heterocyclyl, Oxo oder Hydroxy;
R₂₀ und R₂₁ unabhängig voneinander Wasserstoff, C₁₋₄-Alkyl (vorzugsweise Methyl), C₁₋₄-Alkoxy (vorzugsweise Methoxy), C₁₋₄-Alkylthio (vorzugsweise Methylthio), Halogen, Amino, Methylamino, Dimethylamino, Acetamido, Phenyl, Phenoxy oder Phenylthio sind, wobei Phenyl, Phenoxy und Phenylthio unsubstituiert oder an einer oder mehreren Positionen unabhängig voneinander substituiert (vorzugsweise mono- oder disubstituiert) sein können mit Halogen, Methyl, Methoxy, Methylthio, Cyano, Hydroxy oder Trifluormethyl, oder R₂₀ und R₂₁ zusammen mit den Kohlenstoffatomen, an welche sie gebunden sind, einen 5- oder 6-gliedrigen aromatischen oder heteroaromatischen Ring bilden, welcher gegebenenfalls an einer oder mehreren Positionen unabhängig substituiert ist mit Halogen, Methyl, Methoxy, Methylthio, Methylsulfonyl, Nitro, Cyano, Hydroxy, Trifluormethyl, Trifluormethylthio, Amino, Methylamino, Dimethylamino oder Acetamido;
R₂₂ Wasserstoff, Hydroxy, C₁₋₄-Alkyl, C₃₋₄-Alkenyl, C₁₋₄-Acyl, C₁₋₄-Alkoxycarbonyl, 2-Hydroxyethyl, 2-Cyanoethyl oder Tetrahydropyran-2-yl ist;
R₂₃ und R₂₄ unabhängig voneinander Wasserstoff, C₁₋₄-Alkyl, Hydroxymethyl, C₁₋₄-Alkoxymethyl oder Fluormethyl sind;
R₂₅ Wasserstoff oder C₁₋₄-Alkyl ist;
R₂₆ Wasserstoff, C₁₋₄-Alkyl ist oder mit einem Kohlenstoffatom in Rₐ verknüpft ist, das zu dem Atom, welches an Y₁ bindet, benachbart ist, um einen 5- oder 6-gliedrigen Ring zu bilden, der ein zusätzliches Heteroatom enthalten kann;
R₂₇ Wasserstoff oder C₁₋₄-Alkyl, vorzugsweise Methyl oder Ethyl ist; und
y und z unabhängig voneinander 1 oder 2 sind,
und pharmazeutisch verträgliche Salze, Hydrate, geometrische Isomere, Tautomere, optische Isomere und N-Oxide von dieser.

2. Die Verbindung gemäß Anspruch 1, wobei R₂₀ und R₂₁ unabhängig voneinander Wasserstoff, Halogen oder C₁₋₄-Alkyl sind.

3. Die Verbindung gemäß Anspruch 1, wobei R₂₀ und R₂₁ zusammen mit den Ringkohlenstoffen, an welche sie gebunden sind, einen Ring bilden.

4. Die Verbindung gemäß Anspruch 3, wobei R₂₀ und R₂₁ zusammen mit den Kohlenstoffatomen, an welche sie gebunden sind, Benzol bilden, um einen Chinoxalinring zu bilden, welcher gegebenenfalls mit Halogen mono- oder disubstituiert ist, oder Thiophen bilden, um einen Thieno[3,4-b]pyrazinring zu bilden.

5. Die Verbindung gemäß irgendeinem der Ansprüche 1 bis 4, wobei X₁ und Y₁ beide -O- sind.

6. Die Verbindung gemäß irgendeinem der Ansprüche 1 bis 4, wobei X₁ -S- ist und Y₁ -O- ist.

7. Die Verbindung gemäß irgendeinem der Ansprüche 1 bis 4, wobei X₁ -O- ist und Y₁ -S- ist.

8. Die Verbindung gemäß irgendeinem der Ansprüche 1 bis 4, wobei X₁ und Y₁ beide -S- sind.

9. Die Verbindung gemäß irgendeinem der Ansprüche 1 bis 8, wobei R₂₂ Wasserstoff ist.

10. Die Verbindung gemäß irgendeinem der Ansprüche 1 bis 9, wobei R₂₃ Methyl ist, z 1 ist und R₂₄ Wasserstoff ist, vorzugsweise in der (R)-Konfiguration.

11. Die Verbindung gemäß irgendeinem der Ansprüche 1 bis 10, wobei R₂₅ Wasserstoff ist und R₂₆ Methyl ist.

12. Die Verbindung gemäß irgendeinem der Ansprüche 1 bis 11, wobei R₂₅ Wasserstoff ist.

13. Die Verbindung gemäß Anspruch 1, welche ist:
2-(2-Phenoxy)ethyl-3-(1-piperazinyl)-2-pyrazinylether
2-(2-Fluorphenoxy)ethyl-3-(1-piperazinyl)-2-pyrazinylether
2-(3-Cyanophenoxy)ethyl-3-(1-piperazinyl)-2-pyrazinylether
2-(3-Methoxyphenoxy)ethyl-3-(1-piperazinyl)-2-pyrazinylether
2-[3-(2-Hydroxyethoxy)phenoxy]ethyl-3-(1-piperazinyl)-2-pyrazinylether
2,3-Dihydro-1,4-benzodioxin-2-ylmethyl-3-(1-piperazinyl)-2-pyrazinylether
2-(2-Methoxyphenoxy)ethyl-3-(1-piperazinyl)-2-pyrazinylether
2-(2,5-Difluorphenoxy)ethyl-3-(1-piperazinyl)-2-pyrazinylether
2-(3,5-Dimethoxyphenoxy)ethyl-3-(1-piperazinyl)-2-pyrazinylether
2-(3,4-Dihydro-2*H*-pyrido[3,2-*b*]-1,4-oxazin-2-ylmethoxy)-3-(1-piperazinyl)pyrazin
2-Methyl-1-[3-(2-phenoxyethoxy)-2-pyrazinyl]piperazin, insbesondere das (R)-Enantiomer davon
2-Methyl-1-{3-(2-(3-Pyridinyloxy)ethoxy]-2-pyrazinyl}piperazin, insbesondere das (R)-Enantiomer davon
2-(Chinazolinyl-8-yloxy)ethyl-3-(1-piperazinyl)-2-pyrazinylether
2-(Isochinolinyl-5-yloxy)ethyl-3-(1-piperazinyl)-2-pyrazinylether
2-[2-(3-Pyridinyloxy)ethoxy]-3-(1-piperazinyl)-6,7-difluorchinoxalin
2-[2-(3-Pyridinyloxy)ethoxy]-3-(1-piperazinyl)thieno[3,4-*b*]pyrazin
2-(3,4-Dihydro-2*H*-1,4-benzoxazin-2-ylmethoxy)-3-(1-piperazinyl)pyrazin
2-Methyl-1-{3-[2-(2-amino-8-chinolinyloxy)ethoxy]-2-pyrazinyl}piperazin, insbesondere das (R)-Enantiomer davon
1-{3-[2-(2-Methoxy-3-pyridinyloxy)ethoxy]-2-pyrazinyl}piperazin
2-Methyl-1-{3-[2-(2-methoxy-3-pyridinyloxy)ethoxy]-2-pyrazinyl}piperazin, insbesondere das (R)-Enantiomer davon,
2-{2-[(2-Chlor-3-pyridinyl)oxy]ethoxy}-3-(1-piperazinyl)pyrazin,
2-{2-[(2-Ethoxy-3-pyridinyl)oxy]ethoxy}-3-(1-piperazinyl)pyrazin,
1-(3-{2-[(2-Ethoxy-3-pyridinyl)oxy]ethoxy}-2-pyrazinyl)-2-methylpiperazin, insbesondere das (R)-Enantiomer davon,
2-(2-{[2-(Methylsulfanyl)-3-pyridinyl]oxy}ethoxy)-3-(1-piperazinyl)pyrazin,
2-Methyl-1-[3-(2-{[2-(methylsulfanyl)-3-pyridinyl]oxy}ethoxy)-2-pyrazinyl]piperazin, insbesondere das (R)-Enantiomer davon,
2-{2-[(2-Brom-3-pyridinyl)oxy]ethoxy}-3-(1-piperazinyl)pyrazin,
1-(3-{2-[(2-Brom-3-pyridinyl)oxy]ethoxy}-2-pyrazinyl)-2-methylpiperazin, insbesondere das (R)-Enantiomer davon,
2-(1-Piperazinyl)-3-(2-{3-[2-(2-pyridinyl)ethoxy]phenoxy}ethoxy)pyrazin,
2-(2-{3-[2-(4-Methyl-1,3-thiazol-5-yl)ethoxy]phenoxy}ethoxy)-3-(1-piperazinyl)-pyrazin,
2-(1-Piperazinyl)-3-{2-[3-(tetrahydro-3-furanylmethoxy)phenoxy]ethoxy}pyrazin,
2-(1-Piperazinyl)-3-{2-[3-(tetrahydro-3-furanyloxy)phenoxy]ethoxy}pyrazin,
1-{2-[3-(2-{[3-(1-Piperazinyl)-2-pyrazinyl]oxy}ethoxy)phenoxy]ethyl}-2-pyrrolidinon,
2-{2-[3-(2-Methoxyethoxy)phenoxy]ethoxy}-3-(1-piperazinyl)pyrazin,
2-{[3-(2-{[3-(1-Piperazinyl)-2-pyrazinyl]oxy}ethoxy)phenoxy]methyl}benzonitril,
2-(1-Piperazinyl)-3-{2-[3-(tetrahydro-2H-pyran-4-yloxy)phenoxy]ethoxy}pyrazin,
N,N-Dimethyl-N-{2-[3-(2-{[3-(1-piperazinyl)-2-pyrazinyl]oxy}ethoxy)phenoxy]ethyl}amin,
7-(2-{[3-(1-Piperazinyl)-2-pyrazinyl]oxy}ethoxy)-2H-chromen-2-on,
1-(3-{2-[(2-Chlor-3-pyridinyl)oxy]ethoxy}-2-pyrazinyl)-2-methylpiperazin, insbesondere das (R)-Enantiomer davon,
7-Isochinolinyl-2-{[3-(1-piperazinyl)]-2-pyrazinyl}oxy)ethylether,
2-(2-Chlor-4-methoxyphenoxy)ethyl-3-(1-piperazinyl)-2-pyrazinylether,
4-(2-{[3-(1-Piperazinyl)-2-pyrazinyl]oxy}ethoxy)-2-chinolinamin,
oder ein pharmakologisch verträgliches Salz oder Solvat von diesen.

14. Eine Verbindung gemäß irgendeinem der Ansprüche 1 bis 13 zur Verwendung bei einer Therapie an einem menschlichen Wesen oder einem Tier.

15. Die Verbindung gemäß Anspruch 14, wobei die Therapie auf die Prophylaxe oder Behandlung einer mit Serotonin zusammenhängenden Krankheit, die mit dem 5-HT_{2C}-Rezeptor zusammenhängt, gerichtet ist.

16. Die Verbindung gemäß Anspruch 15, wobei die mit Serotonin zusammenhängende Krankheit ausgewählt ist aus Essstörungen, insbesondere Adipositas.

17. Die Verbindung gemäß Anspruch 15, wobei die mit Serotonin zusammenhängende Krankheit ausgewählt ist aus Gedächtnisstörungen.

18. Die Verbindung gemäß Anspruch 15, wobei die mit Serotonin zusammenhängende Krankheit ausgewählt ist aus Stimmungsstörungen, welche schwergradige Depression und bipolare Depression, einschließlich sowohl milder als auch bipolarer Störung, und saisonal abhängige Depression (SAD) umfassen.

19. Die Verbindung gemäß Anspruch 15, wobei die mit Serotonin zusammenhängende Krankheit ausgewählt ist aus Angststörungen, einschließlich Situationsangst, generalisierter Angststörung, primären Angststörungen und sekundären Angststörungen.

20. Die Verbindung gemäß Anspruch 15, wobei die mit Serotonin zusammenhängende Krankheit ausgewählt ist aus sexuellen Fehlfunktionen und Harnstörungen.

21. Die Verbindung gemäß Anspruch 15, wobei die mit Serotonin zusammenhängende Krankheit Schmerz ist.

22. Die Verbindung gemäß Anspruch 15, wobei die mit Serotonin zusammenhängende Krankheit Schizophrenie ist.

23. Verwendung einer Verbindung gemäß irgendeinem der Ansprüche 1 bis 13 zur Herstellung eines Medikaments zur Behandlung oder Prävention einer mit Serotonin zusammenhängenden Krankheit, insbesondere einer, die mit dem 5-HT_{2C}-Rezeptor zusammenhängt.

24. Die Verwendung gemäß Anspruch 23, wobei die mit Serotonin zusammenhängende Krankheit ausgewählt wird aus Essstörungen, insbesondere Adipositas, Gedächtnisstörungen, Schizophrenie, Stimmungsstörungen, Angststörungen, Schmerz, sexuellen Fehlfunktionen und Harnstörungen.

25. Eine pharmazeutische Zusammensetzung, welche eine Verbindung gemäß irgendeinem der Ansprüche 1 bis 13 als einen aktiven Bestandteil zusammen mit einem pharmakologisch und pharmazeutisch verträglichen Träger umfasst.

26. Ein Verfahren zum Modulieren der 5-HT_{2C}-Rezeptorfunktion, umfassend das Inkontaktbringen des Rezeptors mit einer effektiven inhibitorischen Menge einer Verbindung gemäß irgendeinem der Ansprüche 1 bis 13.

27. Ein Verfahren zur Herstellung einer Verbindung mit der Formel (Ib) gemäß irgendeinem der Ansprüche 1-13, wobei das Verfahren umfasst:
Umsetzen einer Verbindung mit der Formel (IIIa): 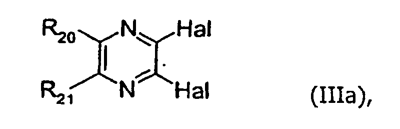
wobei R₂₀ und R₂₁ wie für Formel (Ib) definiert sind und Hal Halogen ist, mit einer Verbindung mit der Formel (IIIb): 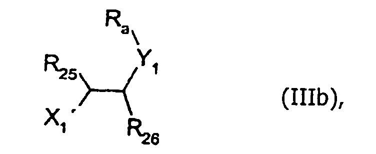 wobei X₁' ausgewählt wird aus -OH, -SH oder -NH(R₂₇) und R₂₅, R₂₆, R₂₇, Y₁ und Rₐ wie für Formel (Ib) definiert sind, in der Gegenwart einer Base wie z.B. Kalium- oder Natrium-tert butoxid oder eines Alkalimetallhydrids wie z.B. Natriumhydrid oder Kaliumhydrid in einem geeigneten Lösungsmittel wie z.B. THF, Dioxan oder DMF, um eine Verbindung mit der Formel (IIIc) herzustellen: 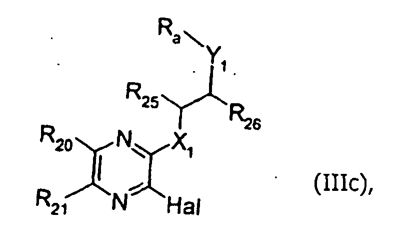 wobei R₂₀, R₂₁, R₂₅, R₂₆, Hal, X₁, Y₁ und Rₐ wie oben definiert sind, und anschließend Umsetzen der Verbindung mit der Formel (IIIc) mit einem geeigneten Piperazinderivat mit der Formel (IIId): 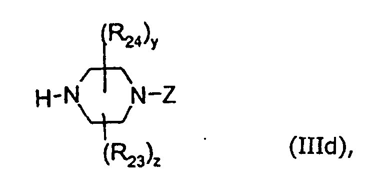 wobei R₂₃, R₂₄, y und z wie für Formel (Ib) definiert sind, um die Verbindung mit der Formel (Ib) herzustellen; und
Z wie für R₂₂ in Formel (Ib) definiert ist oder eine geeignete Schutzgruppe wie z.B. *tert*-Butoxycarbonyl, Trityl oder Benzyl ist; oder

28. Ein Verfahren zur Herstellung einer Verbindung mit der Formel (Ib) gemäß irgendeinem der Ansprüche 1-13, wobei Y₁ ausgewählt wird aus Sauerstoff oder Schwefel, wobei das Verfahren umfasst:
Umsetzen einer Verbindung mit der Formel (IIIe): 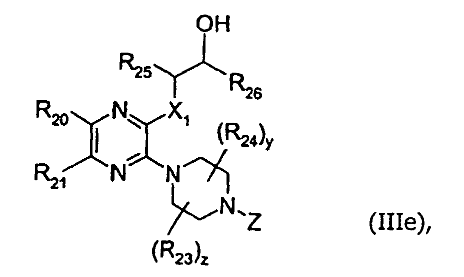
wobei
X₁ ausgewählt wird aus Sauerstoff oder Schwefel,
R₂₀, R_{21,} R_{23,} R_{24,} y und z wie für Formel (Ib) definiert sind,
R₂₅ und R₂₆ jeweils unabhängig Wasserstoff oder Methyl, vorzugsweise Wasserstoff sind; und
Z wie für R₂₂ in Formel (Ib) definiert ist oder eine geeignete Schutzgruppe wie z.B. *tert*-Butoxycarbonyl, Trityl oder Benzyl ist; mit einem geeigneten Phenol oder Thiophenol, das Rₐ in der Formel (Ib) entspricht, unter Mitsunobu-Bedingungen, z.B. in der Gegenwart von Diethylazodicarboxylat (DEAD) oder 1,1'-Azobis(*N,N*-dimethylformamid) (TMAD); und Triphenylphosphin oder Tri-*n*-butylphosphin; in einem geeigneten Lösungsmittel wie z.B. Tetrahydrofuran (THF), um die Verbindung mit der Formel (Ib) herzustellen.

29. Ein Verfahren zur Herstellung einer Verbindung mit der Formel (Ib) gemäß irgendeinem der Ansprüche 1-13 durch Umsetzen einer Verbindung mit der Formel (IIIa): 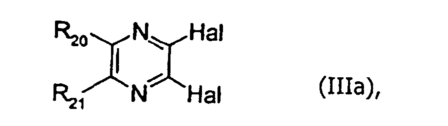 wobei R₂₀ und R₂₁ wie für Formel (Ib) definiert sind und Hal Halogen ist, mit einem Piperazinderivat mit der Formel (IIId): 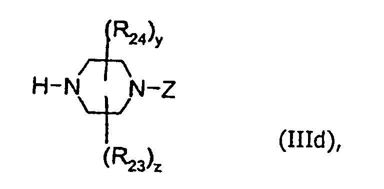 wobei R₂₃, R₂₄, y und z wie für Formel (Ib) definiert sind, und
Z wie für R₂₂ in Formel (Ib) definiert ist oder eine geeignete Schutzgruppe wie z.B. *tert*-Butoxycarbonyl, Trityl oder Benzyl ist; um eine Verbindung mit der Formel (IIIf) herzustellen: 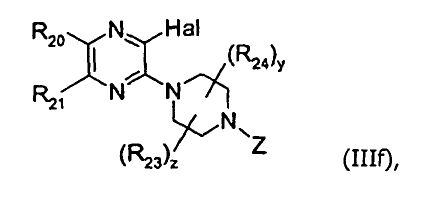 wobei Z, R₂₀, R₂₁, R₂₃, R₂₄, Hal, y und z wie oben definiert sind, und anschließend Umsetzen der Verbindung mit der Formel (IIIf) mit einer Verbindung mit der Formel (IIIb): 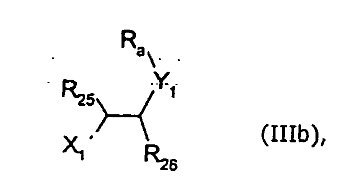 wobei X₁' ausgewählt wird aus -OH, -SH oder -NH(R₂₇) und R₂₅, R₂₆, R₂₇, Y₁ und Rₐ wie für Formel (Ib) definiert sind, in der Gegenwart einer Base wie z.B. Kalium- oder Natrium-*tert*-butoxid oder eines Alkalimetallhydrids wie z.B. Natriumhydrid oder Kaliumhydrid in einem geeigneten Lösungsmittel wie z.B. THF, Dioxan oder DMF, um eine Verbindung mit der Formel (Ib) herzustellen.

30. Ein Verfahren zur Herstellung einer Verbindung mit der Formel (Ib) gemäß irgendeinem der Ansprüche 1-13 durch irgendeinen der folgenden Schritte:
(a) Umwandeln einer resultierenden Verbindung mit der Formel (Ib) in eine andere Verbindung mit der Formel (Ib);
(b) Isolieren eines Isomers einer Verbindung mit der Formel (Ib) aus einer Mischung (z.B. einer racemischen Mischung) von dieser; oder
(c) Umwandeln der freien Base einer Verbindung mit der Formel (Ib) in ein Salz von dieser.

31. Ein Verfahren gemäß irgendeinem der Ansprüche 27 bis 29 zur Herstellung von Verbindungen mit der Formel (Ib) in Anspruch 1, wobei R₂₂ H ist, wobei Z in dem entsprechenden Zwischenprodukt mit der Formel (IIId), (IIIe) oder (IIIf) eine Schutzgruppe ist, die ausgewählt wird aus *tert*-Butoxycarbonyl (*t*-BOC), Benzyl oder Trityl.

32. Ein Verfahren gemäß Anspruch 31, wobei die Schutzgruppe abgespalten wird, um eine Verbindung mit der Formel (Ib) herzustellen.

33. Ein Verfahren gemäß Anspruch 28, wobei das Zwischenprodukt mit der Formel (IIIe) 2-[3-(4-*tert*-Butoxycarbonyl-1-piperazinyl)-2-pyrazinyloxy]ethanol ist.

## Revendications

1. Composé de formule générale (Ib) : 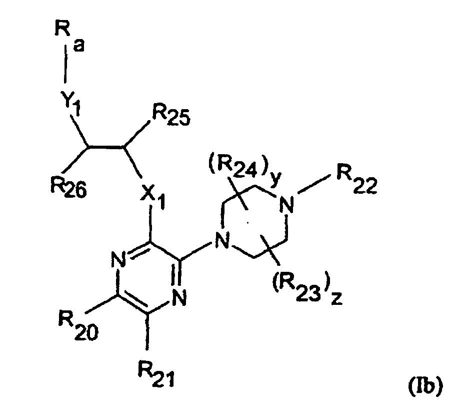 dans laquelle :
X₁ représente indépendamment -O-, -S- ou -N(R₂₇)- ;
Y₁ représente indépendamment -O-, -S- ou -N(R₂₁)- ;
Rₐ représente un aryle ou un hétéroaryle, dont chacun peut être non substitué ou substitué dans une ou plusieurs positions indépendamment les unes des autres par un alkyle en C₁-C₆, un alcoxy en C₁-C₆, un fluoroalcoxy en C₁-C₆, un 2,2,2-trifluoroéthoxy, un alcynyl(en C₃-C₅)oxy, un alcényl(en C₃-C₅)oxy, un diméthylaminoalcoxy en C₁-C₆, un méthylaminoalcoxy en C₁-C₆, un alkyl(en C₁-C₆)thio, un fluorométhyle, un difluorométhyle, un trifluorométhyle, un fluorométhylthio, un difluorométhoxy, un difluorométhylthio, un trifluorométhoxy, un trifluorométhylthio, un halogène, un hydroxy, un nitro, un cyano, un trifluorométhylsulfonyloxy, un alkyl(en C₁-C₆)sulfonamido, un alcényle en C₂-C₆, un alcynyle en C₂-C₆, un acyle en C₁-C₆, un alkyl(en C₁-C₆)carbonylalkyle en C₁-C₆, un alkyl(en C₁-C₆)sulfonyle, un alkyl(en C₁-C₆)sulfonyloxy, un alcoxy(en C₁-C₆)alkyle en C₁-C₆, un alcoxy(en C₁-C₆)carbonyle, un alcoxy(en C₁-C₆)carbonylalkyle en C₁-C₆, un acyl(en C₁-C₆)oxyalkyle en C₁-C₆, un hydroxyalkyle en C₁-C₆, un hydroxyalcoxy en C₁-C₆, un alcoxy(en C₁-C₆)alcoxy en C₁-C₆, un alcoxy(en C₁-C₆)alkyl(en C₁-C₆)thio, un hydroxyalkyl(en C₁-C₆)thio, un hétéroarylalkyl(en C₁-C₆)thio, un arylalkyl(en C₁-C₆)thio, un alcoxy(en C₁-C₆)alkyl(en C₁-C₆)amino, un N-(alcoxy(en C₁-C₆)alkyl(en C₁-C₆))-N-méthylamino, un cycloallcyl(en C₃-C₆)alcoxy en C₁-C₆, un arylalcoxy en C₁-C₆, un hétérocyclylalcoxy en C₁-C₆, un cycloalkyle en C₃-C₈, un cycloalkyl(en C₃-C₈)oxy, un aryle, un aryloxy, un arylthio, un arylsulfonyle, un arylacyle en C₁-C₆, un arylalkyle en C₁-C₆, un arylalcényle en C₂-C₆, un arylalcynyle en C₂-C₆, un hétérocyclylalkyle en C₁-C₆, un hétérocyclylalcényle en C₂-C₆, un hétérocyclylalcynyle en C₂-C₆, un hétérocyclyle, un hétérocyclyloxy, un hétérocyclylthio, un hétérocyclylsulfonyle, un hétérocyclylamino, un hétérocyclylacyle en C₁-C₆, -N(R₂)(R₃), -CON(R₇)(R₈),
où l'un quelconque des résidus cycloalkyle, aryle et hétérocyclyle en tant que substituants sur un aryle ou un hétéroaryle ou en tant que partie de substituants sur un aryle ou un hétéroaryle à son tour peut être substitué dans une ou plusieurs positions indépendamment les unes des autres par un alkyle en C₁-C₄, un alcoxy en C₁-C₄, un méthanesulfonamido, un alkyl(en C₁-C₄)thio, un alkyl(en C₁-C₄)sulfonyle, un acyle en C₁-C₄, un hétérocyclyle, un hétérocyclyloxy, un hétérocyclylthio, un aryloxy, un arylthio, un fluorométhyle, un trifluorométhyle, un trifluorométhoxy, un trifluorométhylthio, un halogène, un hydroxy, un nitro, un cyano, N(R₂)(R₃) ou pour un cycloalkyle en C₃-C₈ et un hétérocyclyle partiellement ou complètement saturé, un oxo ou un hydroxy ;
R₂₀ et R₂₁ indépendamment l'un de l'autre représentent un hydrogène, un alkyle en C₁-C₄ (de préférence un méthyle), un alcoxy en C₁-C₄ (de préférence un méthoxy), un alkyl(en C₁-C₄)thio (de préférence un méthylthio), un halogène, un amino, un méthylamino, un diméthylamino, un acétamido, un phényle, un phénoxy ou un phénylthio, où les phényle, phénoxy et phénylthio peuvent être non substitués ou substitués dans une ou plusieurs positions (de préférence mono- ou disubstitués) indépendamment les unes des autres par un halogène, un méthyle, un méthoxy, un méthylthio, un cyano, un hydroxy ou un trifluorométhyle, ou R₂₀ et R₂₁ conjointement avec les atomes de carbone auxquels ils sont liés forment un cycle aromatique ou hétéroaromatique à 5 ou 6 chaînons, qui éventuellement est indépendamment substitué dans une ou plusieurs positions par un halogène, un méthyle, un méthoxy, un méthylthio, un méthylsulfonyle, un nitro, un cyano, un hydroxy, un trifluorométhyle, un trifluorométhylthio, un amino, un méthylamino, un diméthylamino ou un acétamido ;
R₂₂ représente un hydrogène, un hydroxy, un alkyle en C₁-C₄, un alcényle en C₃-C₄, un acyle en C₁-C₄, un alcoxy(en C₁-C₄)carbonyle, un 2-hydroxyéthyle, un 2-cyanoéthyle ou un tétrahydropyran-2-yle ;
R₂₃ et R₂₄ indépendamment l'un de l'autre représentent un hydrogène, un alkyle en C₁-C₄, un hydroxyméthyle, un alcoxy(en C₁-C₄)méthyle ou un fluorométhyle ;
R₂₅ représente un hydrogène ou un alkyle en C₁-C₄ ;
R₂₆ représente un hydrogène, un alkyle en C₁-C₄ ou est lié à un atome de carbone dans Rₐ adjacent à l'atome se liant à Y₁ pour former un cycle à 5 ou 6 chaînons qui peut contenir un hétéroatome supplémentaire,
R₂₇ représente un hydrogène ou un alkyle en C₁-C₄, de préférence un méthyle ou un éthyle ; et
y et z indépendamment l'un de l'autre valent 1 ou 2,
et des sels, des hydrates, des isomères géométriques, des tautomères, des isomères optiques et des N-oxydes pharmaceutiquement acceptables de celui-ci.

2. Composé selon la revendication 1, dans lequel R₂₀ et R₂₁ indépendamment l'un de l'autre représentent un hydrogène, un halogène ou un alkyle en C₁-C₄.

3. Composé selon la revendication 1, dans lequel R₂₀ et R₂₁ forment un cycle conjointement avec les carbones de cycle auxquels ils sont liés.

4. Composé selon la revendication 3, dans lequel R₂₀ et R₂₁ conjointement avec les atomes de carbone auxquels ils sont liés forment un benzène, pour former un cycle quinoxaline, éventuellement mono- ou disubstitué par un halogène ou un thiophène, pour former un cycle thiéno[3,4-b]pyrazine.

5. Composé selon l'une quelconque des revendications 1 à 4, dans lequel X₁ et Y₁ représentent tous les deux -O-.

6. Composé selon l'une quelconque des revendications 1 à 4, dans lequel X₁ représente -S- et Y₁ représente -O-.

7. Composé selon l'une quelconque des revendications 1 à 4, dans lequel X₁ représente -O- et Y₁ représente -S-.

8. Composé selon l'une quelconque des revendications 1 à 4, dans lequel X₁ et Y₁ représentent tous les deux -S-.

9. Composé selon l'une quelconque des revendications 1 à 8, dans lequel R₂₂ représente un hydrogène.

10. Composé selon l'une quelconque des revendications 1 à 9, dans lequel R₂₃ représente un méthyle, z vaut 1 et R₂₄ représente un hydrogène, de préférence dans la configuration (R).

11. Composé selon l'une quelconque des revendications 1 à 10, dans lequel R₂₅ représente un hydrogène et R₂₆ représente un méthyle.

12. Composé selon l'une quelconque des revendications 1 à 11, dans lequel R₂₅ représente un hydrogène.

13. Composé selon la revendication 1, qui est :
l'éther de 2-(2-phénoxy)éthyle et de 3-(1-pipérazinyl)-2-pyrazinyle,
l'éther de 2-(2-fluorophénoxy)éthyle et de 3-(1-pipérazinyl)-2-pyrazinyle,
l'éther de 2-(3-cyanophénoxy)éthyle et de 3-(1-pipérazinyl)-2-pyrazinyle,
l'éther de 2-(3-méthoxyphénoxy)éthyle 3-(1-pipérazinyl)-2-pyrazinyle,
l'éther de 2-[3-(2-hydroxyéthoxy)phénoxy]éthyle et de 3-(1-pipérazinyl)-2-pyrazinyle,
l'éther de 2,3-dihydro-1,4-benzodioxin-2-ylméthyle et de 3-(1-piperazinyl)-2-pyrazinyle,
l'éther de 2-(2-méthoxyphénoxy)éthyle et de 3-(1-pipérazinyl)-2-pyrazinyle,
l'éther de 2-(2,5-difluorophénoxy)éthyle et de 3-(1-pipérazinyl)-2-pyrazinyle,
l'éther de 2-(3,5-diméthoxyphénoxy)éthyle et de 3-(1-pipérazinyl)-2-pyrazinyle,
la 2-(3,4-dihydro-2*H*-pyrido[3,2-*b*]-1,4-oxazin-2-ylméthoxy)-3-(1-pipérazinyl)pyrazine,
la 2-méthyl-1-[3-(2-phénoxyéthoxy)-2-pyrazinyl]pipérazine, particulièrement l'énantiomère (*R*) de celle-ci,
la 2-méthyl-1-{3-(2-(3-pyridinyloxy)éthoxy]-2-pyrazinyl}pipérazine, particulièrement l'énantiomère (*R*) de celle-ci,
l'éther de 2-(quinazolinyl-8-yloxy)éthyle et de 3-(1-pipérazinyl)-2-pyrazinyle,
l'éther de 2-(isoquinoléinyl-5-yloxy)éthyle et de 3-(1-pipérazinyl)-2-pyrazinyle,
la 2-[2-(3-pyridinyloxy)éthoxy]-3-(1-pipérazinyl)-6,7-difluoroquinoxaline,
la 2-[2-(3-pyridinyloxy)éthoxy]-3-(1-pipérazinyl)thiéno[3,4-b]pyrazine,
la 2-(3,4-dihydro-2*H*-1,4-benzoxazin-2-ylméthoxy)-3-(1-pipérazinyl)pyrazine,
la 2-méthyl-1-{3-[2-(2-amino-8-quinoléinyloxy)éthoxy]-2-pyrazinyl}pipérazine, particulièrement l'énantiomère (*R*) de celle-ci,
la 1-{3-[2-(2-méthoxy-3-pyridinyloxy)éthoxy]-2-pyrazinyl}pipérazine,
la 2-méthyl-1-{3-[2-(2-méthoxy-3-pyridinyloxy)éthoxy]-2-pyrazinyl}pipérazine, particulièrement l'énantiomère (*R*) de celle-ci,
la 2-{2-[(2-chloro-3-pyridinyl)oxy]éthoxy} -3-(1-pipérazinyl)pyrazine,
la 2-{2-[(2-éthoxy-3-pyridinyl)oxy]éthoxy}-3-(1-pipérazinyl)pyrazine,
la 1-(3-{2-[(2-éthoxy-3-pyridinyl)oxy]éthoxy}-2-pyrazinyl)-2-méthylpipérazine, particulièrement l'énandomère (*R*) de celle-ci,
la 2-(2-{[2-(méthylsulfanyl)-3-pyridinyl]oxy} éthoxy)-3-(1-pipérazinyl)-pyrazine,
la 2-méthyl-1-[3-(2-{[2-(méthylsulfanyl)-3-pyridinyl]oxy}éthoxy)-2-pyrazinyl]pipérazine, particulièrement l'énantiomère (*R*) de celle-ci,
la 2-{2-[(2-bromo-3-pyridinyl)oxy]éthoxy}-3-(1-pipérazinyl)pyrazine,
la 1-(3-{2-[(2-bromo-3-pyridinyl)oxy]éthoxy}-2-pyrazinyl)-2-méthylpipérazine, particulièrement l'énantiomère (*R*) de celle-ci,
la 2-(1-pipérazinyl)-3-(2-{3-[2-(2-pyridinyl)éthoxy]phénoxy}éthoxy)pyrazine,
la 2-(2-{3-[2-(4-méthyl-1,3-thiazol-5-yl)éthoxy]phénoxy}éthoxy)-3-(1-pipérazinyl)pyrazine,
la 2-(1-pipérazinyl)-3-{2-[3-(tétrahydro-3-furanylméthoxy)phénoxy]éthoxy}pyrazine,
la 2-(1-pipérazinyl)-3-{2-[3-(tétrahydro-3-furanyloxy)phénoxy]éthoxy}pyrazine,
la 1-{2-[3-(2-{[3-(1-pipérazinyl)-2-pyrazinyl]oxy}éthoxy)phénoxy]éthyle)-2-pyrrolidinone,
la 2-{2-[3-(2-méthoxyéthoxy)phénoxy]éthoxy}-3-(1-pipérazinyl)pyrazine,
le 2-{[3-(2-{(3-(1-pipérazinyl)-2-pyrazinyl]oxy}éthoxy)phénoxy]méthyle}benzonitrile,
la 2-(1-pipérazinyl)-3-{2-[3-(tétrahydro-2H-pyran-4-yloxy)phénoxy]éthoxy}pyrazine,
la N,N-diméthyl-N-{2-[3-(2-{[3-(1-pipérazinyl)-2-pyrazinyl]oxy}éthoxy)-phénoxy]éthyl}amine,
la 7-(2-{[3-(1-pipérazinyl)-2-pyrazinyl]oxy}éthoxy)-2H-chromén-2-one,
la 1-(3-{2-[(2-chloro-3-pyridinyl)oxy]éthoxy}-2-pyrazinyl)-2-méthylpipérazine, particulièrement l'énantiomère (R) de celle-ci,
l'éther de 7-isoquinoléinyle et de 2-{[3-(1-pipérazinyl)]-2-pyrazinyl}oxy)éthyle,
l'éther de 2-(2-chloro-4-méthoxyphénoxy)éthyle et de 3-(1-pipérazinyl)-2-pyrazinyle,
la 4-(2-{[3-(1-pipérazinyl)-2-pyrazinyl]oxy}éthoxy)-2-quinoléinamine,
ou un sel ou un solvate pharmacologiquement acceptable de celui-ci.

14. Composé selon l'une quelconque des revendications 1 à 13 pour une utilisation en thérapie d'un être humain ou d'un animal.

15. Composé selon la revendication 14, où la thérapie est orientée vers la prophylaxie ou le traitement d'une maladie associée à la sérotonine, associée au récepteur 5-HT_{2c}.

16. Composé selon la revendication 15, où la maladie associée à la sérotonine est choisie parmi les troubles de l'alimentation, spécialement l'obésité.

17. Composé selon la revendication 15, où la maladie associée à la sérotonine est choisie parmi les troubles de la mémoire.

18. Composé selon la revendication 15, où la maladie associée à la sérotonine est choisie parmi les troubles de l'humeur, comprenant la dépression majeure et la dépression bipolaire comprenant un trouble à la fois léger et bipolaire, et la dépression saisonnière.

19. Composé selon la revendication 15, où la maladie associée à la sérotonine est choisie parmi les troubles de l'anxiété, y compris l'anxiété de situation, le trouble d'anxiété généralisé, les troubles d'anxiété primaires et les troubles d'anxiété secondaires.

20. Composé selon la revendication 15, où la maladie associée à la sérotonine est choisie parmi les dysfonctionnements sexuels et les troubles urinaires.

21. Composé selon la revendication 15, où la maladie associée à la sérotonine est la douleur.

22. Composé selon la revendication 15, où la maladie associée à la sérotonine est la schizophrénie.

23. Utilisation d'un composé selon l'une quelconque des revendications 1 à 13 pour la fabrication d'un médicament destiné au traitement ou à la prévention d'une maladie associée à la sérotonine, particulièrement associée au récepteur 5-HT_{2c}.

24. Utilisation selon la revendication 23, où la maladie associée à la sérotonine est choisie parmi les troubles de l'alimentation, spécialement l'obésité, les troubles de la mémoire, la schizophrénie, les troubles de l'humeur, des troubles de l'anxiété, la douleur, les dysfonctionnements sexuels et les troubles urinaires.

25. Composition pharmaceutique comprenant un composé selon l'une quelconque des revendications 1 à 13 en tant que principe actif, conjointement avec un support pharmacologiquement et pharmaceutiquement acceptable.

26. Procédé de modulation de la fonction du récepteur 5-HT_{2c}, comprenant la mise en contact du récepteur avec une quantité inhibitrice efficace d'un composé selon l'une quelconque des revendications 1 à 13.

27. Procédé de préparation d'un composé de formule (Ib) selon l'une quelconque des revendications 1 à 13, lequel procédé comprend :
la réaction d'un composé de formule (IIIa) : 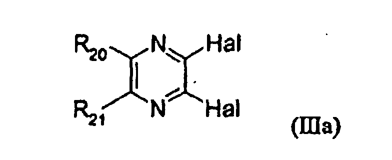
dans laquelle R₂₀ et R₂₁ sont tels que définis pour la formule (Ib) et Hal représente un halogène, avec un composé de formule (IIIb) : 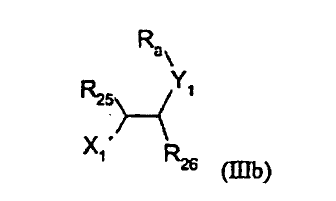 dans laquelle X₁' est choisi parmi -OH, -SH ou -NH(R₂₇) et R₂₅, R₂₆, R₂₇, Y₁ et Rₐ sont tels que définis pour la formule (Ib), en présence d'une base telle que le *tert*-butoxyde de potassium ou de sodium ou un hydrure de métal alcalin tel que l'hydrure de sodium ou l'hydrure de potassium dans un solvant approprié tel que le THF, le dioxanne ou le DMF pour produire un composé de formule (IIIc) : 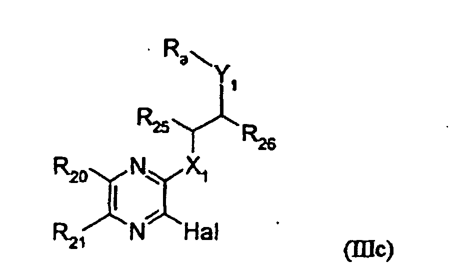 dans laquelle R₂₀, R₂₁, R₂₅, R₂₆, Hal, X₁ et Rₐ sont tels que définis ci-dessus, et ensuite la réaction du composé de formule (IIIc) avec un dérivé de pipérazine approprié de formule (IIId) : 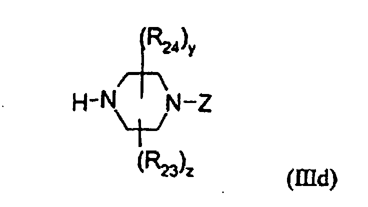 dans laquelle
R₂₃, R₂₄, y et z sont tels que définis pour la formule (Ib) pour produire le composé de formule (Ib) ; et
Z est tel que défini pour R₂₂ dans la formule (Ib) ou représente un groupe protecteur approprié tel qu'un *tert*-butoxycarbonyle, un trityle ou un benzyle ; ou

28. Procédé de préparation d'un composé de formule (Ib) selon l'une quelconque des revendications 1 à 13, où Y₁ est choisi parmi un oxygène ou un soufre, lequel procédé comprend :
la réaction d'un composé de formule (IIIe) : 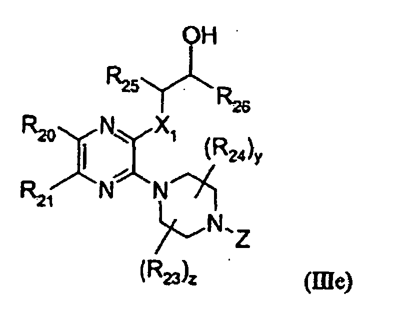
dans laquelle
X₁ est choisi parmi un oxygène ou un soufre,
R₂₀, R₂₁, R₂₃, R₂₄, y et z sont tels que définis pour la formule (Ib),
R₂₅ et R₂₆ représentent chacun indépendamment un hydrogène ou un méthyle, de préférence un hydrogène ; et
Z est tel que défini pour R₂₂ dans la formule (Ib) ou représente un groupe protecteur approprié tel qu'un *tert*-butoxycarbonyle, un trityle ou un benzyle ; avec un phénol ou un thiophénol approprié correspondant à Rₐ dans la formule (Ib) dans les conditions de Mitsunobu, par exemple, en présence d'azodicarboxylate de diéthyle (DEAD) ou de 1,1'-azobis(*N,N*-diméthylformamide) (TMAD) ; et de triphénylphosphine ou de tri-*n*-butylphosphine ; dans un solvant approprié tel que le tétrahydrofuranne (THF) pour produire le composé de formule (Ib).

29. Procédé de préparation d'un composé de formule (Ib) selon l'une quelconque des revendications 1 à 13 en faisant réagir un composé de formule (IIIa) : 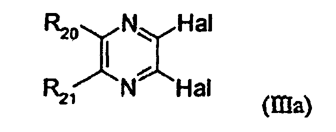 dans laquelle R₂₀ et R₂₁ sont tels que définis pour la formule (Ib) et Hal représente un halogène, avec un dérivé de pipérazine de formule (IIId) : 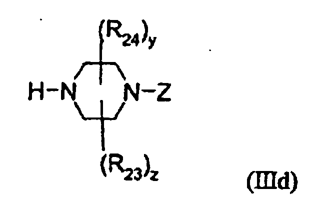 dans laquelle
R_{23,} R_{24,} y et z sont tels que définis pour la formule (Ib), et
Z est tel que défini pour R₂₂ dans la formule (Ib) ou représente un groupe protecteur approprié tel qu'un *tert*-butoxycarbonyle, un trityle ou un benzyle ; pour produire le composé de formule (IIIf) : 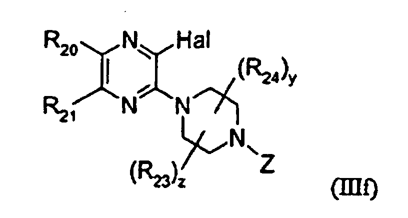 dans laquelle Z, R₂₀, R₂₁,R₂₃, R₂₄, Hal, y et z sont tels que définis ci-dessus et ensuite en faisant réagir le composé de formule (IIIf) avec un composé de formule (IIIb) : 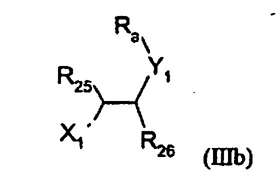 dans laquelle X₁' est choisi parmi -OH, -SH, -NH(R₂₇) et R₂₅, R₂₆, R₂₇, Y₁ et Rₐ sont tels que définis pour la formule (Ib), en présence d'une base telle que le *tert*-butoxyde de potassium ou de sodium ou un hydrure de métal alcalin tel que l'hydrure de sodium ou l'hydrure de potassium dans un solvant approprié tel que le THF, le dioxanne ou le DMF pour produire un composé de formule (Ib).

30. Procédé de préparation d'un composé de formule (Ib) selon l'une quelconque des revendications 1 à 13 par l'un quelconque de
(a) la conversion d'un composé résultant de formule (Ib) en un autre composé de formule (Ib) ;
(b) l'isolement d'un isomère d'un composé de formule (Ib) d'un mélange (par exemple, un mélange racémique) de celui-ci ; ou
(c) la conversion de la base libre d'un composé de formule (Ib) en un sel de celui-ci.

31. Procédé selon l'une quelconque des revendications 27 à 29 pour la préparation de composés de formule (Ib) selon la revendication 1, où R₂₂ représente H, où Z dans l'intermédiaire correspondant de formule (IIId), (IIIe) ou (IIIf) représente un groupe protecteur choisi parmi un *tert*-butoxycarbonyle (*t*-BOC), un benzyle ou un trityle.

32. Procédé selon la revendication 31 où ledit groupe protecteur est éliminé par clivage pour produire un composé de formule (Ib).

33. Procédé selon la revendication 28, dans lequel l'intermédiaire de formule (IIIe) est le 2-[3-(4-*tert*-butoxycarbonyl-1-pipérazinyl)-2-pyrazinyloxy]éthanol.
